# EUROPEAN PATENT APPLICATION

(11) **EP 4 212 526 A1**
(43) Date of publication of application: **19.07.2023**
(21) Application number: 21867021.4
(22) Date of filing: 25.08.2021
(51) Int. Cl.: C07D 405/14, C07D 409/14, H01L 51/00, H01L 51/50

(54) **HETEROCYCLIC COMPOUND AND ORGANIC LIGHT-EMITTING DEVICE COMPRISING SAME**

(30) Priority: 11.09.2020 KR 20200116637
(71) Applicant: LT Materials Co., Ltd., Yongin-si, Gyeonggi-do 17118 (KR)
(72) Inventor: CHOI, Eui-Jeong, Yongin-City, Gyeonggi-do 17118 (KR); NO, Young-Seok, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/011361
(87) International publication number: WO 2022/055155

(57) **Abstract**

The present specification relates to a heterocyclic compound of Chemical Formula 1, and an organic light emitting device and a composition for forming an organic material layer including the same.

## Description

### [Technical Field]

The present specification relates to a heterocyclic compound, and an organic light emitting device including the same.

This application claims priority to and the benefits of Korean Patent Application No. 10-2020-0116637, filed with the Korean Intellectual Property Office on September 11, 2020, the entire contents of which are incorporated herein by reference.

### [Background Art]

An electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

### [Disclosure]

### [Technical Problem]

The present specification is directed to providing a heterocyclic compound, and an organic light emitting device including the same.

### [Technical Solution]

One embodiment of the present specification provides a heterocyclic compound represented by the following Chemical Formula 1.

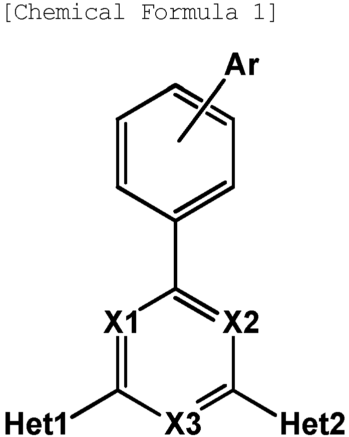

In Chemical Formula 1,
X1 to X3 are N or CR, and at least one of X1 to X3 is N,
R is hydrogen; or deuterium,
Ar is a C9 to C60 aryl group formed with a monocyclic ring,
Het1 is represented by the following Chemical Formula 1-A, and
Het2 is represented by any one of the following Chemical Formulae 1-B to 1-D, 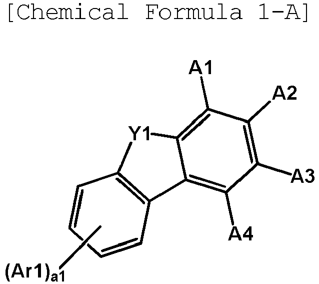 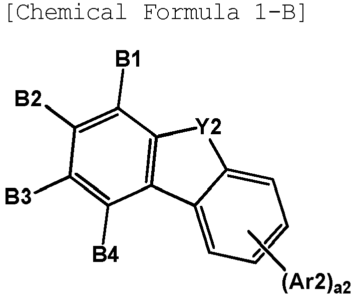 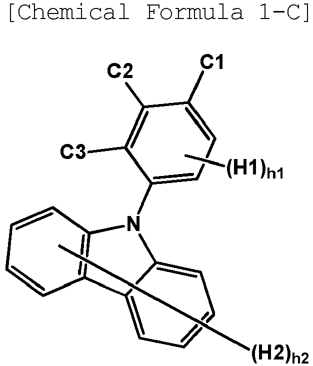 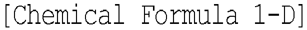 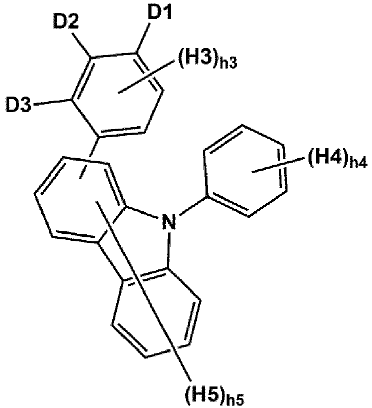
in Chemical Formulae 1-A to 1-D,
Y1 and Y2 are each independently O; S; or NR',
R' is a C6 to C60 aryl group unsubstituted or substituted with deuterium; or a C2 to C60 heteroaryl group,
H1 to H5 are hydrogen; or deuterium,
h1 and h3 are each an integer of 0 to 2, h2 is an integer of 0 to 8, h4 is an integer of 0 to 5, h5 is an integer of 0 to 7, and when h1 and h3 are each 2 or h2, h4 and h5 are each 2 or greater, substituents in the parentheses are the same as or different from each other,
Ar1 and Ar2 are each independently hydrogen; deuterium; or a substituted or unsubstituted C6 to C18 aryl group,
a1 and a2 are each an integer of 0 to 4, and when each 2 or greater, substituents in the parentheses are the same as or different from each other,
A1 to A4, B1 to B4, C1 to C3 and D1 to D3 either bond to Chemical Formula 1, or hydrogen; or deuterium, and
Chemical Formula 1 bonds to any one of A1 to A4 of Chemical Formula 1-A, and bonds to any one of B1 to B4 of Chemical Formula 1-B, any one of C1 to C3 of Chemical Formula 1-C or any one of D1 to D3 of Chemical Formula 1-D, which is represented by Am-Bn, Am-Co or Am-Dp, m and n are each 1, 2, 3 or 4, o and p are each 1, 2 or 3, m and n, m and o or m and p are each the same as or different from each other, and when Y1 and Y2 are each O or S, m and n are different.

Another embodiment of the present specification provides an organic light emitting device including a first electrode; a second electrode provided opposite to the first electrode; and an organic material layer provided between the first electrode and the second electrode, wherein the organic material layer includes one or more types of the heterocyclic compound of Chemical Formula 1.

Another embodiment of the present specification provides a composition for forming an organic material layer, the composition including the heterocyclic compound of Chemical Formula 1; and a compound of the following Chemical Formula 2.

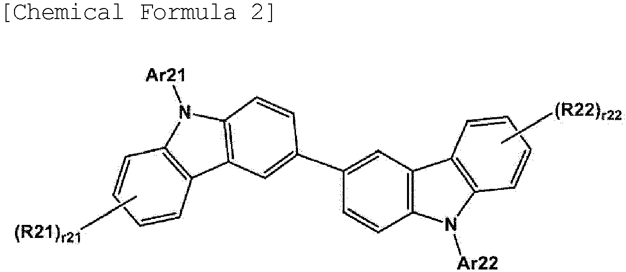

In Chemical Formula 2,
R21 and R22 are each independently hydrogen; deuterium; a halogen group; a cyano group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
Ar21 and Ar22 are each independently a substituted or unsubstituted C6 to C60 aryl group; or a substituted or unsubstituted C2 to C60 heteroaryl group,
r21 is an integer of 0 to 4, and when 2 or greater, R21s are the same as or different from each other, and
r22 is an integer of 0 to 4, and when 2 or greater, R22s are the same as or different from each other.

### [Advantageous Effects]

A compound described in the present specification can be used as a material of an organic material layer of an organic light emitting device. The compound is capable of acting as a hole injection material, a hole transfer material, a light emitting material, an electron transfer material, an electron injection material, a charge generation material or the like. Particularly, the heterocyclic compound can be used as a material of a light emitting layer of an organic light emitting device.

In the heterocyclic compound of Chemical Formula 1, triazine is substituted with one aryl group, and bonds to two heteroaryl groups at specific positions, and as a result, LUMO is more widely expanded making intermolecular electron transfer more efficient. In addition, intramolecular electron transfer is also effective since an electron transfer moiety and a hole transport moiety closely bond. Accordingly, an organic light emitting device using the heterocyclic compound of Chemical Formula 1 has low driving voltage and high efficiency.

Using the heterocyclic compound of Chemical Formula 1 and the compound of Chemical Formula 2 together as a material of a light emitting layer of an organic light emitting device is capable of lowering a driving voltage, enhancing light emission efficiency and enhancing lifetime properties in the device.

### [Description of Drawings]

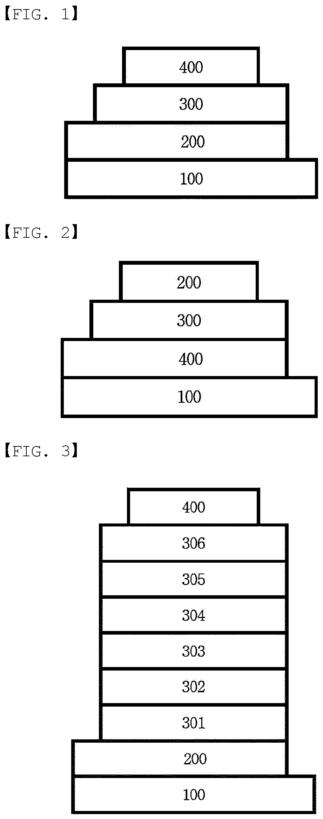

FIG. 1 to FIG. 3 are diagrams each illustrating a lamination structure of an organic light emitting device according to one embodiment of the present specification.

### [Reference Numeral]

- 100:: Substrate
- 200:: Anode
- 300:: Organic Material Layer
- 301:: Hole Injection Layer
- 302:: Hole Transfer Layer
- 303:: Light Emitting Layer
- 304:: Hole Blocking Layer
- 305:: Electron Transfer Layer
- 306:: Electron Injection Layer
- 400:: Cathode

### [Mode for Disclosure]

Hereinafter, the present specification will be described in more detail.

In the present specification, a description of a certain part "including" certain constituents means capable of further including other constituents, and does not exclude other constituents unless particularly stated on the contrary.

A term "substitution" means a hydrogen atom bonding to a carbon atom of a compound being changed to another substituent, and the position of substitution is not limited as long as it is a position at which the hydrogen atom is substituted, that is, a position at which a substituent is capable of substituting, and when two or more substituents substitute, the two or more substituents may be the same as or different from each other.

In the present specification, "substituted or unsubstituted" means being substituted with one or more substituents selected from the group consisting of deuterium; halogen; a cyano group; a C1 to C60 alkyl group; a C2 to C60 alkenyl group; a C2 to C60 alkynyl group; a C3 to C60 cycloalkyl group; a C2 to C60 heterocycloalkyl group; a C6 to C60 aryl group; a C2 to C60 heteroaryl group; a silyl group; a phosphine oxide group; and an amine group, or being unsubstituted, or being substituted with a substituent linking two or more substituents selected from among the substituents illustrated above, or being unsubstituted.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0%, a hydrogen content being 100% or substituents being all hydrogen.

In one embodiment of the present application, deuterium is one of isotopes of hydrogen, is an element having deuteron formed with one proton and one neutron as a nucleus, and may be expressed as hydrogen-2, and the elemental symbol may also be written as D or ²H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 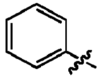 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

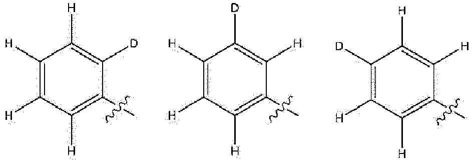

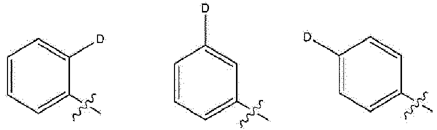

In addition, in one embodiment of the present application, "a phenyl group having a deuterium content of 0%" may mean a phenyl group that does not include a deuterium atom, that is, a phenyl group that has 5 hydrogen atoms.

In the present specification, the halogen may be fluorine, chlorine, bromine or iodine.

In the present specification, the alkyl group includes linear or branched, and may be further substituted with other substituents. The number of carbon atoms of the alkyl group may be from 1 to 60, specifically from 1 to 40 and more specifically from 1 to 20. Specific examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group includes linear or branched, and may be further substituted with other substituents. The number of carbon atoms of the alkenyl group may be from 2 to 60, specifically from 2 to 40 and more specifically from 2 to 20. Specific examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes linear or branched, and may be further substituted with other substituents. The number of carbon atoms of the alkynyl group may be from 2 to 60, specifically from 2 to 40 and more specifically from 2 to 20.

In the present specification, the cycloalkyl group includes monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group includes a spiro group. The number of carbon atoms of the aryl group may be from 6 to 60, specifically from 6 to 40 and more specifically from 6 to 25. When the aryl group is dicyclic or higher, the number of carbon atoms may be from 8 to 60, from 8 to 40 or from 8 to 30. Specific examples of the aryl group may include a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring group thereof, and the like, but are not limited thereto.

In the present specification, the terphenyl group includes linear or branched, and may be selected from among the following structures.

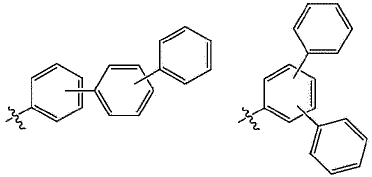

In the present specification, the fluorenyl group may be substituted, and adjacent substituents may bond to each other to form a ring.

When the fluorenyl group is substituted, 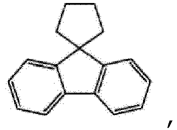 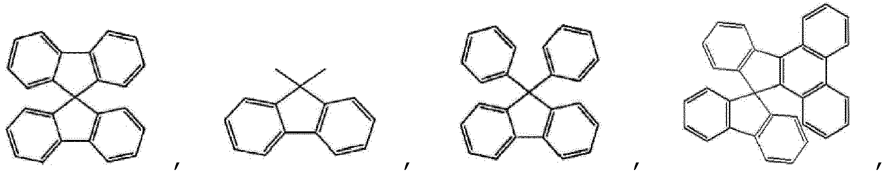 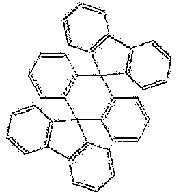 and the like may be included, however, the structure is not limited thereto.

In the present specification, the heteroaryl group includes O, S, SO₂, Se, N or Si as a heteroatom, includes monocyclic or polycyclic, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. When the heteroaryl group is dicyclic or higher, the number of carbon atoms may be from 4 to 60, from 4 to 40 or from 4 to 25. Specific examples of the heteroaryl group may include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, 5,10-dihydrobenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a benzofuro[2,3-d]pyrimidyl group; a benzothieno[2,3-d]pyrimidyl group; a benzofuro[2,3-a]carbazolyl group, a benzothieno[2,3-a]carbazolyl group, a 1,3-dihydroindolo[2,3-a]carbazolyl group, a benzofuro[3,2-a]carbazolyl group, a benzothieno[3,2-a]carbazolyl group, a 1,3-dihydroindolo[3,2-a]carbazolyl group, a benzofuro[2,3-b]carbazolyl group, a benzothieno[2,3-b]carbazolyl group, a 1,3-dihydroindolo[2,3-b]carbazolyl group, a benzofuro[3,2-b]carbazolyl group, a benzothieno[3,2-b]carbazolyl group, a 1,3-dihydroindolo[3,2-b]carbazolyl group, a benzofuro[2,3-c]carbazolyl group, a benzothieno[2,3-c]carbazolyl group, a 1,3-dihydroindolo[2,3-c]carbazolyl group, a benzofuro[3,2-c]carbazolyl group, a benzothieno[3,2-c]carbazolyl group, a 1,3-dihydroindolo[3,2-c]carbazolyl group, a 1,3-dihydroindeno[2,1-b]carbazolyl group, a 5,11-dihydroindeno[1,2-b]carbazolyl group, a 5,12-dihydroindeno[1,2-c]carbazolyl group, a 5,8-dihydroindeno[2,1-c]carbazolyl group, a 7,12-dihydroindeno[1,2-a]carbazolyl group, a 11,12-dihydroindeno[2,1-a]carbazolyl group and the like, but are not limited thereto.

In the present specification, the silyl group is a substituent including Si, having the Si atom directly linked as a radical, and is represented by -Si(R101)(R102)(R103). R101 to R103 are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the phosphine oxide group is represented by -P(=O) (R104) (R105), and R104 and R105 are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. Specifically, the phosphine oxide group may be substituted with an aryl group, and as the aryl group, the examples described above may be applied. Examples of the phosphine oxide group may include a diphenylphosphine oxide group, a dinaphthylphosphine oxide group and the like, but are not limited thereto.

In the present specification, the amine group is represented by -N(R106) (R107), and R106 and R107 are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. The amine group may be selected from the group consisting of -NH₂; a monoalkylamine group; a monoarylamine group; a monoheteroarylamine group; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the examples of the aryl group described above may be applied to the arylene group except that the arylene group is a divalent group.

In the present specification, the examples of the heteroaryl group described above may be applied to the heteroarylene group except that the heteroarylene group is a divalent group.

In one embodiment of the present specification, X1 to X3 of Chemical Formula 1 are N or CR, and at least one of X1 to X3 is N.

In one embodiment of the present specification, X1 to X3 are N or CR, and at least two of X1 to X3 are N.

In one embodiment of the present specification, X1 to X3 are all N.

In one embodiment of the present specification, when X1, X2 or X3 is CR, R is hydrogen; or deuterium.

In one embodiment of the present specification, Ar of Chemical Formula 1 is a C9 to C60 aryl group formed with a monocyclic ring.

In one embodiment of the present specification, Ar is a C9 to C40 aryl group formed with a monocyclic ring.

In one embodiment of the present specification, Ar is a C9 to C20 aryl group formed with a monocyclic ring.

In one embodiment of the present specification, Ar is a C12 aryl group formed with a monocyclic ring.

In one embodiment of the present specification, Ar is a C12 to C60 aryl group formed with a phenyl group.

In one embodiment of the present specification, Ar is a C12 to C40 aryl group formed with a phenyl group.

In one embodiment of the present specification, Ar is a C12 to C20 aryl group formed with a phenyl group.

In one embodiment of the present specification, Ar may be a biphenyl group.

In one embodiment of the present specification, Ar may be a linear terphenyl group.

In one embodiment of the present specification, Ar may be represented by any one of the following structural formulae.

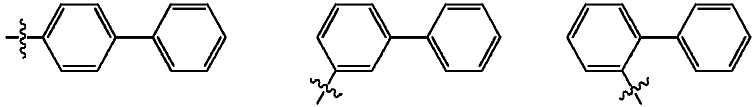

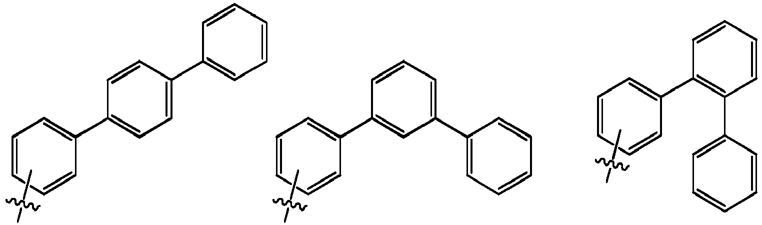

Particularly, as Ar is extended longer, LUMO is more effectively expanded and flatness of the molecular structure increases, which more favorably receives electrons, and accordingly, electron migration occurs more effectively, and the packing structure becomes more stable as well when deposited on a substrate. On the other hand, when 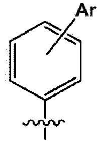 of Chemical Formula 1 is a branched terphenyl group, LUMO is spread in a circle, and lifetime and efficiency are identified to decrease compared to in the materials of the present disclosure.

In one embodiment of the present specification, Het1 of Chemical Formula 1 is represented by the following Chemical Formula 1-A, and Het2 of Chemical Formula 1 is represented by any one of the following Chemical Formulae 1-B to 1-D.

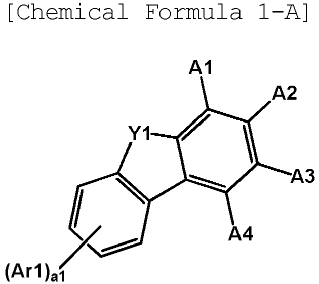

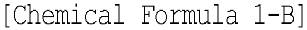

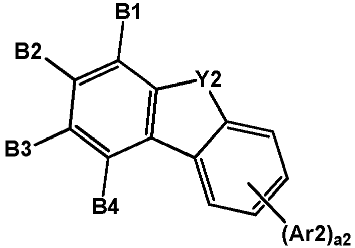

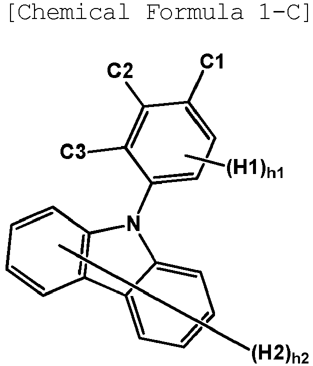

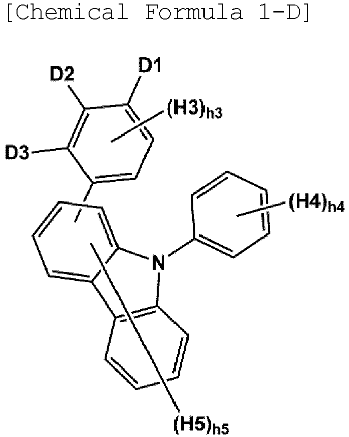

In Chemical Formulae 1-A to 1-D,
Y1 and Y2 are each independently O; S; or NR',
R' is a C6 to C60 aryl group unsubstituted or substituted with deuterium; or a C2 to C60 heteroaryl group,
H1 to H5 are hydrogen; or deuterium,
h1 and h3 are each an integer of 0 to 2, h2 is an integer of 0 to 8, h4 is an integer of 0 to 5, h5 is an integer of 0 to 7, and when h1 and h3 are each 2 or h2, h4 and h5 are each 2 or greater, substituents in the parentheses are the same as or different from each other,
Ar1 and Ar2 are each independently hydrogen; deuterium; or a substituted or unsubstituted C6 to C18 aryl group,
a1 and a2 are each an integer of 0 to 4, and when each 2 or greater, substituents in the parentheses are the same as or different from each other,
A1 to A4, B1 to B4, C1 to C3 and D1 to D3 either bond to Chemical Formula 1, or hydrogen; or deuterium, and
Chemical Formula 1 bonds to any one of A1 to A4 of Chemical Formula 1-A, and bonds to any one of B1 to B4 of Chemical Formula 1-B, any one of C1 to C3 of Chemical Formula 1-C or any one of D1 to D3 of Chemical Formula 1-D, which is represented by Am-Bn, Am-Co or Am-Dp, m and n are each 1, 2, 3 or 4, o and p are each 1, 2 or 3, m and n, m and o or m and p are each the same as or different from each other, and when Y1 and Y2 are each O or S, m and n are different.

In one embodiment of the present specification, Ar1 of Chemical Formula 1-A and Ar2 of Chemical Formula 1-B may be each independently hydrogen; deuterium; a substituted or unsubstituted phenyl group; a substituted or unsubstituted biphenyl group; a substituted or unsubstituted naphthyl group; or a substituted or unsubstituted terphenyl group.

In one embodiment of the present specification, Ar1 and Ar2 may be each independently hydrogen; deuterium; a phenyl group; a biphenyl group; a naphthyl group; or a terphenyl group.

In one embodiment of the present specification, Ar1 and Ar2 may be each independently hydrogen; deuterium; or a phenyl group.

In one embodiment of the present specification, Ar1 and Ar2 may be each independently hydrogen; deuterium; or a phenyl group unsubstituted or substituted with deuterium.

In one embodiment of the present specification, Ar1 and Ar2 may be each independently hydrogen; or deuterium.

In one embodiment of the present specification, Ar1 and Ar2 may be each independently a substituted or unsubstituted C6 to C18 aryl group.

When Ar1 and Ar2 of Chemical Formula 1-A and Chemical Formula 1-B are a C6 to C18 aryl group, the material has various dissociation temperatures depending on the substituent type. Accordingly, the range of choices for the compound of Chemical Formula 2 that may be mixed with the heterocyclic compound of Chemical Formula 1 become wider.

In one embodiment of the present specification, Y1 of Chemical Formula 1-A may be O.

In one embodiment of the present specification, Y1 may be S.

In one embodiment of the present specification, Y1 is NR', and R' may be a C6 to C60 aryl group unsubstituted or substituted with deuterium; or a C2 to C60 heteroaryl group.

In one embodiment of the present specification, Y1 is NR', and R' may be a C6 to C30 aryl group unsubstituted or substituted with deuterium.

In one embodiment of the present specification, Y1 is NR', and R' may be a phenyl group unsubstituted or substituted with deuterium.

In one embodiment of the present specification, Y1 is NR', and R' may be a C6 to C60 aryl group; or a C2 to C60 heteroaryl group.

In one embodiment of the present specification, Y1 is NR', and R' may be a C6 to C30 aryl group; or a C2 to C30 heteroaryl group.

In one embodiment of the present specification, Y1 is NR', and R' may be a C6 to C30 aryl group.

In one embodiment of the present specification, Y1 is NR', and R' may be a phenyl group.

In one embodiment of the present specification, Y2 of Chemical Formula 1-B may be O.

In one embodiment of the present specification, Y2 may be S.

In one embodiment of the present specification, Y2 is NR', and R' may be a C6 to C60 aryl group unsubstituted or substituted with deuterium; or a C2 to C60 heteroaryl group.

In one embodiment of the present specification, Y2 is NR', and R' may be a C6 to C30 aryl group unsubstituted or substituted with deuterium.

In one embodiment of the present specification, Y2 is NR', and R' may be a phenyl group unsubstituted or substituted with deuterium.

In one embodiment of the present specification, Y2 is NR', and R' may be a C6 to C60 aryl group; or a C2 to C60 heteroaryl group.

In one embodiment of the present specification, Y2 is NR', and R' may be a C6 to C30 aryl group; or a C2 to C30 heteroaryl group.

In one embodiment of the present specification, Y2 is NR', and R' may be a C6 to C30 aryl group.

In one embodiment of the present specification, Y2 is NR', and R' may be a phenyl group.

In one embodiment of the present specification, A1 to A4, B1 to B4, C1 to C3 and D1 to D3 either bond to Chemical Formula 1, or hydrogen; or deuterium.

In one embodiment of the present specification, Chemical Formula 1 bonds to any one of A1 to A4 of Chemical Formula 1-A, and bonds to any one of B1 to B4 of Chemical Formula 1-B, any one of C1 to C3 of Chemical Formula 1-C or any one of D1 to D3 of Chemical Formula 1-D, and the rest not bonding to Chemical Formula 1 are hydrogen; or deuterium.

In one embodiment of the present specification, Chemical Formula 1 bonds to any one of A1 to A4 of Chemical Formula 1-A and any one of B1 to B4 of Chemical Formula 1-B, which is represented by Am-Bn, m and n are each 1, 2, 3 or 4, m and n are the same as or different from each other, and when Y1 and Y2 are each O or S, m and n are different.

For example, when Chemical Formula 1 bonds to A1 of Chemical Formula 1-A and bonds to B1 of Chemical Formula 1-B, it may be represented as A1-B1.

In one embodiment of the present specification, when Het2 is Chemical Formula 1-B and Y1 and Y2 are each O or S, m and n are different in Am-Bn.

In one embodiment of the present specification, when Y1 and Y2 are O, m and n are different in Am-Bn.

In one embodiment of the present specification, when Y1 and Y2 are S, m and n are different in Am-Bn.

In one embodiment of the present specification, when any one of Y1 and Y2 is O and the other one is S, m and n are different in Am-Bn.

In one embodiment of the present specification, when Y1 and Y2 are NR', m and n are the same as or different from each other in Am-Bn.

In one embodiment of the present specification, when any one of Y1 and Y2 is O or S and the other one is NR', m and n are the same as or different from each other in Am-Bn.

In one embodiment of the present specification, Chemical Formula 1 bonds to any one of A1 to A4 of Chemical Formula 1-A and bonds to any one of C1 to C3 of Chemical Formula 1-C, which is represented by Am-Co, m is 1, 2, 3 or 4, o is 1, 2 or 3, and m and o are the same as or different from each other.

For example, when Chemical Formula 1 bonds to A1 of Chemical Formula 1-A and bonds to C1 of Chemical Formula 1-C, it may be represented as A1-C1.

In one embodiment of the present specification, Chemical Formula 1 bonds to any one of A1 to A4 of Chemical Formula 1-A and any one of D1 to D3 of Chemical Formula 1-D, which is represented by Am-Dp, m is 1, 2, 3 or 4, p is 1, 2 or 3, and m and p are the same as or different from each other.

For example, when Chemical Formula 1 bonds to A1 of Chemical Formula 1-A and bonds to D1 of Chemical Formula 1-D, it may be represented as A1-D1.

In one embodiment of the present specification, Chemical Formula 1 may be represented by any one of the following Chemical Formulae 1-1 to 1-7.

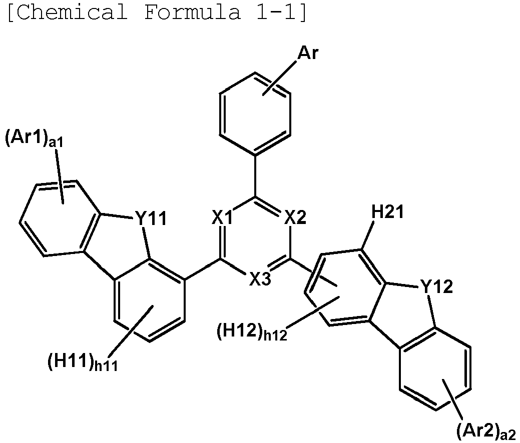

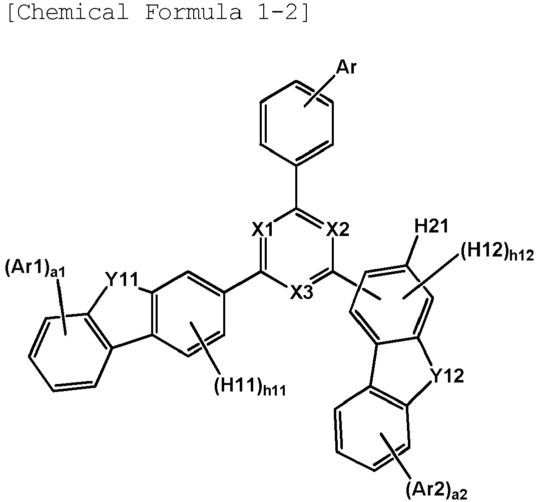

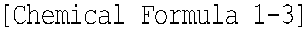

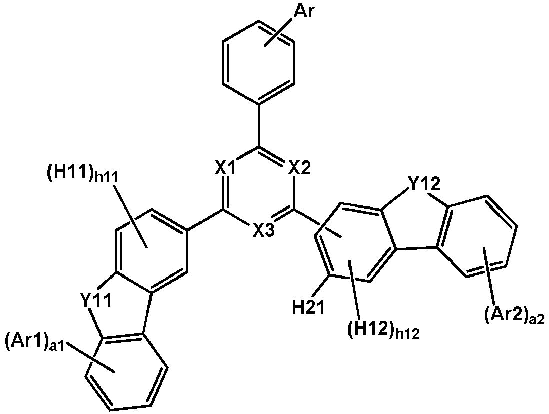

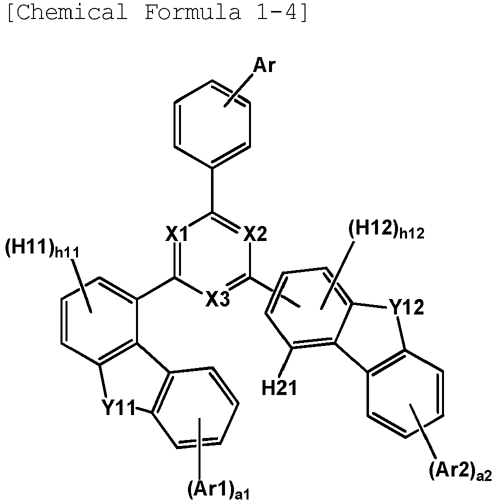

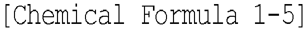

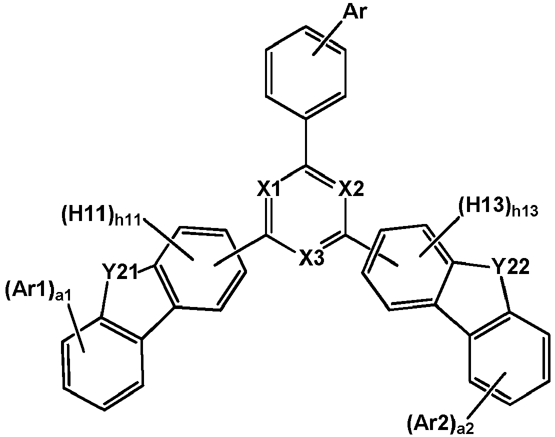

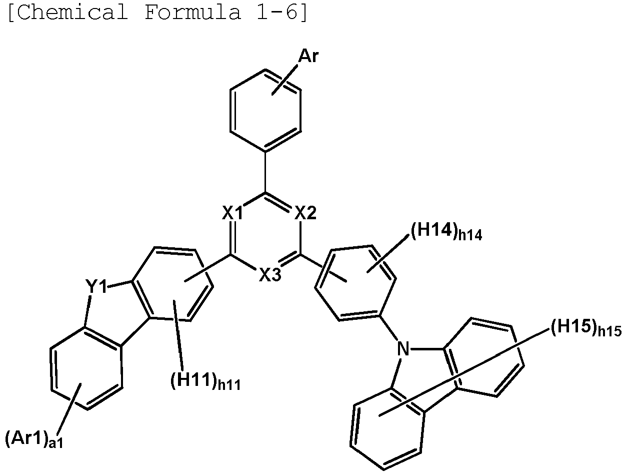

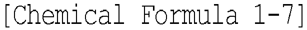

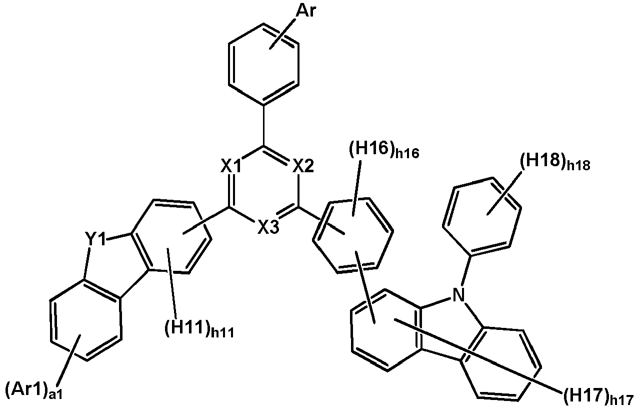

In Chemical Formulae 1-1 to 1-7,
Y11 and Y12 are each independently O or S,
Y21 and Y22 are each independently O; S; or NR', and at least one of Y21 and Y22 is NR',
H11 to H18 and H21 are each independently hydrogen; or deuterium,
h11 and h13 are each an integer of 0 to 3, h12 is an integer of 0 to 2, h14 and h16 are each an integer of 0 to 4, h15 is an integer of 0 to 8, h17 is an integer of 0 to 7 and h18 is an integer of 0 to 5, and when h12 is 2 or h11 and h13 to h18 are each 2 or greater, substituents in the parentheses are the same as or different from each other,
X1 to X3 and Ar each have the same definitions as in Chemical Formula 1, and
Y1, R', Ar1, Ar2, a1 and a2 each have the same definitions as in Chemical Formulae 1-A and 1-B.

In one embodiment of the present specification, Chemical Formula 1 may have a deuterium content of 0% to 100%.

In one embodiment of the present specification, Chemical Formula 1 may have a deuterium content of 0% to 70%.

In one embodiment of the present specification, Chemical Formula 1 may have a deuterium content of 0%, or greater than 0% and less than or equal to 100%.

In one embodiment of the present specification, Chemical Formula 1 may have a deuterium content of 0%, or 10% to 100%, 30% to 100% or 50% to 100%.

In one embodiment of the present specification, Chemical Formula 1 may have a deuterium content of 0%, or greater than 0% and less than or equal to 70%.

In one embodiment of the present specification, Chemical Formula 1 may have a deuterium content of 0%, or 30% to 70%.

In one embodiment of the present specification, Chemical Formula 1 may be represented by any one of the following compounds, but is not limited thereto.

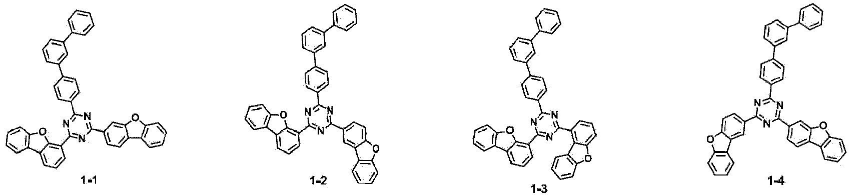

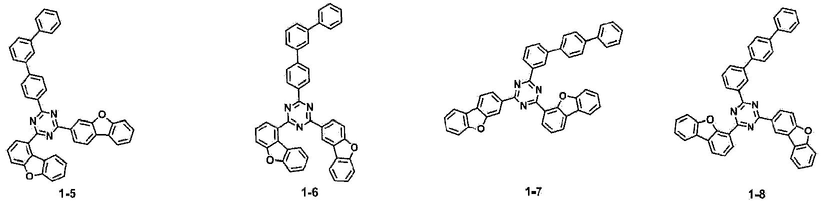

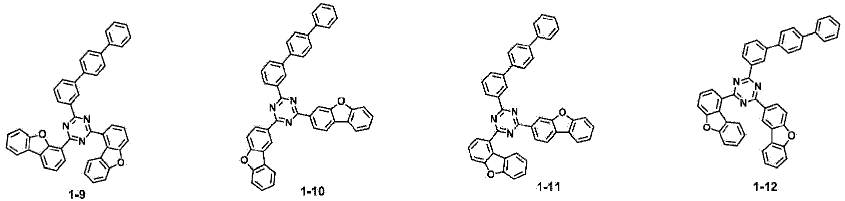

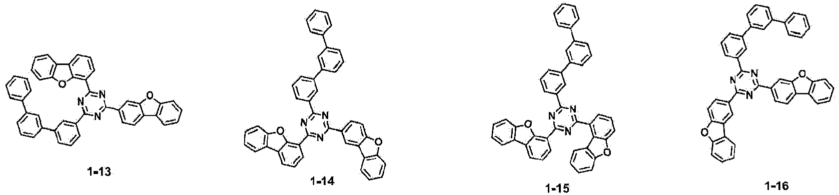

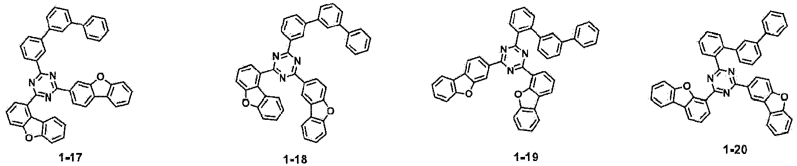

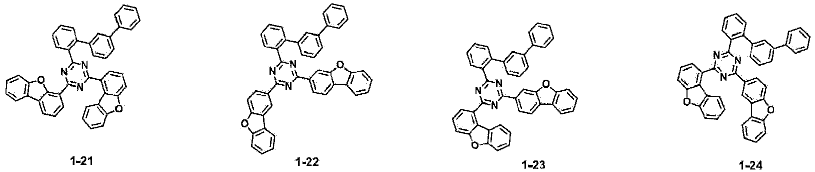

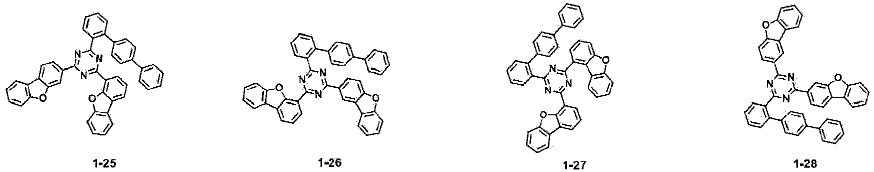

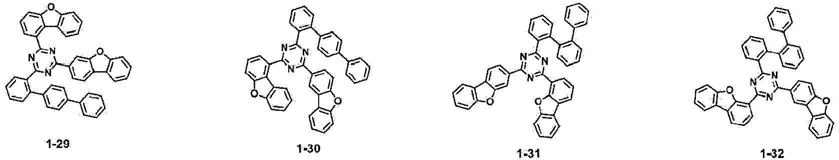

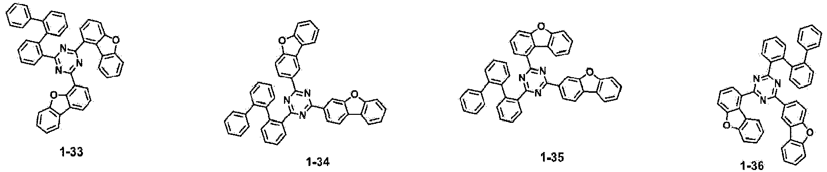

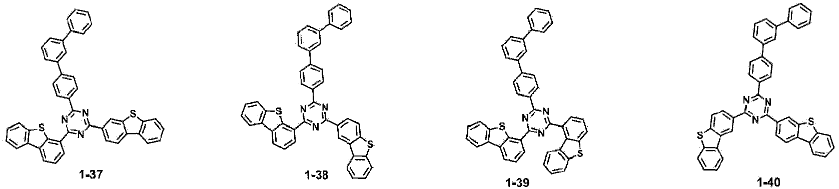

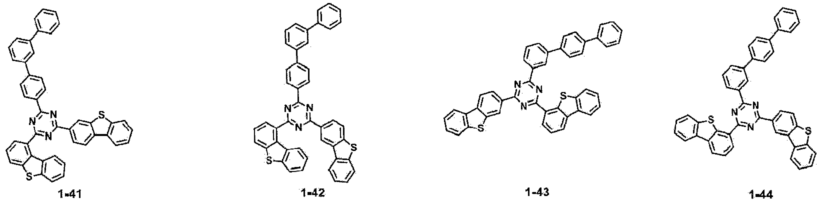

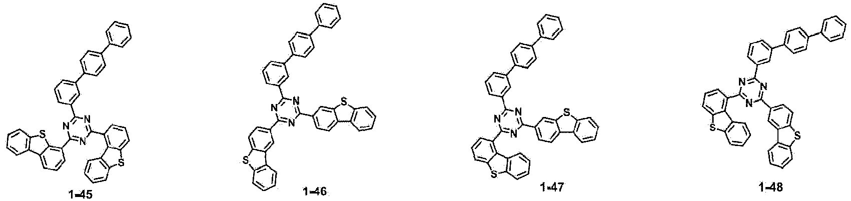

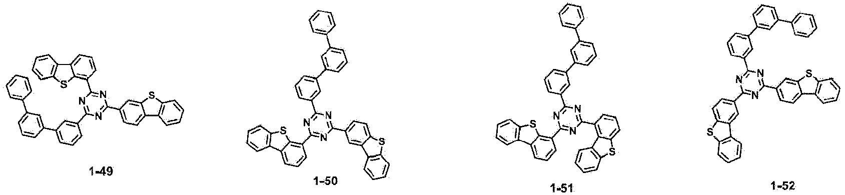

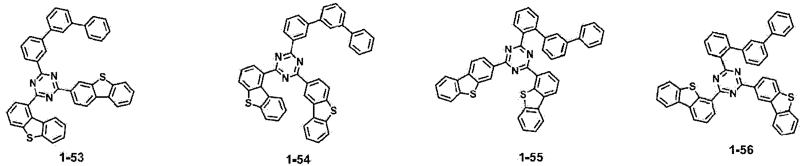

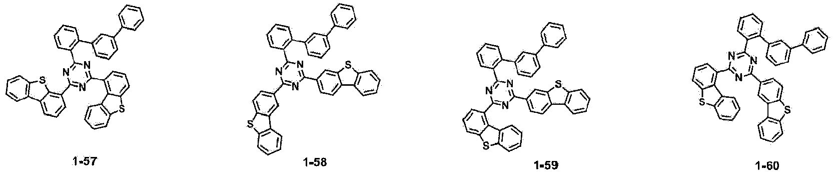

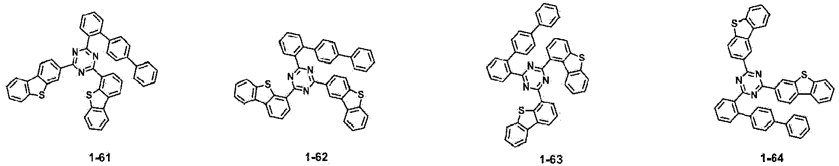

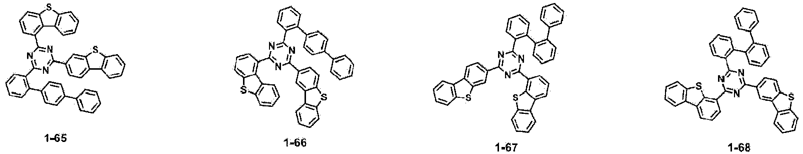

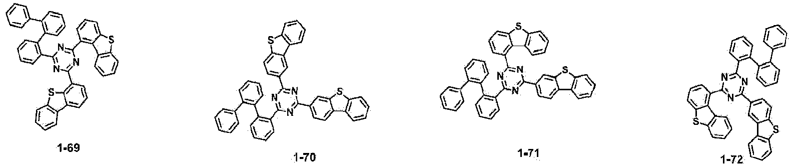

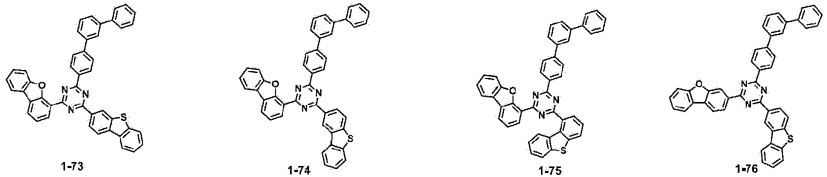

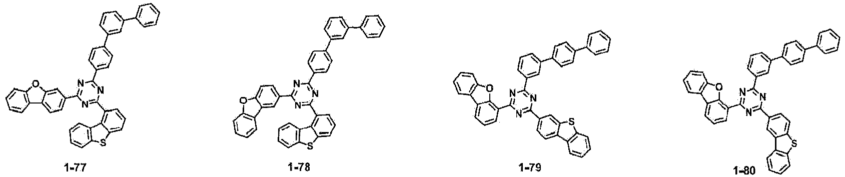

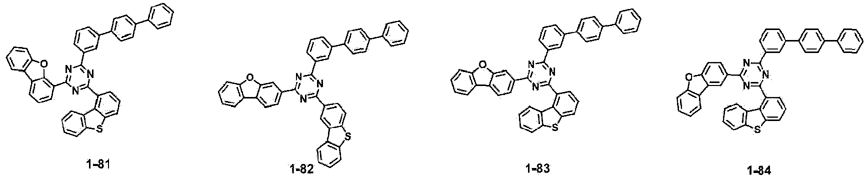

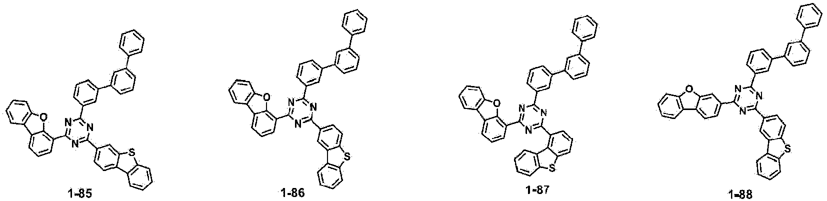

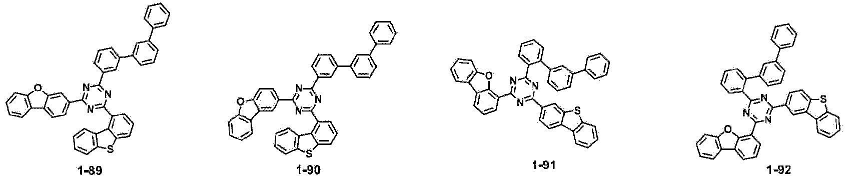

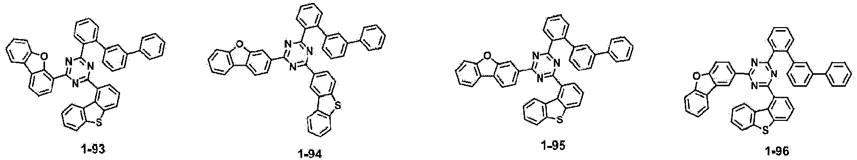

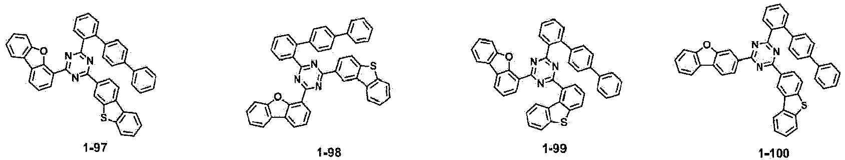

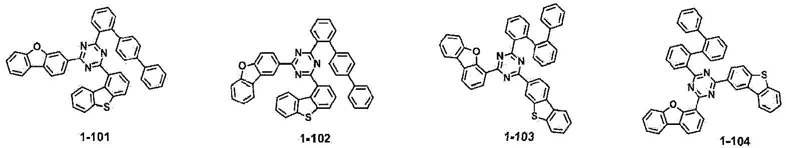

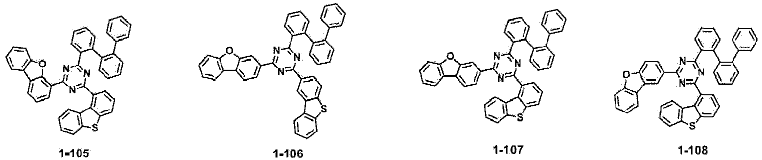

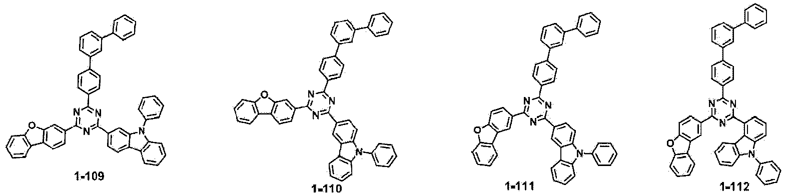

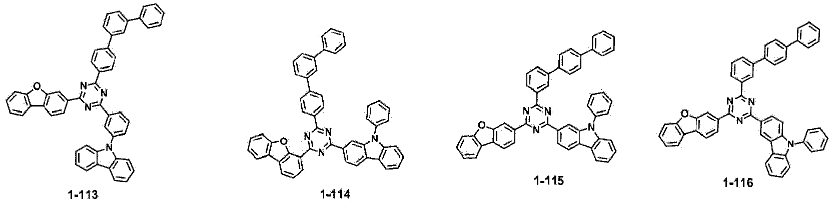

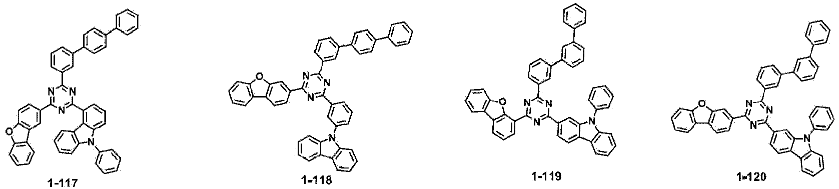

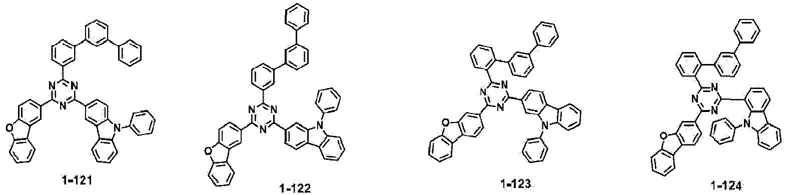

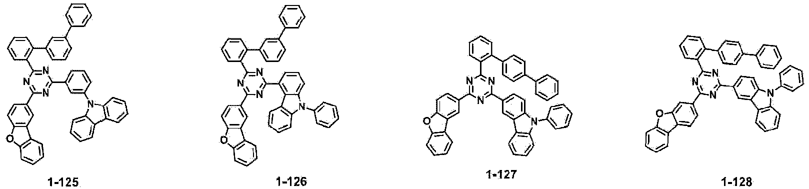

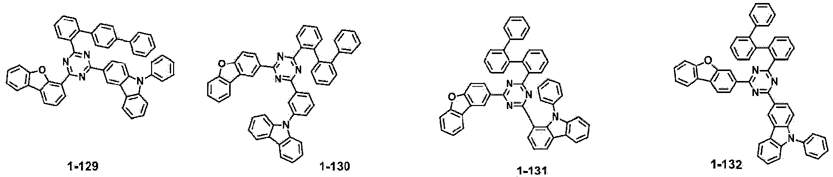

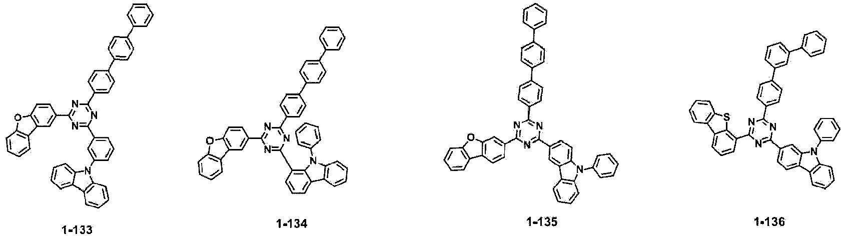

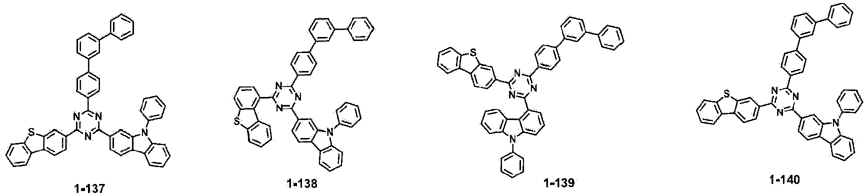

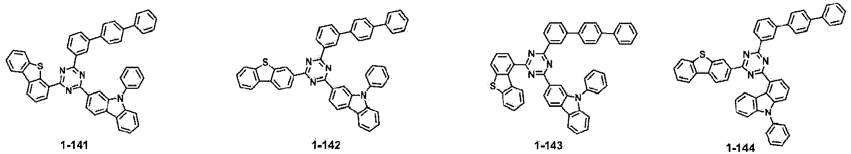

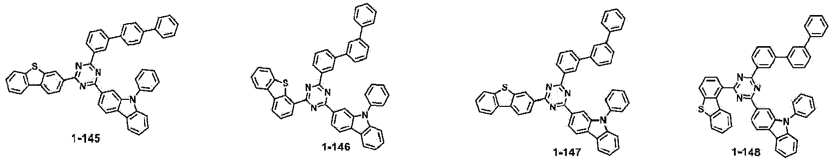

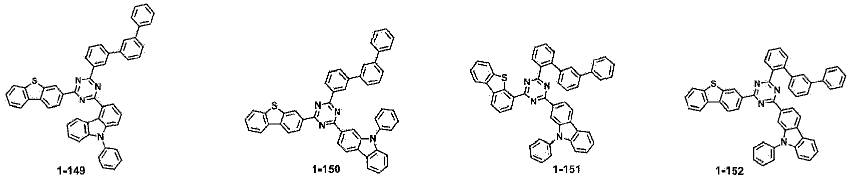

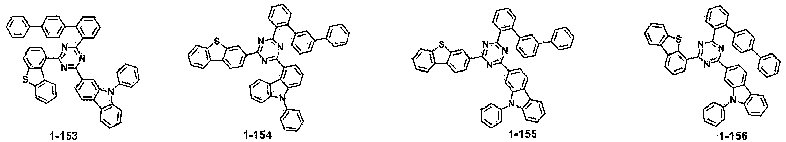

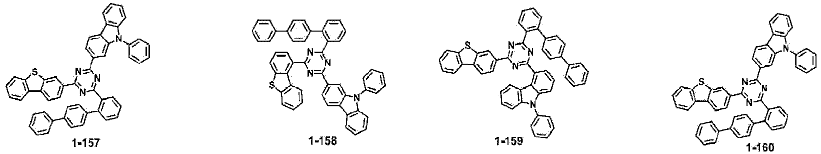

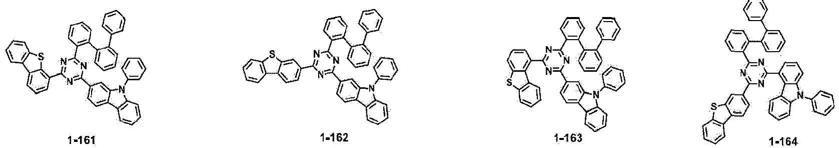

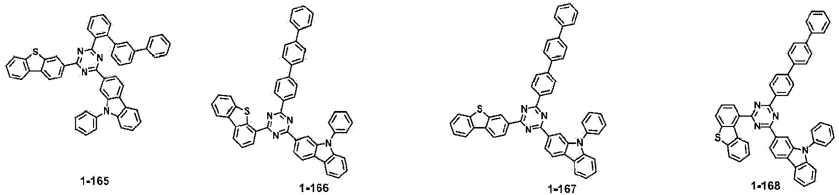

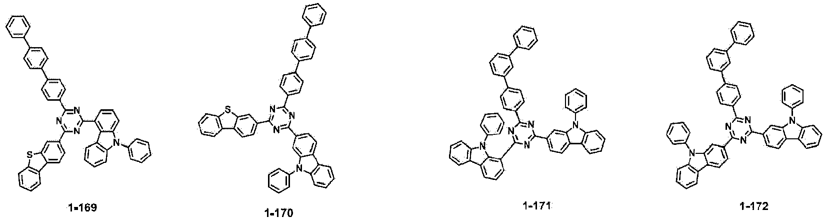

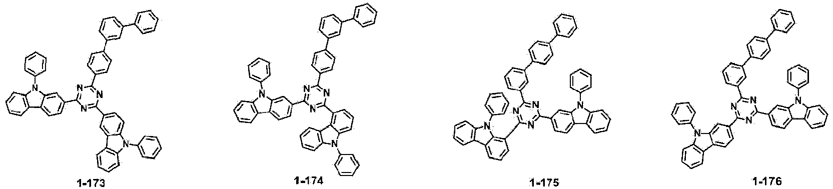

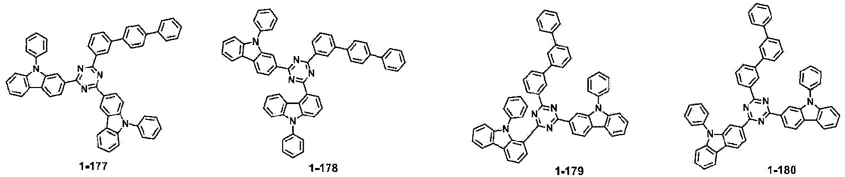

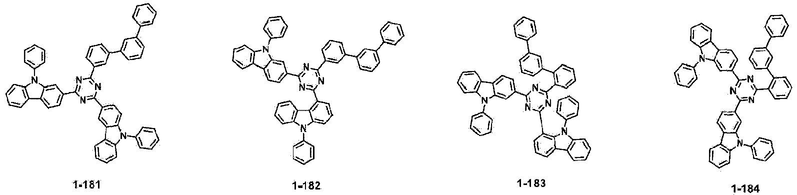

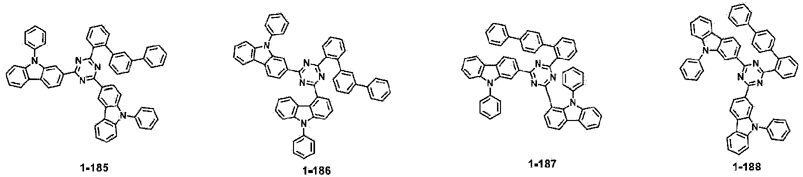

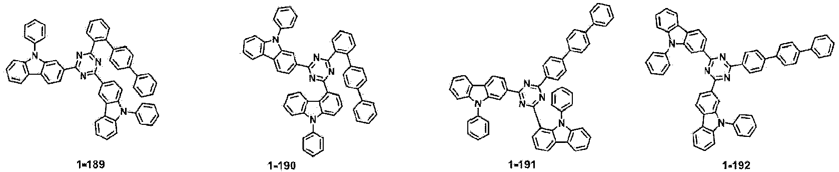

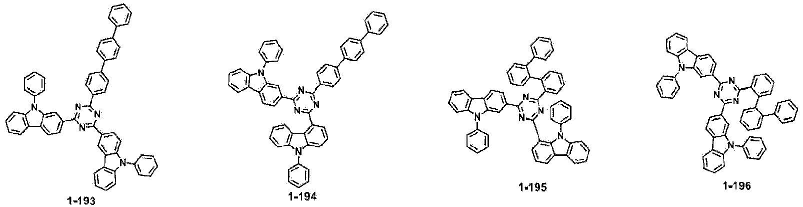

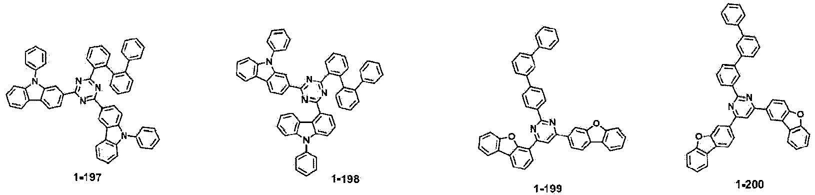

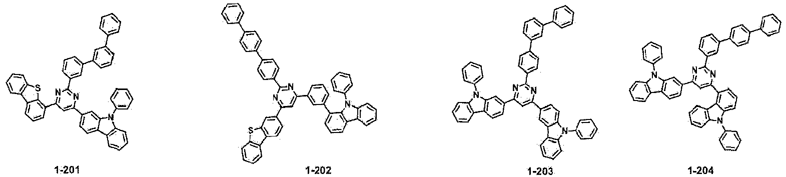

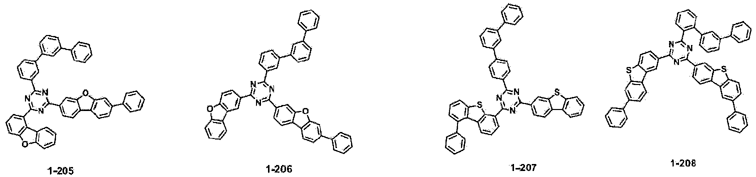

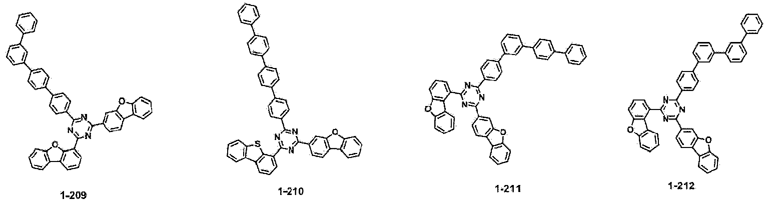

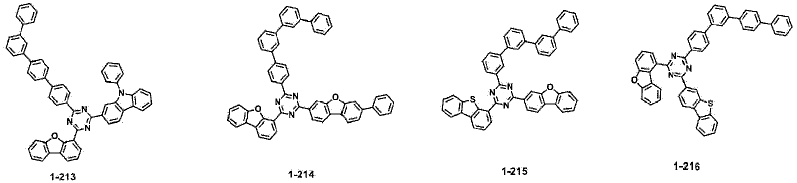

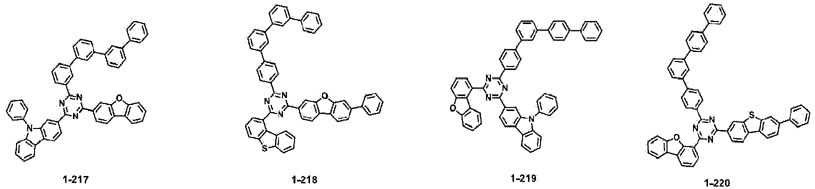

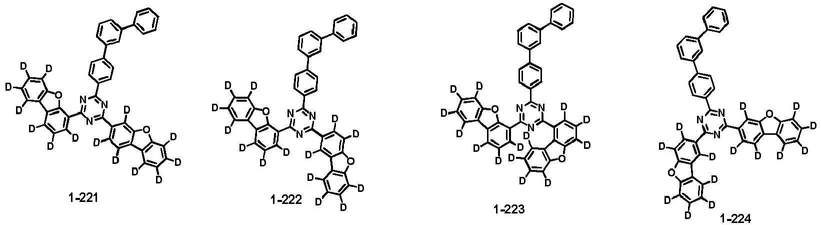

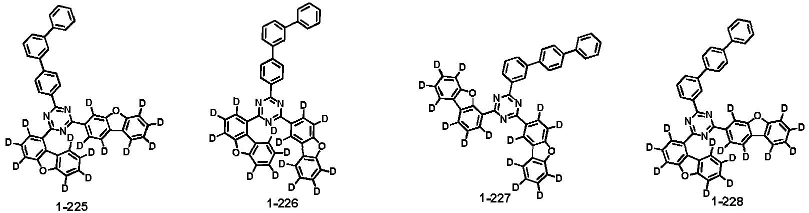

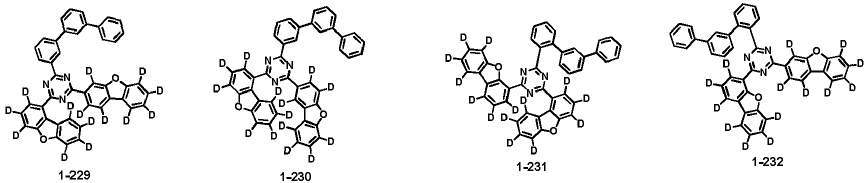

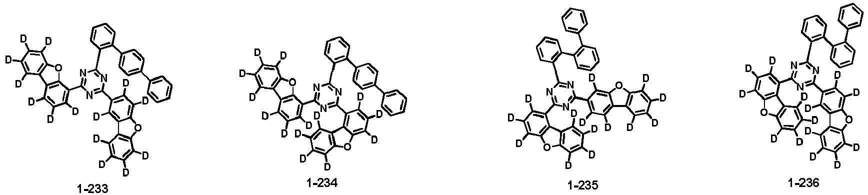

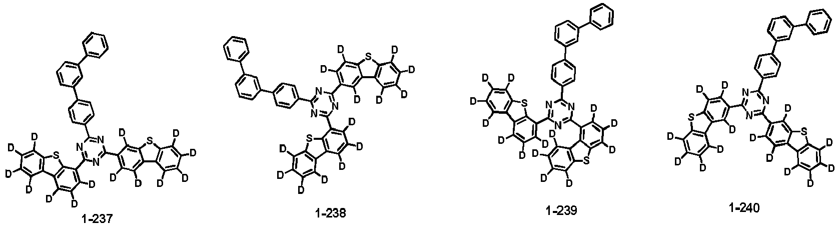

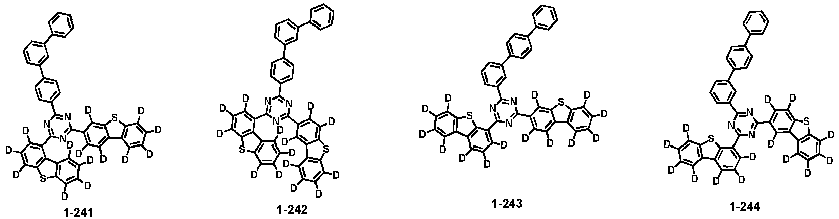

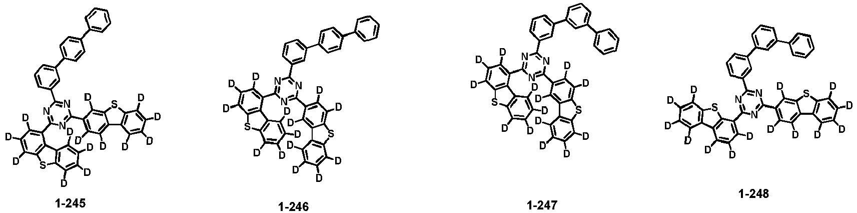

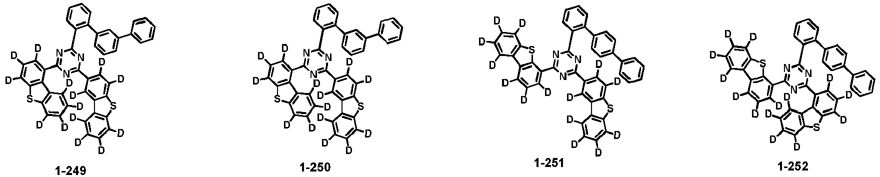

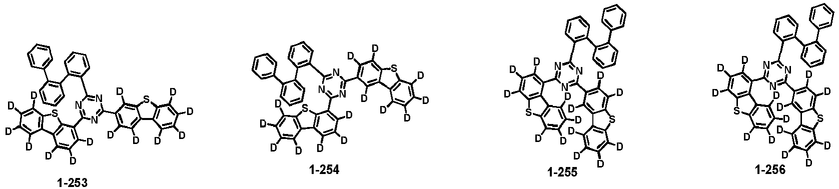

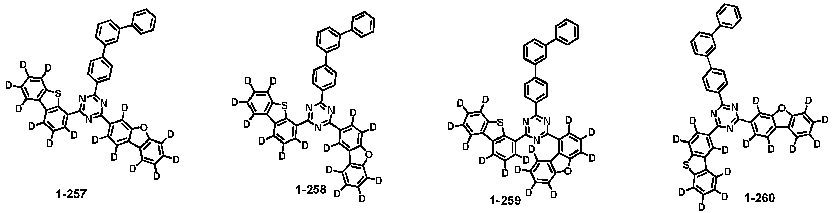

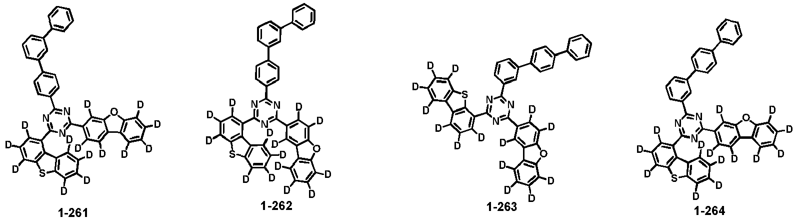

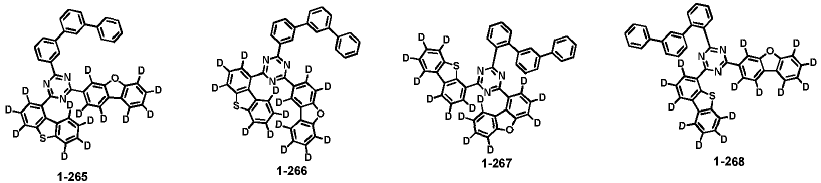

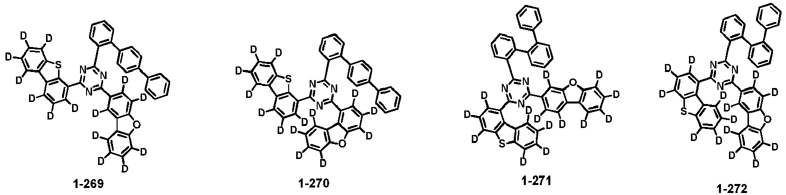

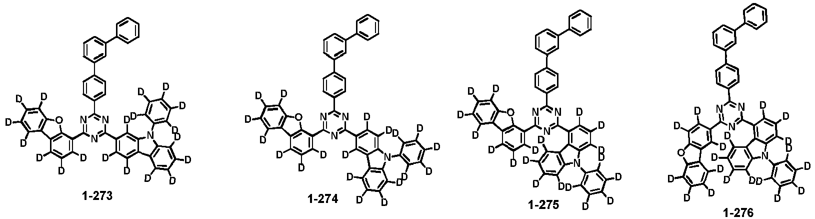

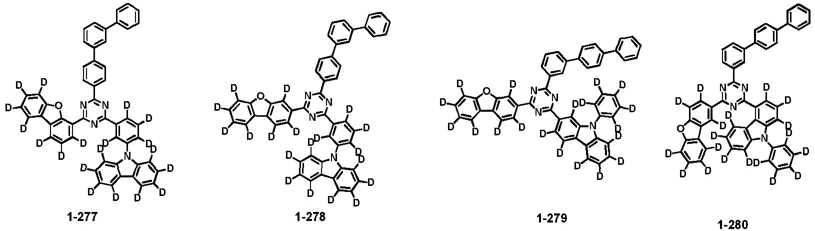

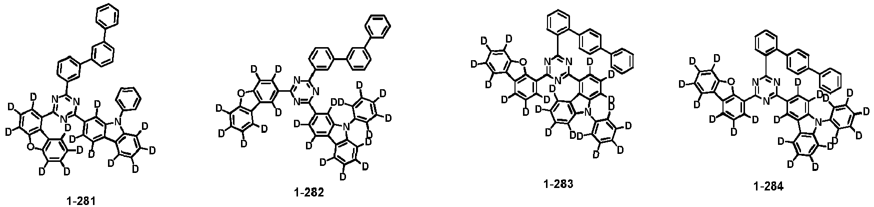

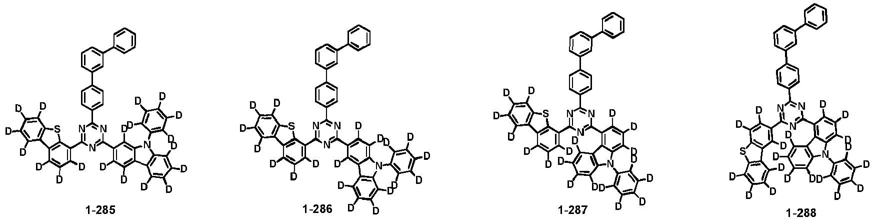

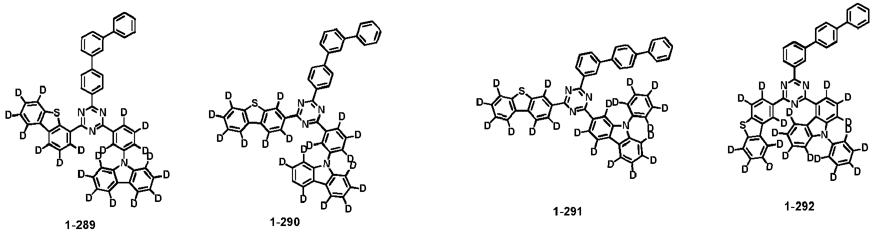

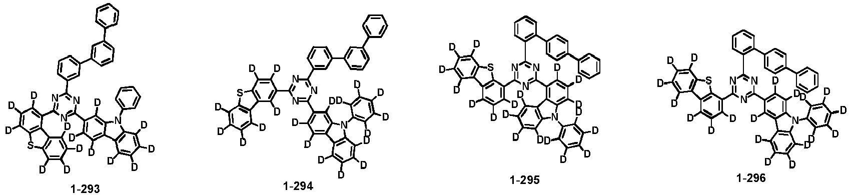

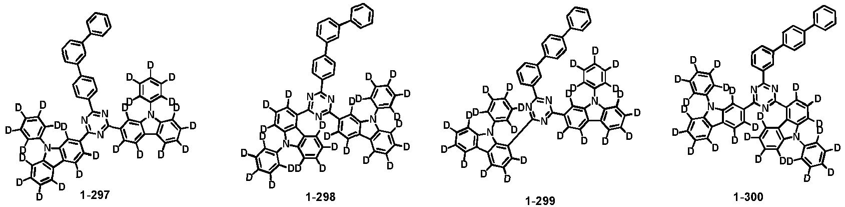

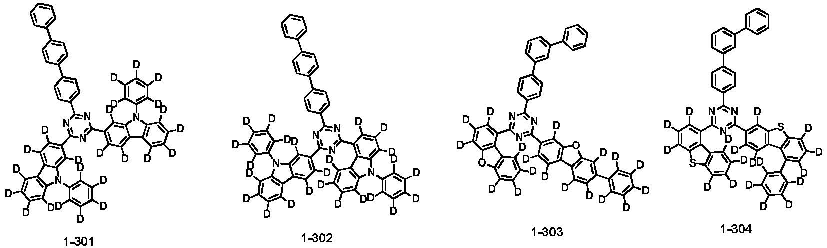

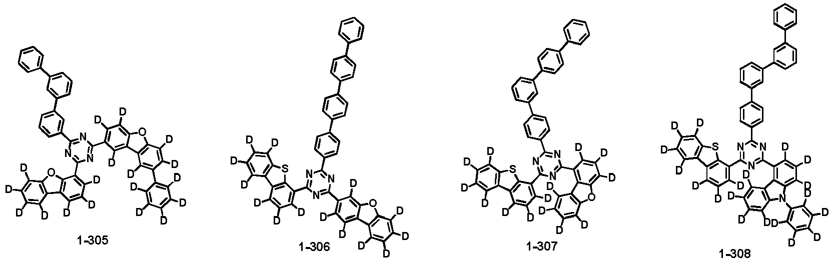

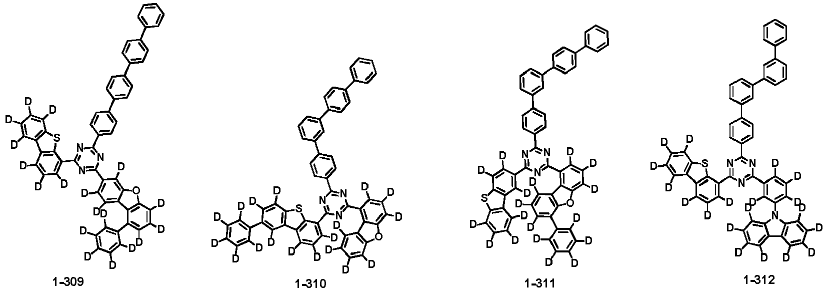

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

One embodiment of the present specification provides an organic light emitting device including a first electrode; a second electrode; and one or more organic material layers provided between the first electrode and the second electrode, wherein one or more layers of the organic material layers include one or more types of the heterocyclic compound of Chemical Formula 1.

In one embodiment of the present specification, one or more layers of the organic material layers include one type of the heterocyclic compound of Chemical Formula 1.

In one embodiment of the present specification, the first electrode may be an anode, and the second electrode may be a cathode.

In another embodiment of the present specification, the first electrode may be a cathode, and the second electrode may be an anode.

In one embodiment of the present specification, the organic light emitting device may be a blue organic light emitting device, and the heterocyclic compound of Chemical Formula 1 may be used as a material of the blue organic light emitting device. For example, the heterocyclic compound of Chemical Formula 1 may be included in a light emitting layer of the blue organic light emitting device.

In one embodiment of the present specification, the organic light emitting device may be a green organic light emitting device, and the heterocyclic compound of Chemical Formula 1 may be used as a material of the green organic light emitting device. For example, the heterocyclic compound of Chemical Formula 1 may be included in a light emitting layer of the green organic light emitting device.

In one embodiment of the present specification, the organic light emitting device may be a red organic light emitting device, and the heterocyclic compound of Chemical Formula 1 may be used as a material of the red organic light emitting device. For example, the heterocyclic compound of Chemical Formula 1 may be included in a light emitting layer of the red organic light emitting device.

The organic light emitting device of the present specification may be manufactured using common organic light emitting device manufacturing methods and materials except that one or more organic material layers are formed using the compound described above.

The compound may be formed into an organic material layer using a solution coating method as well as a vacuum deposition method when manufacturing the organic light emitting device. Herein, the solution coating method means spin coating, dip coating, inkjet printing, screen printing, a spray method, roll coating and the like, but is not limited thereto.

The organic material layer of the organic light emitting device of the present specification may be formed in a single layer structure, but may be formed in a multilayer structure in which two or more organic material layers are laminated. For example, the organic light emitting device of the present disclosure may have a structure including a hole injection layer, a hole transfer layer, a light emitting layer, an electron transfer layer, an electron injection layer and the like as the organic material layer. However, the structure of the organic light emitting device is not limited thereto, and may include a smaller number of organic material layers.

In the organic light emitting device of the present specification, the organic material layer includes a light emitting layer, and the light emitting layer may include the heterocyclic compound of Chemical Formula 1.

In the organic light emitting device of the present specification, the organic material layer includes a light emitting layer, the light emitting layer includes a host, and the host may include the heterocyclic compound of Chemical Formula 1.

In the organic light emitting device of the present specification, the organic material layer includes a light emitting layer, and the light emitting layer may further include, in addition to the heterocyclic compound of Chemical Formula 1, a compound of the following Chemical Formula 2.

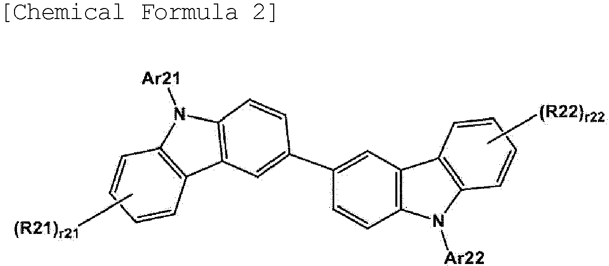

In Chemical Formula 2,
R21 and R22 are each independently hydrogen; deuterium; a halogen group; a cyano group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
Ar21 and Ar22 are each independently a substituted or unsubstituted C6 to C60 aryl group; or a substituted or unsubstituted C2 to C60 heteroaryl group,
r21 is an integer of 0 to 4, and when 2 or greater, R21s are the same as or different from each other, and
r22 is an integer of 0 to 4, and when 2 or greater, R22s are the same as or different from each other.

In one embodiment of the present specification, R21 and R22 of Chemical Formula 2 are each independently hydrogen; deuterium; a substituted or unsubstituted C6 to C60 aryl group; or a substituted or unsubstituted C2 to C60 heteroaryl group.

In one embodiment of the present specification, R21 and R22 are each independently hydrogen; deuterium; a substituted or unsubstituted C6 to C30 aryl group; or a substituted or unsubstituted C2 to C30 heteroaryl group.

In one embodiment of the present specification, R21 and R22 are each independently hydrogen; or a substituted or unsubstituted C6 to C30 aryl group.

In one embodiment of the present specification, R21 and R22 are hydrogen; or deuterium.

In one embodiment of the present specification, Ar21 and Ar22 of Chemical Formula 2 are each independently a substituted or unsubstituted C6 to C40 aryl group; or a substituted or unsubstituted C2 to C40 heteroaryl group.

In one embodiment of the present specification, Ar21 and Ar22 are each independently a substituted or unsubstituted C6 to C40 aryl group.

In one embodiment of the present specification, Ar21 and Ar22 are each independently a substituted or unsubstituted C6 to C30 aryl group.

In one embodiment of the present specification, Ar21 and Ar22 are each independently a substituted or unsubstituted phenyl group; a substituted or unsubstituted biphenyl group; a substituted or unsubstituted terphenyl group; a substituted or unsubstituted naphthyl group; a substituted or unsubstituted fluorenyl group; or a substituted or unsubstituted triphenylene group.

In one embodiment of the present specification, Ar21 and Ar22 are each independently a phenyl group unsubstituted or substituted with a cyano group, a silyl group or an aryl group; a biphenyl group; a terphenyl group; a naphthyl group; a fluorenyl group unsubstituted or substituted with an alkyl group or an aryl group; 9,9'-spirobi[fluorene]; or a triphenylene group.

In one embodiment of the present specification, Ar21 and Ar22 are each independently a phenyl group unsubstituted or substituted with a cyano group, a triphenylsilyl group or an aryl group; a biphenyl group; a terphenyl group; a naphthyl group; a fluorenyl group unsubstituted or substituted with an alkyl group or an aryl group; 9,9'-spirobi[fluorene]; or a triphenylene group.

In one embodiment of the present specification, Chemical Formula 2 may be represented by any one of the following compounds, but is not limited thereto.

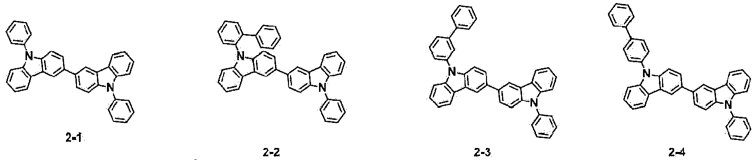

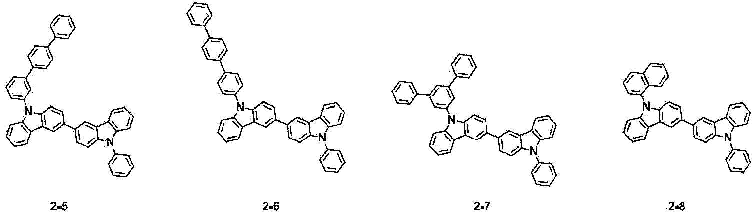

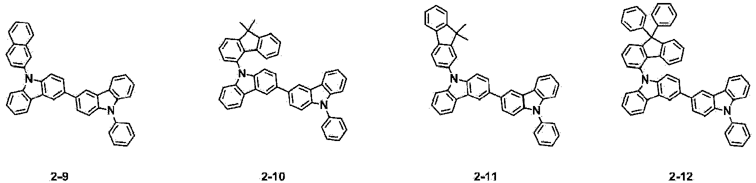

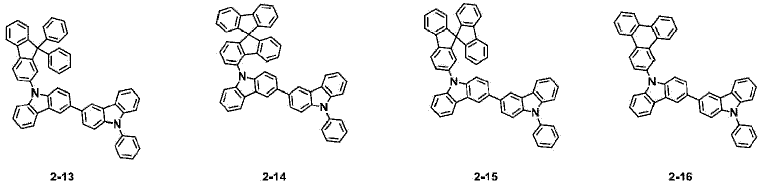

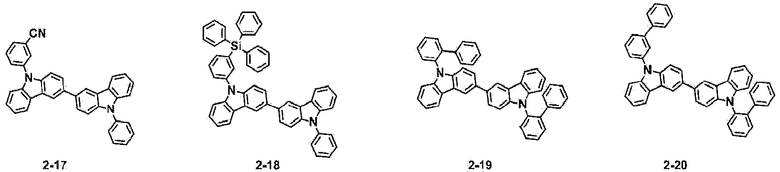

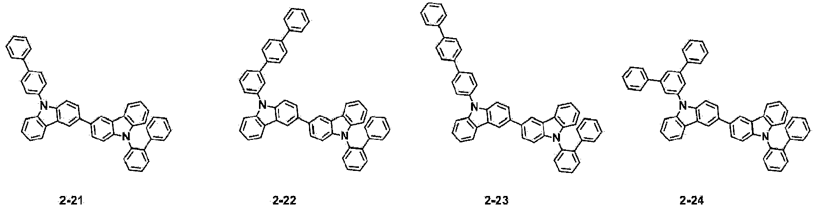

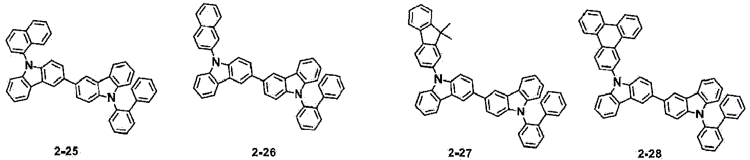

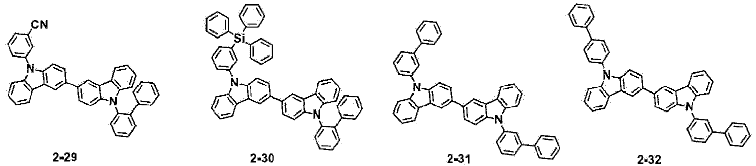

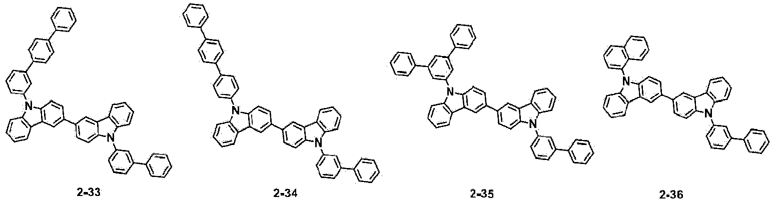

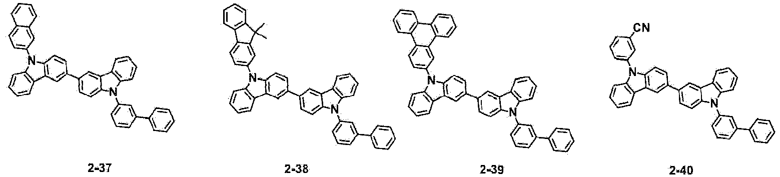

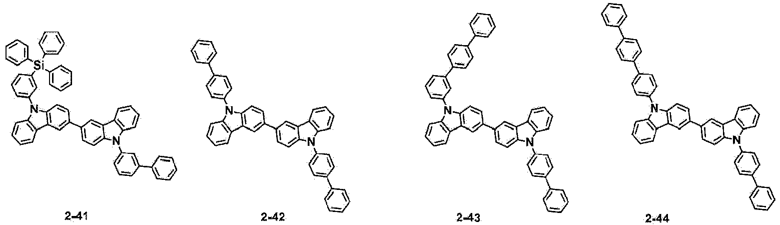

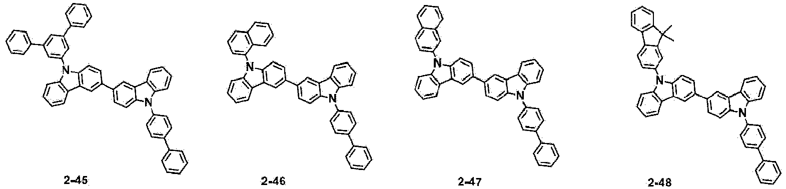

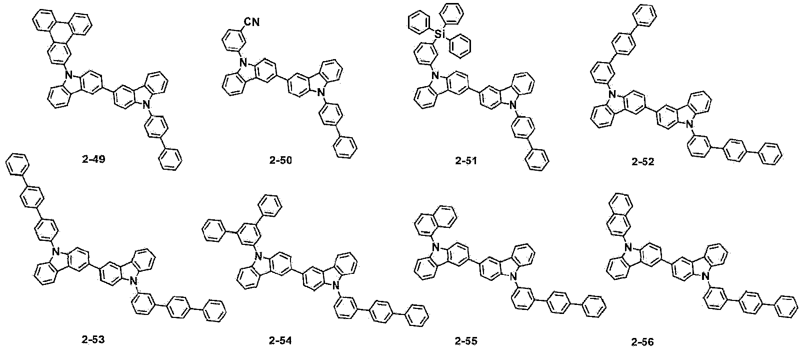

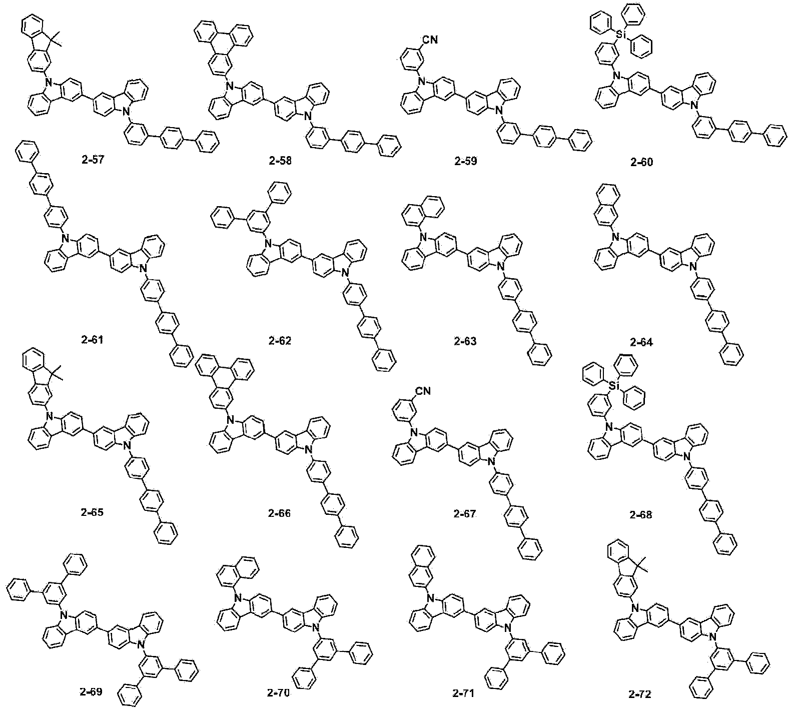

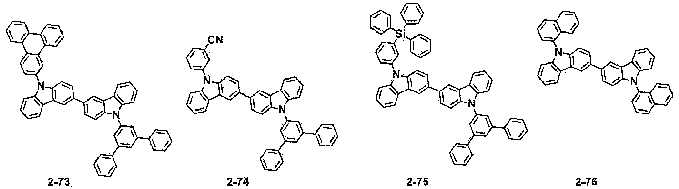

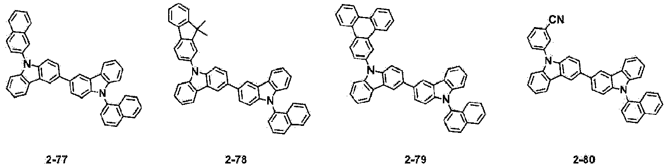

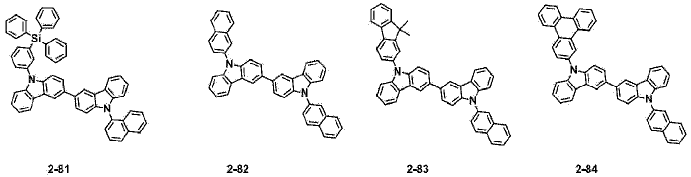

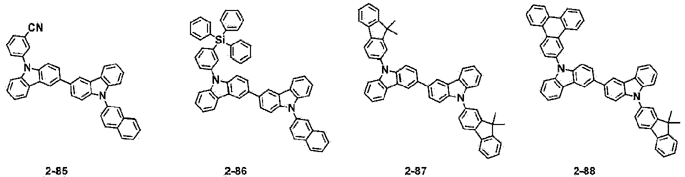

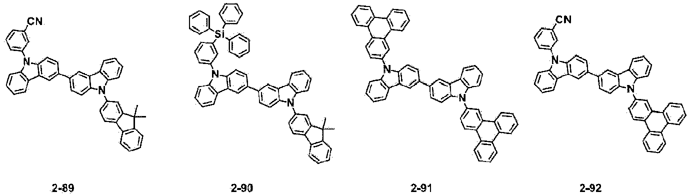

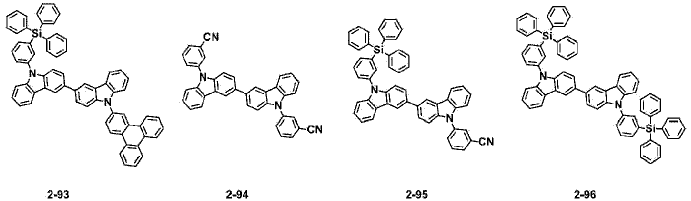

The organic light emitting device of the present disclosure may further include one, two or more layers selected from the group consisting of a light emitting layer, a hole injection layer, a hole transfer layer, an electron injection layer, an electron transfer layer, an electron blocking layer and a hole blocking layer.

FIG. 1 to FIG. 3 illustrate a lamination order of electrodes and organic material layers of the organic light emitting device according to one embodiment of the present specification. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

FIG. 3 illustrates a case of the organic material layer being a multilayer. The organic light emitting device according to FIG. 3 includes a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be included, and other necessary functional layers may be further added.

The organic material layer including the heterocyclic compound of Chemical Formula 1 may further include other materials as necessary.

In the organic light emitting device according to one embodiment of the present specification, materials other than the heterocyclic compound of Chemical Formula 1 are illustrated below, however, these are for illustrative purposes only and not for limiting the scope of the present application, and these materials may be replaced by materials known in the art.

As the anode material, materials having relatively large work function may be used, and transparent conductive oxides, metals, conductive polymers or the like may be used. Specific examples of the anode material include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrenesulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used in addition to the heterocyclic compound of Chemical Formula 1, and as necessary, two or more light emitting materials may be mixed and used. Herein, two or more light emitting materials may be used by being deposited as individual sources of supply or by being premixed and deposited as one source of supply. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

In the present specification, the heterocyclic compound of Chemical Formula 1 may be used as the host material, and Ir(ppy)₃ (tris(2-phenylpyridine)iridium) may be used as the dopant material.

When mixing light emitting material hosts, same series hosts may be mixed, or different series hosts may be mixed. For example, any two or more types of materials among N-type host materials or P-type host materials may be selected and used as a host material of a light emitting layer.

In the present specification, the heterocyclic compound of Chemical Formula 1 may be used as the N-type host material, and the compound of Chemical Formula 2 may be used as the P-type host material.

The organic light emitting device according to one embodiment of the present specification may be a top-emission type, a bottom-emission type or a dual-emission type depending on the materials used.

The compound according to one embodiment of the present specification may also be used in an organic electronic device including an organic solar cell, an organic photo conductor, an organic transistor and the like under a similar principle used in the organic light emitting device.

One embodiment of the present specification provides a composition for forming an organic material layer, the composition including the heterocyclic compound of Chemical Formula 1; and the compound of Chemical Formula 2.

The composition for forming an organic material layer according to one embodiment of the present specification includes the heterocyclic compound of Chemical Formula 1 and the compound of Chemical Formula 2 in a weight ratio of 1:10 to 10:1, a weight ratio of 1:8 to 8:1, a weight ratio of 1:5 to 5:1, or a weight ratio of 1:2 to 2:1.

When the heterocyclic compound of Chemical Formula 1 and the compound of Chemical Formula 2 are included in the weight ratio of the above-mentioned range, an organic light emitting device having a low driving voltage and excellent light emission efficiency and lifetime may be provided. Particularly, when included in a weight ratio of 1:2 to 2:1, the organic light emitting device has significantly enhanced driving voltage, light emission efficiency and lifetime properties.

The composition for forming an organic material layer according to one embodiment of the present specification may be used as a light emitting layer material of an organic light emitting device.

Hereinafter, the present specification will be described in more detail with reference to examples, however, these are for illustrative purposes only, and the scope of the present application is not limited thereto.

### <Preparation Example>

### <Preparation Example 1> Preparation of Compound 1-1 [D]

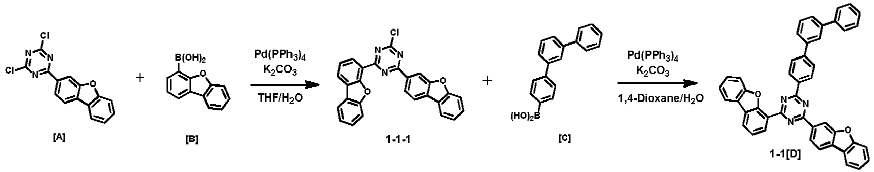

### Preparation of Compound 1-1-1

In a one-neck round bottom flask, a mixture of 2,4-dichloro-6-(dibenzo[b,d]furan-3-yl)-1,3,5-triazine [A] (10 g, 0.032 mol), dibenzo[b,d]furan-4-ylboronic acid [B] (7.46 g, 0.035 mol), Pd(PPh₃)₄ (tetrakis(triphenylphosphine)palladium(0)) (1.85 g, 0.0016 mol), potassium carbonate (K₂CO₃) (8.85 g, 0.064 mol) and tetrahydrofuran (THF) (100 mL)/water (H₂O) (30 mL) was refluxed at 120°C. The result was extracted with dichloromethane (DCM), concentrated, and then silica gel filtered. The result was concentrated, and then treated with dichloromethane/methanol to obtain Compound 1-1-1 (8.03 g, 56%) .

### Preparation of Compound 1-1[D]

In a one-neck round bottom flask, a mixture of Compound 1-1-1 (8.03 g, 0.018 mol), [1,1':3',1"-terphenyl]-4-ylboronic acid (5.43 g, 0.020 mol) [C], Pd(PPh₃)₄ (1.04 g, 0.0009 mol), potassium carbonate (4.98 g, 0.036 mol) and 1,4-dioxane (80 mL)/water (H₂O) (24 mL) was refluxed at 125°C. After the reaction was completed, precipitated solids were filtered. The solids obtained as above were dissolved in dichlorobenzene, and silica gel filtered. The result was concentrated, and then treated with methanol to obtain Compound 1-1 [D] (9.01 g, 78%).

The following Compound D was synthesized in the same manner except that Intermediates A, B and C of the following Table 1 were respectively used instead of 2,4-dichloro-6-(dibenzo[b,d]furan-3-yl)-1,3,5-triazine [A], dibenzo[b,d]furan-4-ylboronic acid [B] and [1,1':3',1"-terphenyl]-4-ylboronic acid [C].

**[Table 1]**

| Compou nd | Intermediate A | Intermedia te B | Intermedia te C | Compound D | Yield |
|---|---|---|---|---|---|
| 1-1 | 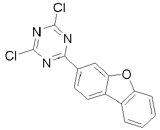 | 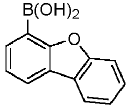 | 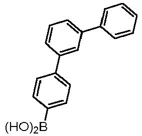 | 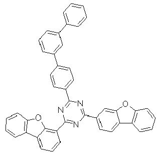 | 78% |
| 1-4 | 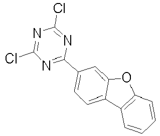 | 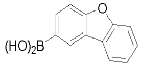 | 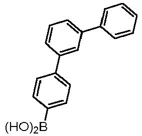 | 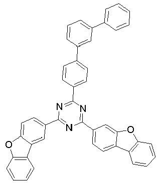 | 83% |
| 1-8 | 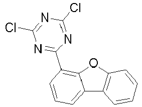 | 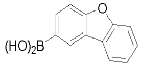 | 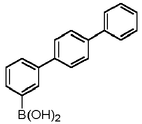 | 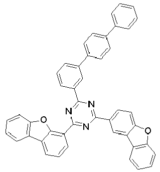 | 70% |
| 1-10 | 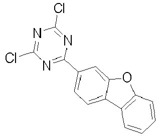 | 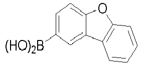 | 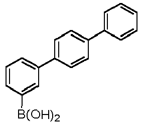 | 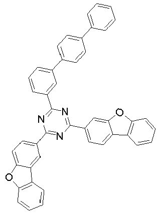 | 74% |
| 1-15 | 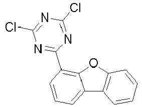 | 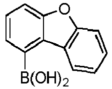 | 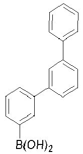 | 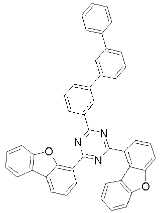 | 65% |
| 1-16 | 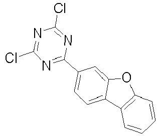 | 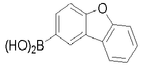 | 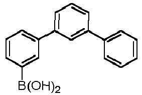 | 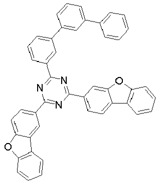 | 67% |
| 1-19 | 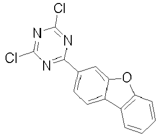 | 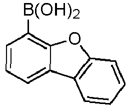 | 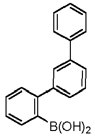 | 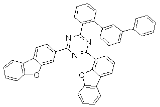 | 88% |
| 1-26 | 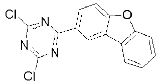 | 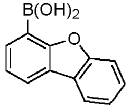 | 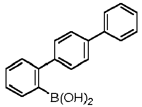 | 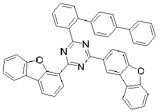 | 80% |
| 1-28 | 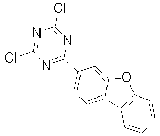 | 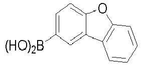 | 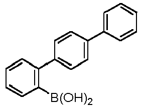 | 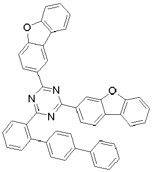 | 83% |
| 1-32 | 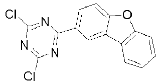 | 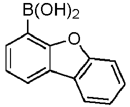 | 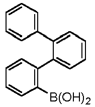 | 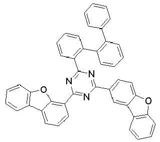 | 71% |
| 1-34 | 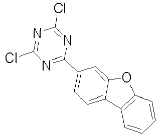 | 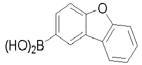 | 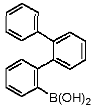 | 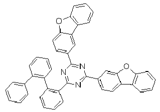 | 68% |
| 1-37 | 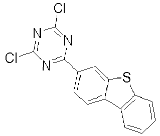 | 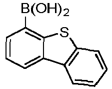 | 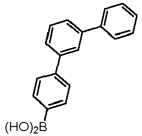 | 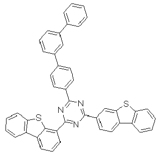 | 76% |
| 1-40 | 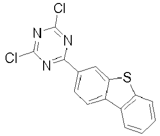 | 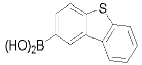 | 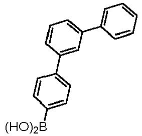 | 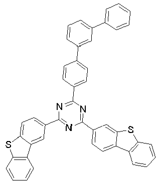 | 75% |
| 1-41 | 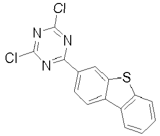 | 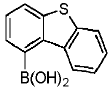 | 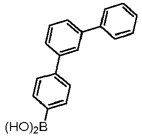 | 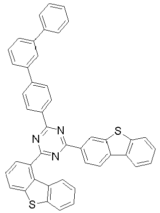 | 85% |
| 1-44 | 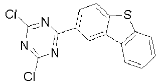 | 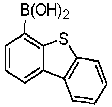 | 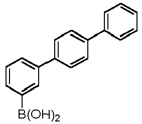 | 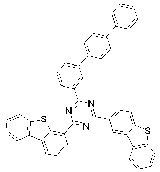 | 70% |
| 1-48 | 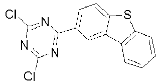 | 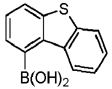 | 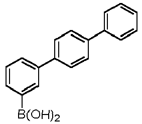 | 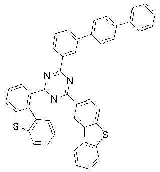 | 65% |
| 1-52 | 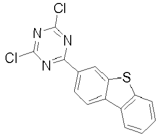 | 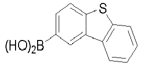 | 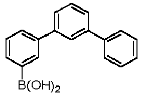 | 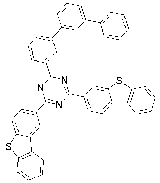 | 61% |
| 1-57 | 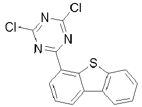 | 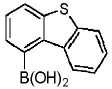 | 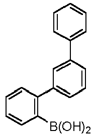 | 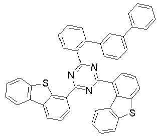 | 69% |
| 1-61 | 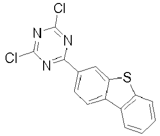 | 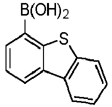 | 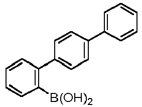 | 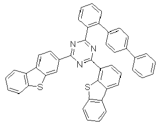 | 65% |
| 1-68 | 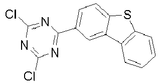 | 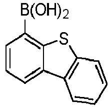 | 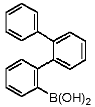 | 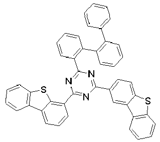 | 75% |
| 1-72 | 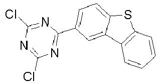 | 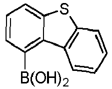 | 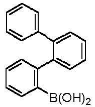 | 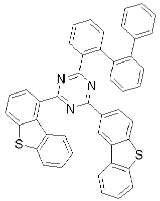 | 83% |
| 1-73 | 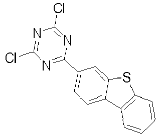 | 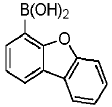 | 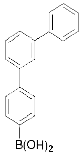 | 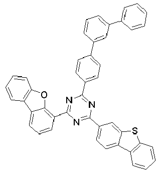 | 74% |
| 1-79 | 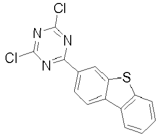 | 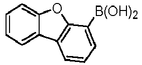 | 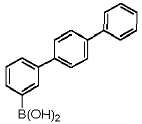 | 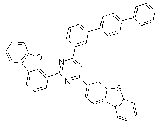 | 76% |
| 1-81 | 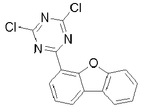 | 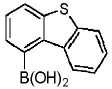 | 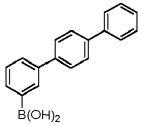 | 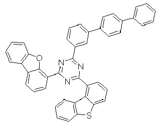 | 82% |
| 1-86 | 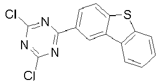 | 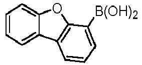 | 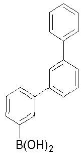 | 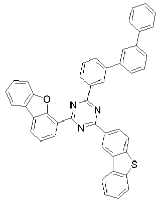 | 85% |
| 1-90 | 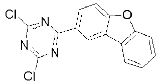 | 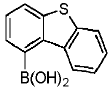 | 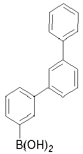 | 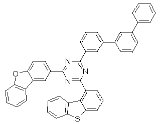 | 81% |
| 1-92 | 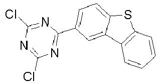 | 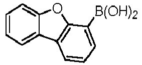 | 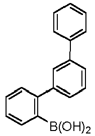 | 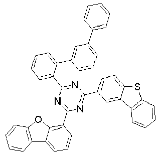 | 84% |
| 1-95 | 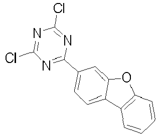 | 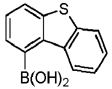 | 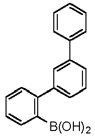 | 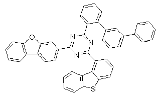 | 59% |
| 1-99 | 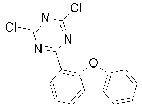 | 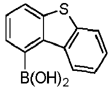 | 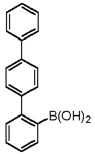 | 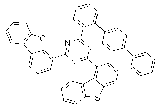 | 65% |
| 1-100 | 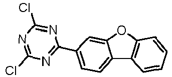 | 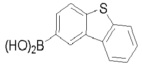 | 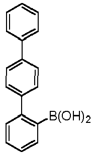 | 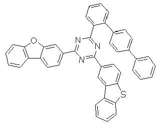 | 60% |
| 1-103 | 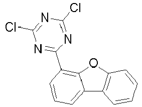 | 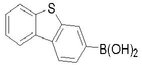 | 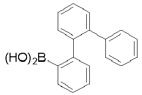 | 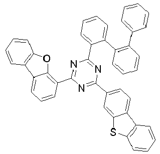 | 70% |
| 1-105 | 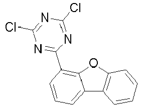 | 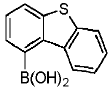 | 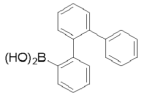 | 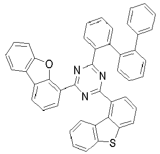 | 81% |
| 1-108 | 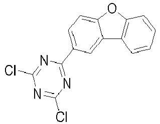 | 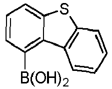 | 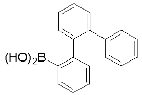 | 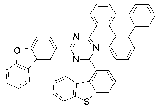 | 69% |
| 1-109 | 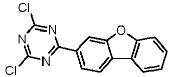 | 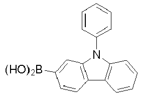 | 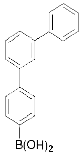 | 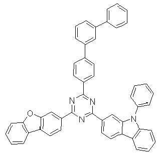 | 61% |
| 1-112 | 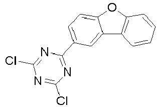 | 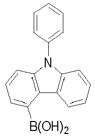 | 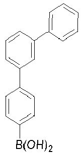 | 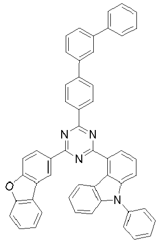 | 54% |
| 1-115 | 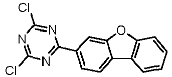 | 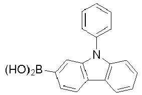 | 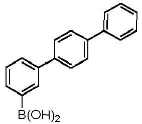 | 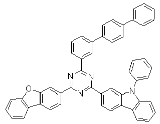 | 88% |
| 1-119 | 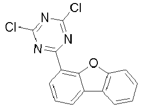 | 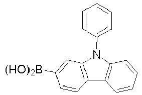 | 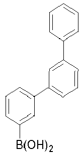 | 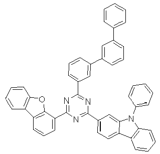 | 87% |
| 1-121 | 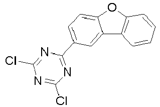 | 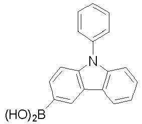 | 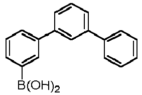 | 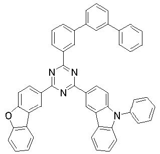 | 93% |
| 1-125 | 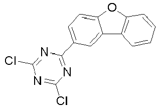 | 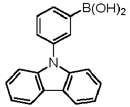 | 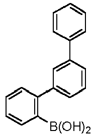 | 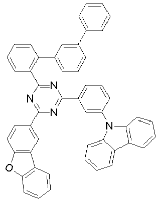 | 76% |
| 1-128 | 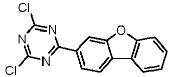 | 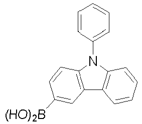 | 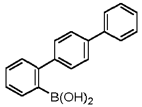 | 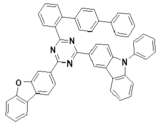 | 71% |
| 1-131 | 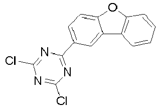 | 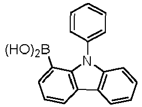 | 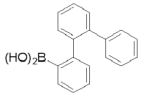 | 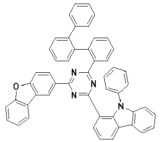 | 63% |
| 1-134 | 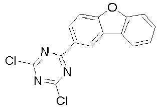 | 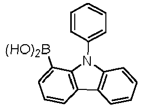 | 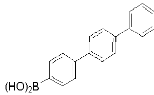 | 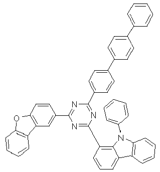 | 69% |
| 1-135 | 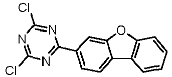 | 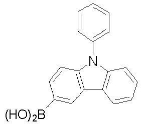 | 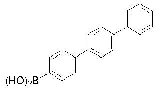 | 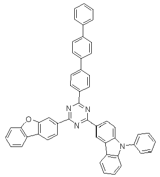 | 96% |
| 1-139 | 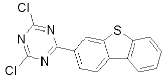 | 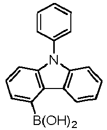 | 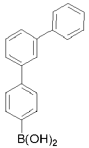 | 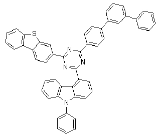 | 90% |
| 1-140 | 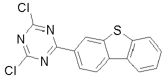 | 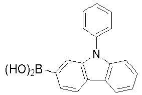 | 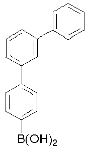 | 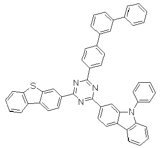 | 86% |
| 1-144 | 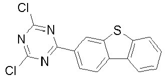 | 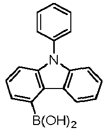 | 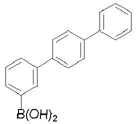 | 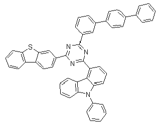 | 87% |
| 1-146 | 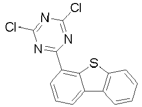 | 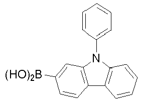 | 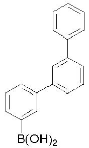 | 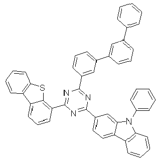 | 93% |
| 1-148 | 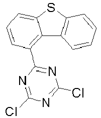 | 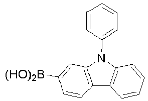 | 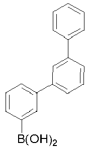 | 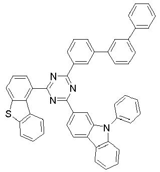 | 87% |
| 1-155 | 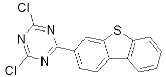 | 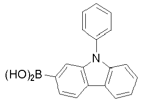 | 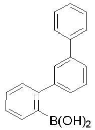 | 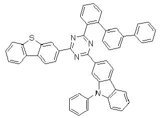 | 88% |
| 1-160 | 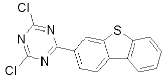 | 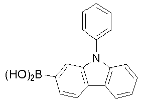 | 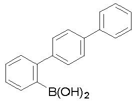 | 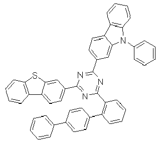 | 80% |
| 1-161 | 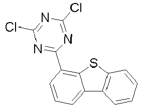 | 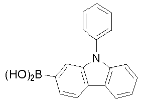 | 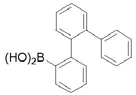 | 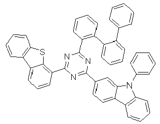 | 74% |
| 1-167 | 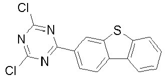 | 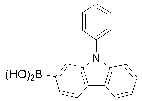 | 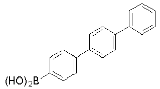 | 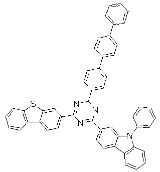 | 96% |
| 1-172 | 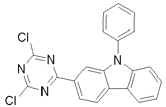 | 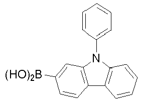 | 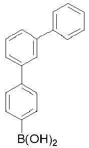 | 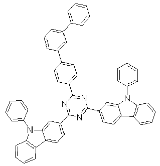 | 97% |
| 1-176 | 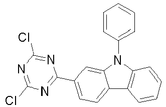 | 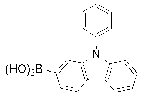 | 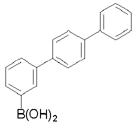 | 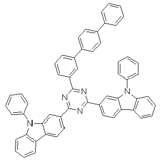 | 92% |
| 1-179 | 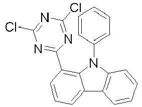 | 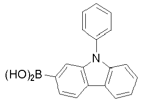 | 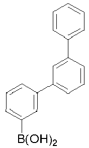 | 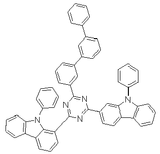 | 80% |
| 1-180 | 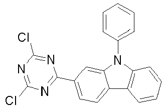 | 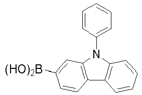 | 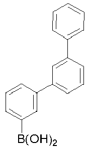 | 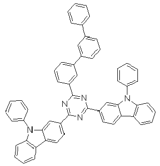 | 92% |
| 1-182 | 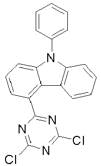 | 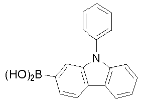 | 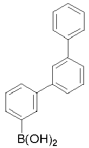 | 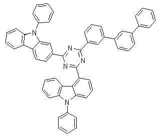 | 71% |
| 1-188 | 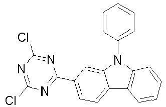 | 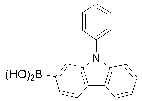 | 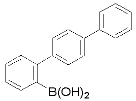 | 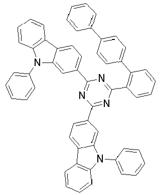 | 60% |
| 1-189 | 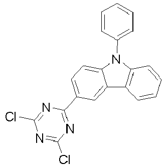 | 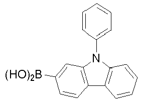 | 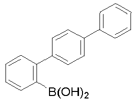 | 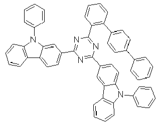 | 63% |
| 1-192 | 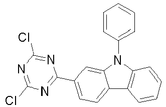 | 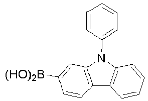 | 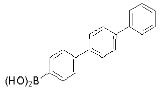 | 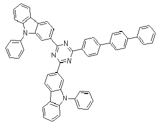 | 87% |
| 1-194 | 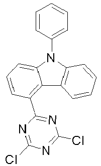 | 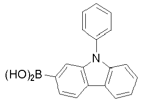 | 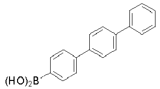 | 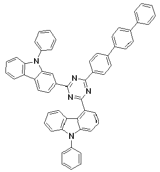 | 85% |
| 1-198 | 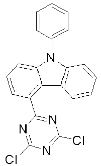 | 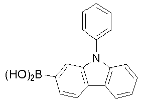 | 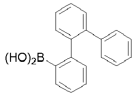 | 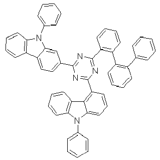 | 59% |
| 1-221 | 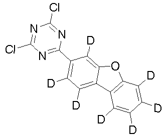 | 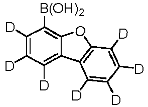 | 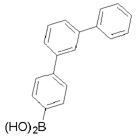 | 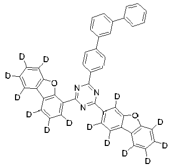 | 73% |
| 1-223 | 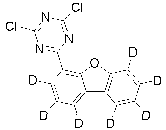 | 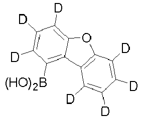 | 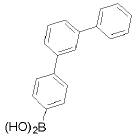 | 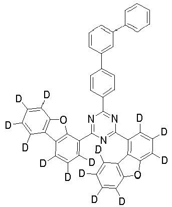 | 69% |
| 1-230 | 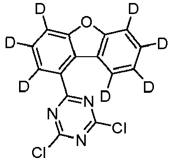 | 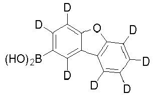 | 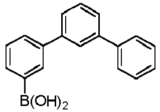 | 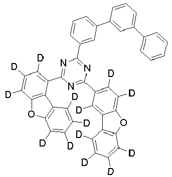 | 79% |
| 1-232 | 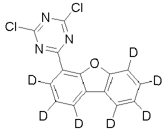 | 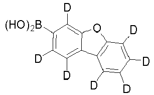 | 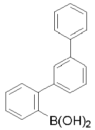 | 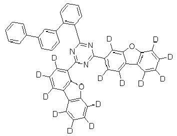 | 83% |
| 1-238 | 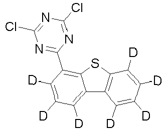 | 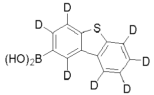 | 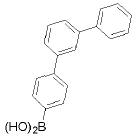 | 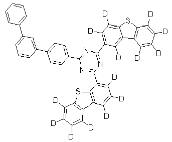 | 71% |
| 1-242 | 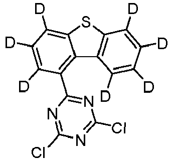 | 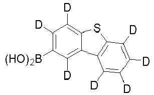 | 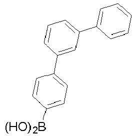 | 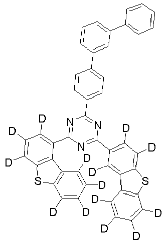 | 65% |
| 1-244 | 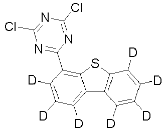 | 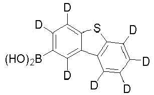 | 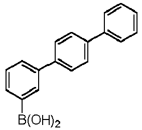 | 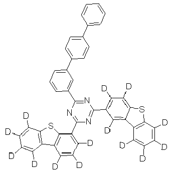 | 94% |
| 1-250 | 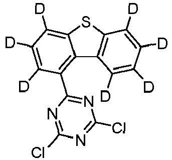 | 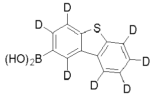 | 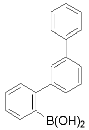 | 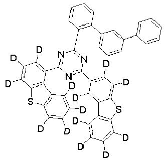 | 77% |
| 1-259 | 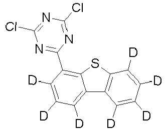 | 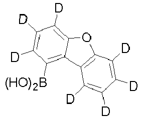 | 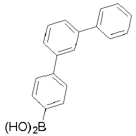 | 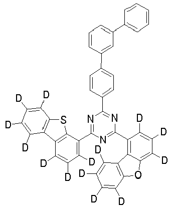 | 72% |
| 1-260 | 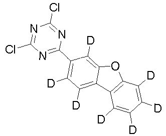 | 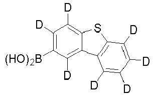 | 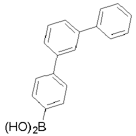 | 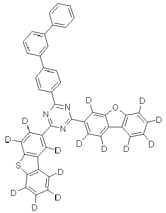 | 83% |
| 1-266 | 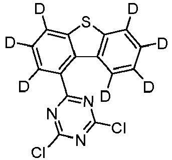 | 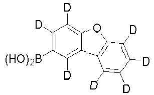 | 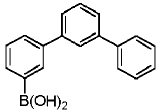 | 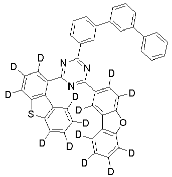 | 76% |
| 1-272 | 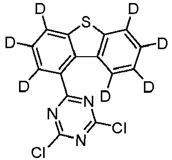 | 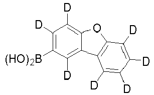 | 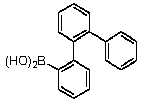 | 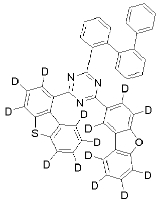 | 83% |
| 1-273 | 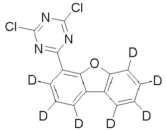 | 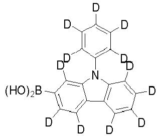 | 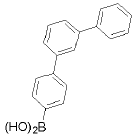 | 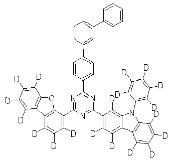 | 85% |
| 1-276 | 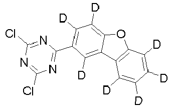 | 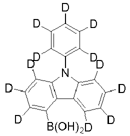 | 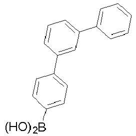 | 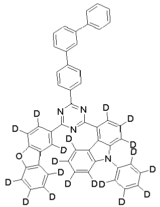 | 91% |
| 1-277 | 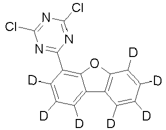 | 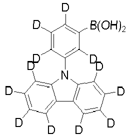 | 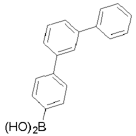 | 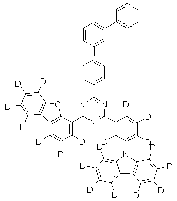 | 79% |
| 1-278 | 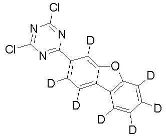 | 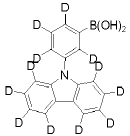 | 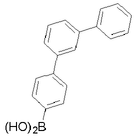 | 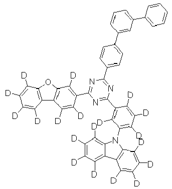 | 88% |
| 1-281 | 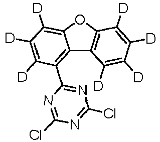 | 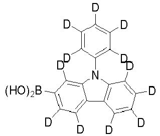 | 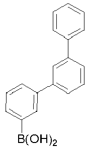 | 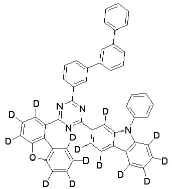 | 69% |
| 1-286 | 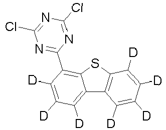 | 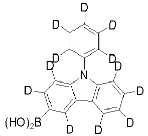 | 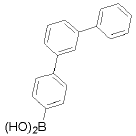 | 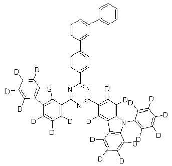 | 66% |
| 1-291 | 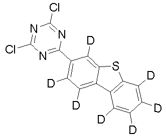 | 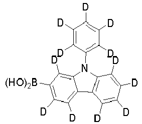 | 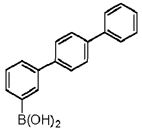 | 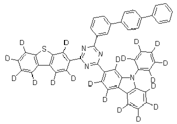 | 73% |
| 1-294 | 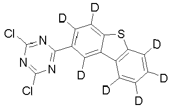 | 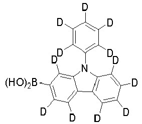 | 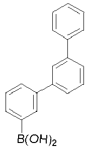 | 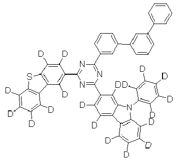 | 53% |
| 1-297 | 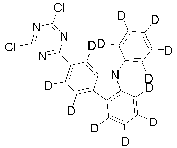 | 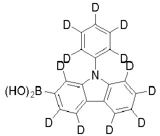 | 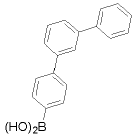 | 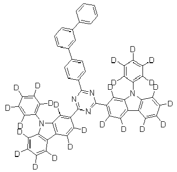 | 68% |
| 1-301 | 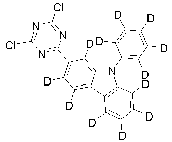 | 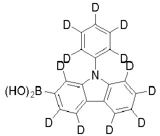 | 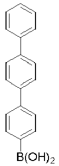 | 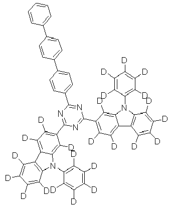 | 74% |
| 1-303 | 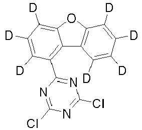 | 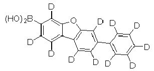 | 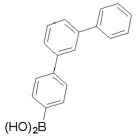 | 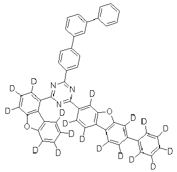 | 55% |
| 1-304 | 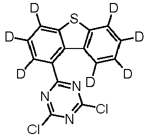 | 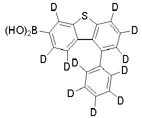 | 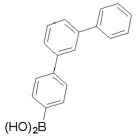 | 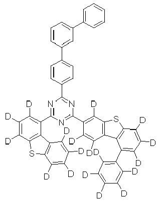 | 62% |

### <Preparation Example 2> Preparation of Compound 2-23 [G]

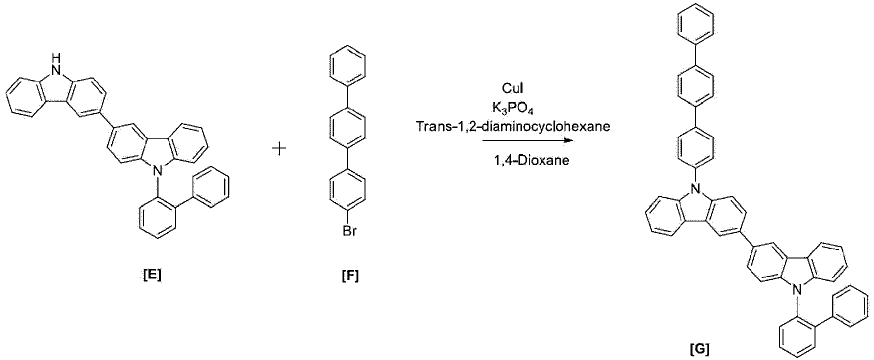

### Preparation of Compound 2-23 [G]

In a one-neck round bottom flask, 9-([1,1'-biphenyl]-2-yl)-9H,9'H-3,3'-bicarbazole [E] (10 g, 0.021 mol), 4-bromo-1,1':4',1"-terphenyl [F] (7.14 g, 0.023 mol), CuI (4.00 g, 0.021 mol), trans-1,2-diaminocyclohexane (2.40 g, 0.021 mol) and K₃PO₄ (8.92 g, 0.042 mol) were dissolved in 1,4-dioxane (100 mL), and refluxed for 8 hours at 125°C. After the reaction was completed, the result was extracted by introducing distilled water and DCM thereto at room temperature, and after drying the organic layer with MgS0₄, the solvent was removed using a rotary evaporator. The reaction material was purified by column chromatography (DCM:hexane=1:3), and recrystallized with methanol to obtain Compound 2-23 [G] (13.17 g, 88%).

The following Compound G was synthesized in the same manner except that Intermediates E and F of the following Table 2 were respectively used instead of 9-([1,1'-biphenyl]-2-yl)-9H,9'H-3,3'-bicarbazole and 4-bromo-1,1':4',1"-terphenyl.

**[Table 2]**

| Compou nd | Intermediate E | Intermediate F | Compound G | Yield |
|---|---|---|---|---|
| 2-23 | 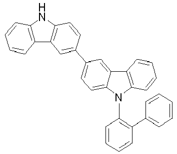 | 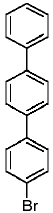 | 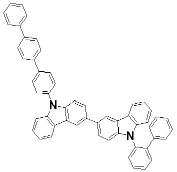 | 88% |
| 2-32 | 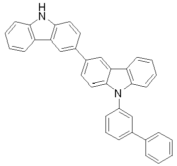 | 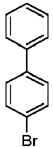 | 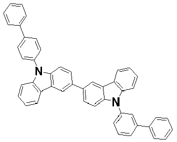 | 92% |
| 2-33 | 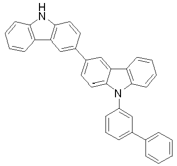 | 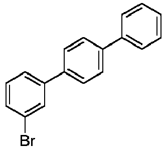 | 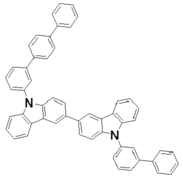 | 74% |
| 2-34 | 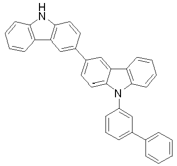 | 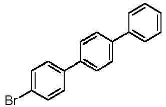 | 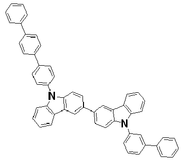 | 65% |
| 2-42 | 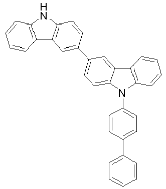 | 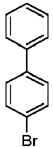 | 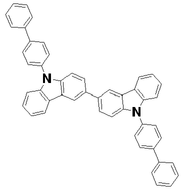 | 69% |
| 2-44 | 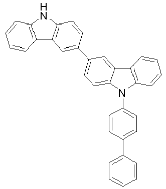 | 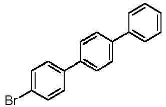 | 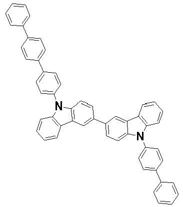 | 85% |

Compounds other than the compounds described in Table 1 and Table 2 were also prepared in the same manner as in the preparation examples described above.

Synthesis identification results for the compounds prepared above are shown in the following Table 3 and Table 4. Table 3 shows measurement values of FD-mass spectrometry (FD-Mass: field desorption mass spectrometry), and Table 4 shows measurement values of ¹H NMR (DMSO, 300 Mz) .

**[Table 3]**

| Compou nd | FD-Mass | Compou nd | FD-Mass |
|---|---|---|---|
| 1-1 | m/z=641.73 (C45H27N3O2=641.21) | 1-4 | m/z=641.73 (C45H27N3O2=641.21) |
| 1-8 | m/z=641.73 (C45H27N3O2=641.21) | 1-10 | m/z=641.73 (C45H27N3O2=641.21) |
| 1-15 | m/z=641.73 (C45H27N3O2=641.21) | 1-16 | m/z=641.73 (C45H27N3O2=641.21) |
| 1-19 | m/z=641.73 (C45H27N3O2=641.21) | 1-26 | m/z=641.73 (C45H27N3O2=641.21) |
| 1-28 | m/z=641.73 (C45H27N3O2=641.21) | 1-32 | m/z=641.73 (C45H27N3O2=641.21) |
| 1-34 | m/z=641.73 (C45H27N3O2=641.21) | 1-37 | m/z=673.85 (C45H27N3S2=673.16) |
| 1-40 | m/z=673.85 (C45H27N3S2=673.16) | 1-41 | m/z=673.85 (C45H27N3S2=673.16) |
| 1-44 | m/z=673.85 (C45H27N3S2=673.16) | 1-48 | m/z=673.85 (C45H27N3S2=673.16) |
| 1-52 | m/z=673.85 (C45H27N3S2=673.16) | 1-57 | m/z=673.85 (C45H27N3S2=673.16) |
| 1-61 | m/z=673.85 (C45H27N3S2=673.16) | 1-68 | m/z=673.85 (C45H27N3S2=673.16) |
| 1-72 | m/z=673.85 (C45H27N3S2=673.16) | 1-73 | m/z=657.79 (C45H27N3OS=657.19) |
| 1-79 | m/z=657.79 (C45H27N3OS=657.19) | 1-81 | m/z=657.79 (C45H27N3OS=657.19) |
| 1-86 | m/z=657.79 (C45H27N3OS=657.19) | 1-90 | m/z=657.79 (C45H27N3OS=657.19) |
| 1-92 | m/z=657.79 (C45H27N3OS=657.19) | 1-95 | m/z=657.79 (C45H27N3OS=657.19) |
| 1-99 | m/z=657.79 (C45H27N3OS=657.19) | 1-100 | m/z=657.79 (C45H27N3OS=657.19) |
| 1-103 | m/z=657.79 (C45H27N3OS=657.19) | 1-105 | m/z=657.79 (C45H27N3OS=657.19) |
| 1-108 | m/z=657.79 (C45H27N3OS=657.19) | 1-109 | m/z=716.84 (C51H34N4O=716.26) |
| 1-112 | m/z=716.84 (C51H34N4O=716.26) | 1-115 | m/z=716.84 (C51H34N4O=716.26) |
| 1-119 | m/z=716.84 (C51H34N4O=716.26) | 1-121 | m/z=716.84 (C51H34N4O=716.26) |
| 1-125 | m/z=716.84 (C51H34N4O=716.26) | 1-128 | m/z=716.84 (C51H34N4O=716.26) |
| 1-131 | m/z=716.84 (C51H34N4O=716.26) | 1-134 | m/z=716.84 (C51H34N4O=716.26) |
| 1-135 | m/z=716.84 (C51H34N4O=716.26) | 1-139 | m/z=732.91 (C51H32N4S=732.23) |
| 1-140 | m/z=732.91 (C51H32N4S=732.23) | 1-144 | m/z=732.91 (C51H32N4S=732.23) |
| 1-146 | m/z=732.91 (C51H32N4S=732.23) | 1-148 | m/z=732.91 (C51H32N4S=732.23) |
| 1-155 | m/z=732.91 (C51H32N4S=732.23) | 1-160 | m/z=732.91 (C51H32N4S=732.23) |
| 1-161 | m/z=732.91 (C51H32N4S=732.23) | 1-167 | m/z=732.91 (C51H32N4S=732.23) |
| 1-172 | m/z=791.96 (C57H37N5=791.30) | 1-176 | m/z=791.96 (C57H37N5=791.30) |
| 1-179 | m/z=791.96 (C57H37N5=791.30) | 1-180 | m/z=791.96 (C57H37N5=791.30) |
| 1-182 | m/z=791.96 (C57H37N5=791.30) | 1-188 | m/z=791.96 (C57H37N5=791.30) |
| 1-189 | m/z=791.96 (C57H37N5=791.30) | 1-192 | m/z=791.96 (C57H37N5=791.30) |
| 1-194 | m/z=791.96 (C57H37N5=791.30) | 1-198 | m/z=791.96 (C57H37N5=791.30) |
| 1-221 | m/z=655.82 (C45H13D14N3O2=655.30) | 1-223 | m/z=655.82 (C45H13D14N3O2=655.30) |
| 1-230 | m/z=655.82 (C45H13D14N3O2=655.30) | 1-232 | m/z=655.82 (C45H13D14N3O2=655.30) |
| 1-238 | m/z=687.94 (C45H13D14N3S2=687.25) | 1-242 | m/z=687.94 (C45H13D14N3S2=687.25) |
| 1-244 | m/z=687.94 (C45H13D14N3S2=687.25) | 1-250 | m/z=687.94 (C45H13D14N3S2=687.25) |
| 1-259 | m/z=671.88 (C45H13D14N3OS=671.28) | 1-260 | m/z=671.88 (C45H13D14N3OS=671.28) |
| 1-266 | m/z=671.88 (C45H13D14N3OS=671.28) | 1-272 | m/z=671.88 (C45H13D14N3OS=671.28) |
| 1-273 | m/z=735.96 (C51H13D19N4O=735.38) | 1-276 | m/z=735.96 (C51H13D19N4O=735.38) |
| 1-277 | m/z=735.96 (C51H13D19N4O=735.38) | 1-278 | m/z=735.96 (C51H13D19N4O=735.38) |
| 1-281 | m/z=735.96 (C51H13D19N4O=735.38) | 1-286 | m/z=752.02 (C51H13D19N4S=751.35) |
| 1-291 | m/z=752.02 (C51H13D19N4S=751.35) | 1-294 | m/z=752.02 (C51H13D19N4S=751.35) |
| 1-297 | m/z=816.10 (C57H13D24N5=815.46) | 1-301 | m/z=816.10 (C57H13D24N5=815.46) |
| 1-303 | m/z=735.94 (C51H13D18N3O2=735.35) | 1-304 | m/z=768.06 (C51H13D18N3S2=767.31) |
| 2-23 | m/z=712.90 (C54H36N2=712.29) | 2-32 | m/z=636.80 (C48H32N2=636.26) |
| 2-33 | m/z=712.90 (C54H36N2=712.29) | 2-34 | m/z=712.90 (C54H36N2=712.29) |
| 2-42 | m/z=636.80 (C48H32N2=636.26) | 2-44 | m/z=712.90 (C54H36N2=712.29) |

**[Table 4]**

| Compound | ¹H NMR (DMSO, 300 Mz) |
|---|---|
| 1-1 | δ=7.73∼8.08 (13H, m), 7.25 (14H, m) |
| 1-4 | δ=7.73∼8.03 (14H, m), 7.25-7.61 (13H, m) |
| 1-8 | δ=8.38 (1H, d), 8.08 (1H, d), 7.73∼7.98 (10H, m), 7.25∼7.61 (15H, m) |
| 1-10 | δ=8.38 (1H, d), 7.73∼8.03 (12H, m), 7.25∼7.61 (14H, m) |
| 1-15 | δ=8.38 (1H, d), 8.08 (1H, d), 7.31∼7.98 (25H, m) |
| 1-16 | δ=8.38 (1H, d), 7.73∼8.03 (14H, m), 7.31∼7.61 (12H, m) |
| 1-19 | δ=7.73∼8.08 (13H, m), 7.31∼7.61 (14H, m) |
| 1-26 | δ=8.08 (1H, d), 7.75∼7.98 (10H, m), 7.25∼7.60 (16H, m) |
| 1-28 | δ=7.75∼8.03 (12H, m), 7.25∼7.60 (15H, m) |
| 1-32 | δ=8.08 (1H, d), 7.79∼7.98 (12H, m), 7.31∼7.60 (14H, m) |
| 1-34 | δ=7.76∼8.03 (14H, m), 7.31∼7.60 (13H, m) |
| 1-37 | δ=8.55 (1H, d), 8.45 (1H, d), 8.20∼8.24 (2H, m), 7.92∼7.96 (7H, m), 7.70-7.75 (4H, m), 7.41∼7.61 (9H, m), 7.25 (2H, d) |
| 1-40 | δ=8.45 (2H, d), 8.20∼8.24 (2H, m), 8.12 (1H, s), 7.92∼7.99 (8H, m), 7.73∼7.75 (3H, m), 7.41∼7.61 (9H, m), 7.25 (2H, d) |
| 1-41 | δ=8.45 (1H, d), 8.20∼8.24 (2H, m), 7.93∼8.03 (8H, m), 7.41∼7.75 (13H, m), 7.25 (2H, d) |
| 1-44 | δ=8.55 (1H, d), 8.38∼8.45 (3H, m), 8.12 (1H, s), 7.92∼7.99 (6H, m), 7.70∼7.75 (4H, m), 7.41∼7.61 (8H, m), 7.25 (4H, s) |
| 1-48 | δ=8.38∼8.45 (3H, m), 8.12 (1H, s), 7.92∼8.03 (7H, m), 7.41∼7.75 (12H, m), 7.25 (4H, s) |
| 1-52 | δ=8.38∼8.45 (3H, m), 8.20∼8.24 (2H, m), 8.12 (1H, s), 7.92∼7.99 (7H, m), 7.73∼7.75 (4H, m), 7.41∼7.61 (10H, m) |
| 1-57 | δ=8.55 (1H, d), 8.45 (2H, d), 7.92∼8.03 (8H, m), 7.41∼7.75 (16H, m) |
| 1-61 | δ=8.55 (1H, d), 8.45 (1H, d), 8.20∼8.24 (2H, m), 7.92∼7.96 (6H, m), 7.70∼7.75 (3H, m), 7.41∼7.60 (9H, m), 7.25 (4H, s) |
| 1-68 | δ=8.55 (1H, d), 8.45 (1H, d), 8.12 (1H, s), 7.92-7.99 (9H, m), 7.79 (2H, d), 7.70 (1H, t), 7.41∼7.60 (11H, m) |
| 1-72 | δ=8.45 (1H, d), 8.12 (1H, s), 7.60-8.03 (10H, m), 7.79 (2H, d), 7.41∼7.68 (12H, m) |
| 1-73 | δ=8.45 (1H, d), 8.20∼8.24 (2H, m), 8.08 (1H, d), 7.88∼7.98 (7H, m), 7.73∼7.75 (3H, m), 7.25∼7.61 (13H, m) |
| 1-79 | δ=8.38∼8.45 (2H, m), 8.20∼8.24 (2H, m), 8.08 (1H, d), 7.88∼7.98 (5H, m), 7.73∼7.75 (3H, m), 7.25∼7.61 (14H, m) |
| 1-81 | δ=8.38∼8.45 (2H, m), 7.93∼8.08 (7H, m), 7.25-7.75 (18H, m) |
| 1-86 | δ=8.38∼8.45 (2H, m), 8.08∼8.12 (2H, m), 7.88∼7.99 (7H, m), 7.73∼7.75 (4H, m), 7.31∼7.61 (12H, m), |
| 1-90 | δ=8.38∼8.45 (2H, m), 7.31∼8.03 (25H, m) |
| 1-92 | δ=8.45 (1H, d), 8.08∼8.12 (2H, m), 7.88∼7.99 (8H, m), 7.73∼7.75 (3H, m), 7.31-7.61 (13H, m) |
| 1-95 | δ=8.45 (1H, d), 7.93∼8.03 (8H, m), 7.31∼7.82 (18H, m) |
| 1-99 | δ=8.45 (1H, d), 7.93∼8.08 (8H, m), 7.25∼7.75 (18H, m) |
| 1-100 | δ=8.45 (1H, d), 8.12 (1H, s), 7.92∼8.03 (7H, m), 7.75∼7.82 (4H, m), 7.25∼7.60 (14H, m) |
| 1-103 | δ=8.45 (1H, d), 8.20∼8.24 (2H, m), 8.08 (1H, d), 7.88∼7.98 (8H, m), 7.79 (2H, d), 7.31∼7.60 (13H, m) |
| 1-105 | δ=8.45 (1H, d), 7.88∼8.08 (10H, m), 7.76∼7.79 (2H, d), 7.31∼7.68 (14H, m) |
| 1-108 | δ=8.45 (1H, d), 7.79∼8.03 (13H, m), 7.31∼7.68 (13H, m) |
| 1-109 | δ=8.62 (1H, d), 8.19∼8.22 (2H, m), 7.94∼8.03 (5H, m), 7.73∼7.82 (6H, m), 7.20∼7.62 (18H, m) |
| 1-112 | δ=8.19∼8.22 (2H, t), 7.73∼8.04 (13H, m), 7.20∼7.62 (19H, m) |
| 1-115 | δ=8.62 (1H, d), 8.38 (1H, d), 8.19∼8.22 (2H, t), 7.94∼8.03 (3H, m), 7.73∼7.82 (6H, m), 7.25∼7.62 (19H, m) |
| 1-119 | δ=8.62 (1H, d), 8.38 (1H, d), 8.19∼8.22 (2H, t), 8.08 (1H, d), 7.88∼7.98 (4H, m), 7.73∼7.75 (5H, m), 7.31∼7.62 (17H, m), 7.20 (1H, t) |
| 1-121 | δ=8.38 (1H, d), 8.30 (1H, d), 8.13-8.19 (2H, m), 7.73∼7.98 (11H, m), 7.31∼7.62 (16H, m), 7.20 (1H, t) |
| 1-125 | δ=8.55 (1H, d), 8.19∼8.24 (3H, m), 7.31∼7.98 (21H, m), 7.16∼7.20 (2H, t) |
| 1-128 | δ=8.30 (1H, d), 8.13∼8.19 (2H, m), 7.89∼8.03 (5H, m), 7.75∼7.82 (4H, m), 7.20∼7.62 (20H, m) |
| 1-131 | δ=8.65 (1H, d), 8.30 (1H, d), 8.19 (1H, d), 7.79∼7.98 (10H, m), 7.31∼7.62 (18H, m), 7.20 (1H, t) |
| 1-134 | δ=8.65 (1H, d), 8.30 (1H, d), 8.19 (1H, d), 7.96∼7.98 (3H, m), 7.75∼7.88 (5H, m), 7.20∼7.62 (21H, m) |
| 1-135 | δ=8.30 (1H, d), 8.13∼8.19 (2H, m), 7.96∼8.03 (4H, m), 7.89 (1H, s), 7.75∼7.82 (4H, m), 7.20∼7.62 (20H, m) |
| 1-139 | δ=8.45 (1H, d), 8.19-8.24 (4H, m), 8.04 (1H, d), 7.93∼7.96 (5H, m), 7.73∼7.75 (3H, m), 7.41∼7.62 (15H, m), 7.25 (2H, m) |
| 1-140 | δ=8.62 (1H, d), 8.45 (1H, d), 8.19∼8.24 (4H, m), 7.93∼7.96 (5H, m), 7.73∼7.75 (4H, m), 7.41∼7.62 (14H, m), 7.20∼7.25 (3H, m) |
| 1-144 | δ=8.38∼8.45 (2H, m), 8.19∼8.24 (4H, m), 8.04 (1H, d), 7.93∼7.94 (3H, m), 7.73∼7.75 (3H, m), 7.41∼7.62 (14H, m), 7.20∼7.25 (4H, m) |
| 1-146 | δ=8.55∼8.62 (2H, m), 8.38∼8.45 (2H, m), 8.19∼8.22 (2H, m), 7.92∼7.94 (4H, m), 7.41∼7.75 (21H, m), 7.20 (1H, t) |
| 1-148 | δ=8.62 (1H, d), 8.38-8.45 (2H, m), 8.19∼8.22 (2H, m), 8.03 (1H, d), 7.93∼7.94 (4H, m), 7.41∼7.75 (21H, m), 7.20 (1H, t) |
| 1-155 | δ=8.62 (1H, d), 8.45 (1H, d), 8.19∼8.24 (4H, m), 7.93∼7.96 (5H, m), 7.73∼7.75 (4H, m), 7.41∼7.62 (16H, m), 7.20 (1H, t) |
| 1-160 | δ=8.62 (1H, d), 8.45 (1H, d), 8.19∼8.24 (4H, m), 7.93∼7.96 (4H, m), 7.74∼7.75 (3H, m), 7.41∼7.62 (14H, m), 7.20-7.25 (5H, m) |
| 1-161 | δ=8.55∼8.62 (2H, m), 8.45 (1H, d), 8.19∼8.22 (2H, m), 7.92∼7.96 (6H, m), 7.41∼7.79 (20H, m), 7.20 (1H, t) |
| 1-167 | δ=8.62 (1H, d), 8.45 (1H, d), 8.19-8.24 (4H, m), 7.93∼7.96 (4H, m), 7.74∼7.75 (3H, m), 7.41∼7.62 (12H, m), 7.20∼7.25 (7H, m) |
| 1-172 | δ=8.62 (2H, d), 8.19-8.22 (4H, t), 7.94∼7.96 (3H, d), 7.73∼7.75 (5H, m), 7.41∼7.62 (19H, m), 7.20∼7.25 (4H, m) |
| 1-176 | δ=8.62 (2H, d), 8.38 (1H, d), 8.19-8.22 (3H, t), 7.94 (1H,s), 7.74∼7.75 (5H, m), 7.41∼7.62 (18H, m), 7.20∼7.25 (6H, m) |
| 1-179 | δ=8.62∼8.65 (2H, t), 8.38 (1H, d), 8.30 (1H, d), 8.19∼8.22 (3H, m), 7.94 (2H, s), 7.73∼7.75 (5H, m), 7.41∼7.61 (21H, m), 7.20 (2H, t) |
| 1-180 | δ=8.62 (1H, d), 8.38 (1H, d), 8.19∼8.22 (4H, m), 7.94 (2H, s), 7.73∼7.75 (6H, m), 7.41∼7.62 (20H, m), 7.20 (2H, t) |
| 1-182 | δ=8.62 (1H, d), 8.38 (1H, d), 8.19∼8.22 (4H, m), 8.04 (1H, d), 7.94 (2H, s), 7.73∼7.75 (5H, m), 7.41∼7.62 (21H, m), 7.20 (2H, t) |
| 1-188 | δ=8.62 (2H, d), 8.19-8.22 (4H, t), 7.96 (2H, t), 7.74∼7.75 (4H, d), 7.41∼7.62 (19H, m), 7.20∼7.25 (6H, m) |
| 1-189 | δ=8.62 (1H, d), 8.30 (1H, d), 8.13-8.22 (4H, m), 7.89∼7.96 (3H, m), 7.74∼7.75 (3H, d), 7.41∼7.62 (19H, m), 7.20∼7.25 (6H, m) |
| 1-192 | δ=8.62 (2H, d), 8.19∼8.22 (4H, t), 7.96 (2H, d), 7.74∼7.75 (4H, m), 7.41∼7.62 (17H, m), 7.20∼7.25 (8H, m) |
| 1-194 | δ=8.62 (1H, d), 8.19∼8.22 (4H, t), 8.04 (1H, d), 7.96 (2H, d), 7.74∼7.75 (3H, d), 7.41∼7.62 (18H, m), 7.20∼7.25 (8H, m) |
| 1-198 | δ=8.62 (1H, d), 8.19∼8.22 (4H, t), 7.96∼8.04 (5H, m), 7.74∼7.79 (3H, m), 7.41∼7.62 (21H, m), 7.20 (2H, t) |
| 1-221 | δ=7.94∼7.96 (3H, m), 7.73∼7.75 (3H, m), 7.61 (2H, d), 7.41∼7.49 (3H, m), 7.25 (2H, d) |
| 1-223 | δ=7.94∼7.96 (3H, m), 7.73∼7.75 (3H, m), 7.61 (2H, d), 7.41∼7.49 (3H, m), 7.25 (2H, d) |
| 1-230 | δ=8.38 (1H, d), 7.94 (2H, s), 7.73∼7.75 (4H, m), 7.61 (3H, d), 7.41∼7.49 (3H, m) |
| 1-232 | δ=7.94∼7.96 (3H, m), 7.73∼7.75 (3H, m), 7.60∼7.61 (4H, m), 7.41∼7.49 (3H, m) |
| 1-238 | δ=7.94∼7.96 (3H, m), 7.73∼7.75 (3H, m), 7.61 (2H, d), 7.41∼7.49 (3H, m), 7.25 (2H, d) |
| 1-242 | δ=7.94∼7.96 (3H, m), 7.73∼7.75 (3H, m), 7.61 (2H, d), 7.41∼7.49 (3H, m), 7.25 (2H, d) |
| 1-244 | δ=8.38 (1H, d), 7.94 (1H, s), 7.73∼7.75 (3H, m), 7.61 (1H, d), 7.41∼7.49 (3H, m), 7.25 (4H, s) |
| 1-250 | δ=7.94∼7.96 (3H, m), 7.73∼7.75 (3H, m), 7.60∼7.61 (4H, m), 7.41∼7.49 (3H, m) |
| 1-259 | δ=7.94∼7.96 (3H, m), 7.73∼7.75 (3H, m), 7.61 (2H, d), 7.41∼7.49 (3H, m), 7.25 (2H, d) |
| 1-260 | δ=7.94∼7.96 (3H, m), 7.73∼7.75 (3H, m), 7.61 (2H, d), 7.41∼7.49 (3H, m), 7.25 (2H, d) |
| 1-266 | δ=8.38 (1H, d), 7.94 (2H, s), 7.73∼7.75 (4H, m), 7.61 (3H, d), 7.41∼7.49 (3H, m) |
| 1-272 | δ=7.96 (4H, m), 7.79 (2H, d), 7.60 (4H, m), 7.41∼7.46 (3H, m) |
| 1-273 | δ=7.94∼7.96 (3H, m), 7.73∼7.75 (3H, m), 7.61 (2H, d), 7.41∼7.49 (3H, m), 7.25 (2H, d) |
| 1-276 | δ=7.94∼7.96 (3H, m), 7.73∼7.75 (3H, m), 7.61 (2H, d), 7.41∼7.49 (3H, m), 7.25 (2H, d) |
| 1-277 | δ=7.94∼7.96 (3H, m), 7.73∼7.75 (3H, m), 7.61 (2H, d), 7.41∼7.49 (3H, m), 7.25 (2H, d) |
| 1-278 | δ=7.94∼7.96 (3H, m), 7.73∼7.75 (3H, m), 7.61 (2H, d), 7.41∼7.49 (3H, m), 7.25 (2H, d) |
| 1-281 | δ=8.38 (1H, d), 7.94 (1H, s), 7.73∼7.75 (3H, m), 7.61 (1H, d), 7.41∼7.49 (3H, m), 7.25 (4H, s) |
| 1-286 | δ=7.94∼7.96 (3H, m), 7.73∼7.75 (3H, m), 7.61 (2H, d), 7.41∼7.49 (3H, m), 7.25 (2H, d) |
| 1-291 | δ=8.38 (1H, d), 7.94 (1H, s), 7.73∼7.75 (3H, m), 7.61 (1H, d), 7.41∼7.49 (3H, m), 7.25 (4H, s) |
| 1-294 | δ=8.38 (1H, d), 7.94 (2H, s), 7.73∼7.75 (4H, m), 7.61 (3H, d), 7.41∼7.49 (3H, m) |
| 1-297 | δ=7.94∼7.96 (3H, m), 7.73∼7.75 (3H, m), 7.61 (2H, d), 7.41∼7.49 (3H, m), 7.25 (2H, d) |
| 1-301 | δ=7.96 (2H, d), 7.75 (2H, d), 7.41∼7.49 (3H, m), 7.25 (6H, m) |
| 1-303 | δ=7.94∼7.96 (3H, m), 7.73∼7.75 (3H, m), 7.61 (2H, d), 7.41∼7.49 (3H, m), 7.25 (2H, d) |
| 1-304 | δ=7.94∼7.96 (3H, m), 7.73∼7.75 (3H, m), 7.61 (2H, d), 7.41∼7.49 (3H, m), 7.25 (2H, d) |
| 2-23 | δ=8.55 (1H, d), 8.30(1H, d), 8.13∼8.19 (2H, m), 7.91∼7.99 (10H, m), 7.75∼7.80 (4H, m), 7.35∼7.58 (8H, m), 7.16∼7.25 (10H, m) |
| 2-32 | δ=8.55 (1H, d), 8.30 (1H, d), 8.13∼8.21 (3H, m), 7.89∼7.99 (8H, m), 7.35∼7.77 (17H, m), 7.16∼7.20 (2H, m) |
| 2-33 | δ=8.55 (1H, d), 8.30 (1H, d), 8.13∼8.21 (3H, m), 7.89∼7.94 (4H, m), 7.35∼7.77 (20H, m), 7.16∼7.25 (6H, m) |
| 2-34 | δ=8.55 (1H, d), 8.30 (1H, d), 8.13∼8.21 (3H, m), 7.89∼7.99 (8H, m), 7.35∼7.77 (17H, m), 7.16∼7.25 (6H, m) |
| 2-42 | δ=8.55 (1H, d), 8.30 (1H, d), 8.13∼8.19 (2H, m), 7.91∼7.99 (12H, m), 7.75∼7.77 (5H, m), 7.58 (1H, d), 7.35∼7.50 (8H, m), 7.16∼7.20 (2H, m) |
| 2-44 | δ=8.55 (1H, d), 8.30 (1H, d), 8.13∼8.19 (2H, m), 7.89∼7.99 (12H, m), 7.75∼7.77 (5H, m), 7.41∼7.50 (8H, m), 7.16∼7.25 (6H, m) |

### <Experimental Example 1>

### 1) Manufacture of Organic Light Emitting Device

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl(phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. The light emitting layer was deposited to 400 Å using a compound described in the following Table 5 as a host, and doping Ir(ppy)₃ (tris(2-phenylpyridine)iridium) to the host by 7% as a green phosphorescent dopant. After that, BCP was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 10⁻⁸ torr to 10⁻⁶ torr for each material to be used in the OLED manufacture.

### 2) Driving Voltage and Light Emission Efficiency of Organic Electroluminescent Device

For each of the organic electroluminescent devices manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc.. Properties of the organic electroluminescent devices of the present disclosure are as shown in the following Table 5.

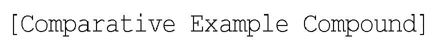

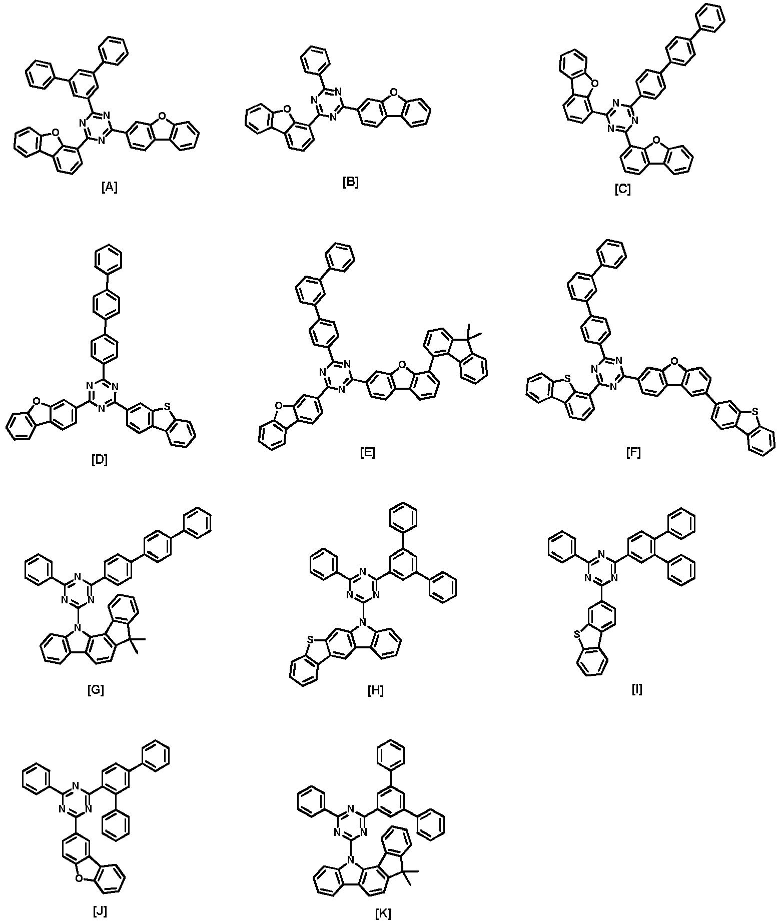

**[Table 5]**

| | Compound | Driving Voltage (V) | Efficienc y (cd/A) | Color Coordinate (x, y) | Lifetime (T₉₀) |
|---|---|---|---|---|---|
| Comparative Example 1 | A | 6.48 | 41.7 | (0.250, 0.676) | 50 |
| Comparative Example 2 | B | 6.22 | 42.9 | (0.245, 0.670) | 54 |
| Comparative Example 3 | C | 5.51 | 45.0 | (0.247, 0.684) | 70 |
| Comparative Example 4 | D | 5.68 | 44.8 | (0.271, 0.689) | 68 |
| Comparative Example 5 | E | 6.02 | 43.5 | (0.256, 0.677) | 61 |
| Comparative Example 6 | F | 5.90 | 44.1 | (0.273, 0.683) | 62 |
| Comparative Example 7 | G | 6.05 | 45.3 | (0.244, 0.663) | 70 |
| Comparative Example 8 | H | 6.15 | 43.2 | (0.253, 0.670) | 66 |
| Comparative Example 9 | I | 6.10 | 40.3 | (0.255, 0.660) | 55 |
| Comparative Example 10 | J | 6.13 | 40.8 | (0.261, 0.672) | 58 |
| Comparative Example 11 | K | 6. | 41.2 | (0.263, 0.652) | 53 |
| Example 1 | 1-1 | 3.01 | 84.7 | (0.283, 0.681) | 144 |
| Example 2 | 1-4 | 3.47 | 81.5 | (0.270, 0.675) | 141 |
| Example 3 | 1-8 | 3.33 | 77.6 | (0.269, 0.674) | 129 |
| Example 4 | 1-10 | 3.29 | 79.4 | (0.254, 0.687) | 138 |
| Example 5 | 1-15 | 3.25 | 80.1 | (0.240, 0.672) | 133 |
| Example 6 | 1-16 | 3.19 | 84.7 | (0.235, 0.670) | 129 |
| Example 7 | 1-19 | 3.11 | 83.6 | (0.268, 0.686) | 125 |
| Example 8 | 1-26 | 3.28 | 75.9 | (0.283, 0.675) | 129 |
| Example 9 | 1-28 | 3.42 | 79.4 | (0.273, 0.670) | 140 |
| Example 10 | 1-32 | 3.50 | 82.6 | (0.249, 0.673) | 133 |
| Example 11 | 1-34 | 3.44 | 85.0 | (0.245, 0.675) | 138 |
| Example 12 | 1-37 | 3.18 | 84.2 | (0.287, 0.659) | 145 |
| Example 13 | 1-40 | 3.20 | 83.2 | (0.248, 0.663) | 144 |
| Example 14 | 1-41 | 3.13 | 77.2 | (0.263, 0.670) | 138 |
| Example 15 | 1-44 | 3.47 | 75.9 | (0.231, 0.665) | 125 |
| Example 16 | 1-48 | 3.25 | 83.4 | (0.286, 0.688) | 129 |
| Example 17 | 1-52 | 3.11 | 86.6 | (0.270, 0.673) | 131 |
| Example 18 | 1-57 | 3.47 | 74.9 | (0.281, 0.670) | 133 |
| Example 19 | 1-61 | 3.15 | 75.3 | (0.273, 0.670) | 142 |
| Example 20 | 1-68 | 3.48 | 80.2 | (0.245, 0.673) | 144 |
| Example 21 | 1-72 | 3.51 | 83.3 | (0.240, 0.672) | 140 |
| Example 22 | 1-73 | 3.59 | 75.0 | (0.267, 0.659) | 126 |
| Example 23 | 1-79 | 3.68 | 69.2 | (0.258, 0.667) | 123 |
| Example 24 | 1-81 | 3.79 | 74.3 | (0.267, 0.672) | 110 |
| Example 25 | 1-86 | 3.62 | 70.2 | (0.237, 0.664) | 109 |
| Example 26 | 1-90 | 3.55 | 68.5 | (0.247, 0.655) | 105 |
| Example 27 | 1-92 | 3.50 | 69.3 | (0.268, 0.683) | 119 |
| Example 28 | 1-95 | 3.71 | 65.7 | (0.267, 0.675) | 120 |
| Example 29 | 1-99 | 3.59 | 66.1 | (0.233, 0.667) | 123 |
| Example 30 | 1-100 | 3.54 | 73.2 | (0.256, 0.667) | 118 |
| Example 31 | 1-103 | 3.51 | 74.2 | (0.260, 0.672) | 109 |
| Example 32 | 1-105 | 3.88 | 69.3 | (0.235, 0.664) | 116 |
| Example 33 | 1-108 | 4.01 | 66.5 | (0.247, 0.657) | 125 |
| Example 34 | 1-109 | 4.39 | 65.0 | (0.248, 0.673) | 95 |
| Example 35 | 1-112 | 4.22 | 62.3 | (0.260, 0.672) | 90 |
| Example 36 | 1-115 | 4.13 | 61.2 | (0.267, 0.657) | 93 |
| Example 37 | 1-119 | 4.48 | 64.3 | (0.257, 0.660) | 86 |
| Example 38 | 1-121 | 5.00 | 62.2 | (0.250, 0.667) | 83 |
| Example 39 | 1-125 | 4.67 | 63.9 | (0.267, 0.682) | 81 |
| Example 40 | 1-128 | 4.59 | 64.1 | (0.239, 0.664) | 80 |
| Example 41 | 1-131 | 4.18 | 60.8 | (0.257, 0.657) | 84 |
| Example 42 | 1-134 | 4.63 | 61.9 | (0.269, 0.685) | 88 |
| Example 43 | 1-135 | 4.28 | 62.0 | (0.275, 0.674) | 95 |
| Example 44 | 1-139 | 4.75 | 65.5 | (0.245, 0.670) | 91 |
| Example 45 | 1-140 | 4.68 | 61.4 | (0.247, 0.677) | 85 |
| Example 46 | 1-144 | 4.47 | 62.8 | (0.257, 0.669) | 87 |
| Example 47 | 1-146 | 4.49 | 64.4 | (0.254, 0.657) | 84 |
| Example 48 | 1-148 | 4.38 | 60.7 | (0.267, 0.670) | 80 |
| Example 49 | 1-155 | 4.22 | 62.5 | (0.255, 0.673) | 83 |
| Example 50 | 1-160 | 4.19 | 63.1 | (0.246, 0.672) | 88 |
| Example 51 | 1-161 | 4.55 | 64.8 | (0.268, 0.657) | 91 |
| Example 52 | 1-167 | 4.13 | 65.0 | (0.248, 0.657) | 93 |
| Example 53 | 1-172 | 5.50 | 60.2 | (0.266, 0.662) | 83 |
| Example 54 | 1-176 | 5.18 | 59.3 | (0.255, 0.674) | 85 |
| Example 55 | 1-179 | 5.46 | 57.0 | (0.271, 0.687) | 80 |
| Example 56 | 1-180 | 5.19 | 50.9 | (0.287, 0.681) | 91 |
| Example 57 | 1-182 | 5.28 | 51.5 | (0.274, 0.675) | 87 |
| Example 58 | 1-188 | 5.11 | 56,7 | (0.265, 0.673) | 85 |
| Example 59 | 1-189 | 5.17 | 57.7 | (0.246, 0.652) | 83 |
| Example 60 | 1-192 | 5.38 | 55.3 | (0.264, 0.657) | 88 |
| Example 61 | 1-194 | 5.44 | 54.1 | (0.258, 0.656) | 87 |
| Example 62 | 1-198 | 5.43 | 58.6 | (0.270, 0.667) | 90 |
| Example 63 | 1-221 | 3.08 | 86.3 | (0.253, 0.646) | 153 |
| Example 64 | 1-223 | 3.39 | 85.6 | (0.242, 0.673) | 152 |
| Example 65 | 1-230 | 3.15 | 82.7 | (0.257, 0.664) | 150 |
| Example 66 | 1-232 | 3.12 | 87.0 | (0.275, 0.679) | 155 |
| Example 67 | 1-238 | 3.76 | 74.2 | (0.246, 0.657) | 140 |
| Example 68 | 1-242 | 3.68 | 76.3 | (0.277, 0.684) | 148 |
| Example 69 | 1-244 | 3.72 | 79.5 | (0.246, 0.665) | 141 |
| Example 70 | 1-250 | 3.80 | 80.4 | (0.252, 0.673) | 146 |
| Example 71 | 1-259 | 3.85 | 74.2 | (0.251, 0.661) | 134 |
| Example 72 | 1-260 | 4.01 | 75.0 | (0.267, 0.652) | 130 |
| Example 73 | 1-266 | 3.77 | 69.3 | (0.247, 0.654) | 128 |
| Example 74 | 1-272 | 3.67 | 67.8 | (0.279, 0.678) | 121 |
| Example 75 | 1-273 | 4.43 | 65.2 | (0.256, 0.656) | 100 |
| Example 76 | 1-276 | 4.38 | 64.7 | (0.273, 0.674) | 98 |
| Example 77 | 1-277 | 4.47 | 63.5 | (0.268, 0.675) | 111 |
| Example 78 | 1-278 | 4.52 | 65.9 | (0.254, 0.673) | 105 |
| Example 79 | 1-281 | 4.03 | 64.3 | (0.259, 0.665) | 110 |
| Example 80 | 1-286 | 5.02 | 60.2 | (0.266, 0.657) | 90 |
| Example 81 | 1-291 | 4.95 | 61.3 | (0.280, 0.674) | 93 |
| Example 82 | 1-294 | 4.88 | 62.4 | (0.273, 0.672) | 95 |
| Example 83 | 1-297 | 5.23 | 58.7 | (0.265, 0.670) | 105 |
| Example 84 | 1-301 | 5.38 | 60.3 | (0.240, 0.675) | 100 |
| Example 85 | 1-303 | 3.59 | 74.3 | (0.266, 0.658) | 135 |
| Example 86 | 1-304 | 3.53 | 70.9 | (0.263, 0.654) | 132 |

As seen from the results of Table 5, the organic electroluminescent device using the organic electroluminescent device light emitting layer material of the present disclosure had lower driving voltage and enhanced light emission efficiency, and significantly improved lifetime as well compared to Comparative Examples 1 to 11.

Particularly, as Ar of Chemical Formula 1 is extended longer, LUMO is more effectively expanded and flatness of the molecular structure increases. Accordingly, electrons are more favorably received, and as a result, electron migration occurs more effectively and the packing structure becomes more stable as well when deposited on the substrate. On the other hand, in the structure excluded from Ar of Chemical Formula 1, LUMO is spread in a circle, and lifetime and efficiency were identified to decrease compared to in the materials of the present disclosure. For the same reason, it is identified through Table 5 that driving, lifetime and efficiency tend to decrease as Ar is further bent to ortho or meta.

In addition, when Y1 and Y2 are each O or S in the bonding of Chemical Formula 1, and Chemical Formula 1-A and Chemical Formula 1-B, holes and electrons have more superior charge balance when m and n of Am-Bn are not equal compared to when m and n are the same. In dibenzofuran and dibenzothiophene, No. 2 and No. 4 positions are relatively electron-rich and No. 1 and No. 3 positions are relatively electron-deficient due to their resonance structures. Accordingly, the structure in which one side of triazine bonds to the relatively electron-rich position and the other side bonds to the relatively electron-deficient position has a better charge balance with P-type materials compared to when bonding to the same position, and accordingly, the lifetime is enhanced.

In addition, it is seen that, among the compounds of the present disclosure, light emission efficiency and lifetime are superior when including deuterium compared to the compounds of the present disclosure substituted with only hydrogen. Specifically, Compound 1-1 of Example 1 and Compound 1-221 of Example 63 have the same structure and are only different in the substitution of deuterium, and it is identified that Example 63 has high light emission efficiency and long lifetime while having a similar driving voltage with Example 1. This is due to the fact that the compound substituted with deuterium has higher stability for heat and energy compared to the compound not substituted with deuterium. Bonding dissociation energy is energy required to break bonds, and since bonding dissociation energy of carbon and deuterium is larger than bonding dissociation energy of carbon and hydrogen, the compound substituted with deuterium has higher stability for heat and energy. In addition, it was identified that the material substituted with deuterium tended to have an increased Tg (glass transition temperature) value compared to the compound not substituted with deuterium. From this point of view, it may be explained that the compound substituted with deuterium has high stability for heat, and thereby has long lifetime and high light emission efficiency. Moreover, since deuterium has twice the atomic mass of hydrogen, the compound substituted with deuterium has lower zero-point energy and vibrational energy compared to the hydrogen-bonding compound, and accordingly, energy in the ground state is reduced and the thin film is in a non-crystalline state due to weak intermolecular interactions, which enhances a lifetime of the device.

### <Experimental Example 2>

### 1) Manufacture of Organic Light Emitting Device

A glass substrate on which ITO was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl(phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. As the light emitting layer, one type of the compound of Chemical Formula 1 and one type of the compound of Chemical Formula 2 described in the following Table 6 were pre-mixed and then deposited to 400 Å in one source of supply as a host, and, as a green phosphorescent dopant, Ir(ppy)₃ was doped and deposited by 7% of the deposited thickness of the light emitting layer. After that, BCP was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 10⁻⁸ torr to 10⁻⁶ torr for each material to be used in the OLED manufacture.

For each of the organic electroluminescent devices manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc..

Results of measuring driving voltage, light emission efficiency, color coordinate (CIE) and lifetime of the organic light emitting devices manufactured according to the present disclosure are as shown in the following Table 6.

**[Table 6]**

| | Light Emitting Layer Compound | Ratio (Weight Ratio) | Driving Voltage (V) | Efficie ncy (cd/A) | Color Coordinate (x, y) | Lifetime (T₉₀) |
|---|---|---|---|---|---|---|
| Example 1 | 1-1:2-32 | 1:2 | 2.03 | 87.5 | (0.244, 0.660) | 478 |
| Example 2 | 1-1:2-32 | 1:1 | 2.00 | 90.2 | (0.240, 0.647) | 500 |
| Example 3 | 1-1:2-32 | 2:1 | 2.13 | 86.3 | (0.284, 0.679) | 470 |
| Example 4 | 1-20:2-32 | 1:2 | 2.39 | 82.4 | (0.247, 0.614) | 443 |
| Example 5 | 1-20:2-32 | 1:1 | 2.34 | 83.6 | (0.248, 0.659) | 450 |
| Example 6 | 1-20:2-32 | 2:1 | 2.40 | 81.7 | (0.253, 0.702) | 421 |
| Example 7 | 1-47:2-42 | 1:2 | 2.20 | 84.7 | (0.252, 0.724) | 468 |
| Example 8 | 1-47:2-42 | 1:1 | 2.15 | 85.6 | (0.241, 0.624) | 473 |
| Example 9 | 1-47:2-42 | 2:1 | 2.18 | 84.0 | (0.254, 0.710) | 460 |
| Example 10 | 1-56:2-42 | 1:2 | 2.46 | 82.7 | (0.240, 0.754) | 430 |
| Example 11 | 1-56:2-42 | 1:1 | 2.38 | 83.0 | (0.247, 0.657) | 448 |
| Example 12 | 1-56:2-42 | 2:1 | 2.42 | 81.3 | (0.250, 0.731) | 421 |
| Example 13 | 1-73:2-33 | 1:2 | 3.12 | 76.5 | (0.248, 0.705) | 387 |
| Example 14 | 1-73:2-33 | 1:1 | 3.03 | 79.3 | (0.230, 0.741) | 400 |
| Example 15 | 1-73:2-33 | 2:1 | 3.18 | 78.2 | (0.233, 0.714) | 380 |
| Example 16 | 1-88:2-34 | 1:2 | 3.39 | 75.3 | (0.241, 0.702) | 354 |
| Example 17 | 1-88:2-34 | 1:1 | 3.25 | 76.4 | (0.231, 0.625) | 360 |
| Example 18 | 1-88:2-34 | 2:1 | 3.43 | 75.0 | (0.246, 0.710) | 326 |
| Example 19 | 1-109:2-44 | 1:2 | 4.38 | 73.2 | (0.242, 0.713) | 280 |
| Example 20 | 1-109:2-44 | 1:1 | 4.25 | 74.8 | (0.235, 0.717) | 284 |
| Example 21 | 1-109:2-44 | 2:1 | 4.46 | 74.1 | (0.250, 0.618) | 276 |
| Example 22 | 1-133:2-34 | 1:2 | 4.18 | 74.3 | (0.263, 0.707) | 297 |
| Example 23 | 1-133:2-34 | 1:1 | 4.11 | 75.2 | (0.245, 0.619) | 302 |
| Example 24 | 1-133:2-34 | 2:1 | 4.23 | 74.0 | (0.235, 0.643) | 290 |
| Example 25 | 1-140:2-34 | 1:2 | 4.28 | 73.9 | (0.250, 0.627) | 271 |
| Example 26 | 1-140:2-34 | 1:1 | 4.24 | 74.2 | (0.232, 0.732) | 278 |
| Example 27 | 1-140:2-34 | 2:1 | 4.26 | 73.6 | (0.235, 0.665) | 270 |
| Example 28 | 1-152:2-34 | 1:2 | 4.52 | 72.0 | (0.246, 0.654) | 268 |
| Example 29 | 1-152:2-34 | 1:1 | 4.43 | 72.5 | (0.233, 0.715) | 270 |
| Example 30 | 1-152:2-34 | 2:1 | 4.58 | 71.6 | (0.232, 0.621) | 265 |
| Example 31 | 1-176:2-34 | 1:2 | 4.75 | 62.1 | (0.243, 0.657) | 221 |
| Example 32 | 1-176:2-34 | 1:1 | 4.68 | 63.2 | (0.257, 0.667) | 233 |
| Example 33 | 1-176:2-34 | 2:1 | 4.89 | 60.8 | (0.258, 0.730) | 215 |
| Example 34 | 1-192:2-44 | 1:2 | 4.59 | 64.2 | (0.258, 0.710) | 243 |
| Example 35 | 1-192:2-44 | 1:1 | 4.53 | 65.7 | (0.256, 0.702) | 250 |
| Example 36 | 1-192:2-44 | 2:1 | 4.64 | 63.6 | (0.242, 0.613) | 237 |
| Example 37 | 1-221:2-32 | 1:2 | 2.08 | 87.9 | (0.254, 0.663) | 550 |
| Example 38 | 1-221:2-32 | 1:1 | 2.11 | 88.0 | (0.250, 0.647) | 543 |
| Example 39 | 1-221:2-32 | 2:1 | 2.28 | 89.3 | (0.264, 0.659) | 542 |
| Example 40 | 1-223:2-34 | 1:2 | 2.32 | 89.2 | (0.287, 0.624) | 549 |
| Example 41 | 1-223:2-34 | 1:1 | 2.35 | 89.9 | (0.248, 0.655) | 546 |
| Example 42 | 1-223:2-34 | 2:1 | 2.39 | 90.3 | (0.254, 0.701) | 541 |
| Example 43 | 1-230:2-42 | 1:2 | 2.10 | 87.3 | (0.256, 0.624) | 523 |
| Example 44 | 1-230:2-42 | 1:1 | 2.15 | 88.2 | (0.251, 0.634) | 520 |
| Example 45 | 1-230:2-42 | 2:1 | 2.23 | 88.9 | (0.268, 0.697) | 513 |
| Example 46 | 1-232:2-44 | 1:2 | 2.33 | 86.9 | (0.247, 0.689) | 539 |
| Example 47 | 1-232:2-44 | 1:1 | 2.42 | 87.3 | (0.245, 0.646) | 532 |
| Example 48 | 1-232:2-44 | 2:1 | 2.46 | 88.0 | (0.259, 0.625) | 526 |
| Example 49 | 1-238:2-33 | 1:2 | 2.38 | 82.3 | (0.272, 0.742) | 503 |
| Example 50 | 1-238:2-33 | 1:1 | 2.42 | 82.7 | (0.247, 0.667) | 498 |
| Example 51 | 1-238:2-33 | 2:1 | 2.45 | 83.6 | (0.255, 0.697) | 482 |
| Example 52 | 1-242:2-44 | 1:2 | 2.33 | 84.3 | (0.248, 0.727) | 476 |
| Example 53 | 1-242:2-44 | 1:1 | 2.37 | 84.9 | (0.258, 0.682) | 472 |
| Example 54 | 1-242:2-44 | 2:1 | 2.40 | 85.7 | (0.254, 0.678) | 470 |
| Example 55 | 1-244:2-33 | 1:2 | 2.39 | 85.4 | (0.244, 0.657) | 468 |
| Example 56 | 1-244:2-33 | 1:1 | 2.42 | 86.3 | (0.254, 0.676) | 462 |
| Example 57 | 1-244:2-33 | 2:1 | 2.44 | 86.9 | (0.267, 0.604) | 460 |
| Example 58 | 1-250:2-32 | 1:2 | 2.40 | 81.3 | (0.258, 0.649) | 462 |
| Example 59 | 1-250:2-32 | 1:1 | 2.42 | 81.9 | (0.283, 0.712) | 460 |
| Example 60 | 1-250:2-32 | 2:1 | 2.46 | 82.3 | (0.258, 0.734) | 458 |
| Example 61 | 1-259:2-44 | 1:2 | 3.22 | 75.3 | (0.245, 0.620) | 382 |
| Example 62 | 1-259:2-44 | 1:1 | 3.25 | 76.8 | (0.258, 0.709) | 380 |
| Example 63 | 1-259:2-44 | 2:1 | 3.31 | 77.1 | (0.243, 0.754) | 377 |
| Example 64 | 1-260:2-23 | 1:2 | 3.29 | 76.9 | (0.247, 0.641) | 388 |
| Example 65 | 1-260:2-23 | 1:1 | 3.32 | 77.3 | (0.253, 0.714) | 381 |
| Example 66 | 1-260:2-23 | 2:1 | 3.35 | 77.9 | (0.281, 0.702) | 372 |
| Example 67 | 1-266:2-42 | 1:2 | 3.26 | 76.9 | (0.248, 0.625) | 365 |
| Example 68 | 1-266:2-42 | 1:1 | 3.31 | 77.8 | (0.266, 0.670) | 362 |
| Example 69 | 1-266:2-42 | 2:1 | 3.38 | 78.2 | (0.252, 0.720) | 355 |
| Example 70 | 1-272:2-33 | 1:2 | 3.15 | 78.3 | (0.265, 0.658) | 378 |
| Example 71 | 1-272:2-33 | 1:1 | 3.19 | 79.3 | (0.254, 0.637) | 362 |
| Example 72 | 1-272:2-33 | 2:1 | 3.21 | 79.9 | (0.235, 0.638) | 359 |
| Example 73 | 1-273:2-23 | 1:2 | 4.03 | 73.6 | (0.245, 0.635) | 400 |
| Example 74 | 1-273:2-23 | 1:1 | 4.13 | 74.8 | (0.266, 0.658) | 392 |
| Example 75 | 1-273:2-23 | 2:1 | 4.21 | 75.2 | (0.248, 0.615) | 382 |
| Example 76 | 1-276:2-34 | 1:2 | 4.19 | 74.0 | (0.268, 0.625) | 377 |
| Example 77 | 1-276:2-34 | 1:1 | 4.20 | 74.6 | (0.249, 0.653) | 365 |
| Example 78 | 1-276:2-34 | 2:1 | 4.28 | 75.2 | (0.269, 0.669) | 361 |
| Example 79 | 1-277: 2-42 | 1:2 | 4.33 | 73.9 | (0.253, 0.707) | 369 |
| Example 80 | 1-277:2-42 | 1:1 | 4.22 | 74.1 | (0.257, 0.662) | 362 |
| Example 81 | 1-277:2-42 | 2:1 | 4.39 | 74.5 | (0.283, 0.668) | 360 |
| Example 82 | 1-278:2-33 | 1:2 | 4.40 | 74.4 | (0.257, 0.659) | 367 |
| Example 83 | 1-278:2-33 | 1:1 | 4.01 | 74.8 | (0.263, 0.673) | 362 |
| Example 84 | 1-278:2-33 | 2:1 | 4.10 | 75.1 | (0.287, 0.671) | 359 |
| Example 85 | 1-281:2-23 | 1:2 | 4.19 | 74.9 | (0.258, 0.697) | 403 |
| Example 86 | 1-281:2-23 | 1:1 | 4.22 | 75.2 | (0.266, 0.672) | 398 |
| Example 87 | 1-281:2-23 | 2:1 | 4.35 | 75.9 | (0.285, 0.670) | 382 |
| Example 88 | 1-286:2-34 | 1:2 | 4.46 | 70.8 | (0.273, 0.667) | 352 |
| Example 89 | 1-286:2-34 | 1:1 | 4.51 | 71.3 | (0.285, 0.673) | 350 |
| Example 90 | 1-286:2-34 | 2:1 | 4.55 | 72.2 | (0.275, 0.678) | 346 |
| Example 91 | 1-291:2-44 | 1:2 | 4.38 | 72.3 | (0.263, 0.653) | 348 |
| Example 92 | 1-291:2-44 | 1:1 | 4.41 | 72.6 | (0.265, 0.673) | 342 |
| Example 93 | 1-291:2-44 | 2:1 | 4.46 | 73.1 | (0.263, 0.651) | 339 |
| Example 94 | 1-294:2-32 | 1:2 | 4.42 | 70.9 | (0.252, 0.661) | 333 |
| Example 95 | 1-294:2-32 | 1:1 | 4.44 | 71.3 | (0.257, 0.645) | 330 |
| Example 96 | 1-294:2-32 | 2:1 | 4.53 | 72.8 | (0.257, 0.672) | 329 |
| Example 97 | 1-297:2-42 | 1:2 | 4.61 | 60.5 | (0.247, 0.664) | 350 |
| Example 98 | 1-297:2-42 | 1:1 | 4.58 | 61.8 | (0.252, 0.657) | 345 |
| Example 99 | 1-297:2-42 | 2:1 | 4.55 | 62.3 | (0.269, 0.675) | 342 |
| Example 100 | 1-301:2-34 | 1:2 | 4.76 | 64.7 | (0.267, 0.671) | 328 |
| Example 101 | 1-301:2-34 | 1:1 | 4.66 | 65.2 | (0.285, 0.675) | 321 |
| Example 102 | 1-301:2-34 | 2:1 | 4.52 | 66.3 | (0.286, 0.673) | 318 |
| Example 103 | 1-303:2-44 | 1:2 | 3.08 | 80.7 | (0.278, 0.667) | 389 |
| Example 104 | 1-303:2-44 | 1:1 | 3.19 | 81.3 | (0.258, 0.657) | 385 |
| Example 105 | 1-303:2-44 | 2:1 | 3.22 | 81.9 | (0.263, 0.661) | 381 |
| Example 106 | 1-304:2-23 | 1:2 | 3.13 | 81.3 | (0.257, 0.674) | 371 |
| Example 107 | 1-304:2-23 | 1:1 | 3.27 | 81.6 | (0.275, 0.687) | 362 |
| Example 108 | 1-304:2-23 | 2:1 | 3.35 | 82.7 | (0.267, 0.685) | 360 |

As seen from the results of Table 5 and Table 6, effects of more superior efficiency and lifetime are obtained when including the compound of Chemical Formula 1 and the compound of Chemical Formula 2 at the same time. Such results may lead to a forecast that an exciplex phenomenon occurs when including the two compounds at the same time. The exciplex phenomenon is a phenomenon of releasing energy having sizes of a donor (P-host) HOMO level and an acceptor (N-host) LUMO level due to electron exchanges between two molecules. When a donor (P-host) having a favorable hole transfer ability and an acceptor (N-host) having a favorable electron transfer ability are used as a host of a light emitting layer, holes are injected to the P-host and electrons are injected to the N-host, and therefore, a driving voltage may be lowered, which resultantly helps with enhancement in the lifetime. In the present disclosure, it was identified that superior device properties were obtained when, as a light emitting layer host, using the compound of Chemical Formula 1 as an acceptor role and the compound of Chemical Formula 2 as a donor role.

In addition, it was seen that, an excellent lifetime was obtained when using a combination of the compound including deuterium among the compounds of the present disclosure and the compound of Chemical Formula 2 compared to when using a combination of the compound of the present disclosure substituted only with hydrogen and the compound of Chemical Formula 2.

## Claims

1. A heterocyclic compound represented by the following Chemical Formula 1: 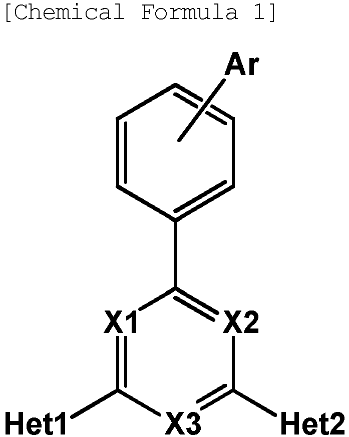
wherein, in Chemical Formula 1,
X1 to X3 are N or CR, and at least one of X1 to X3 is N;
R is hydrogen; or deuterium,
Ar is a C9 to C60 aryl group formed with a monocyclic ring,
Het1 is represented by the following Chemical Formula 1-A, and
Het2 is represented by any one of the following Chemical Formulae 1-B to 1-D, 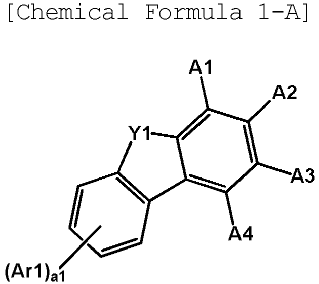 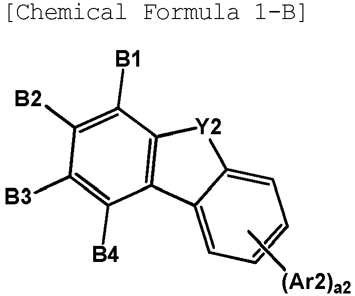 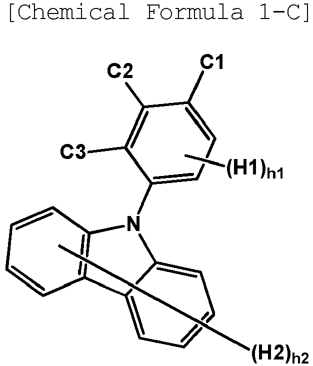 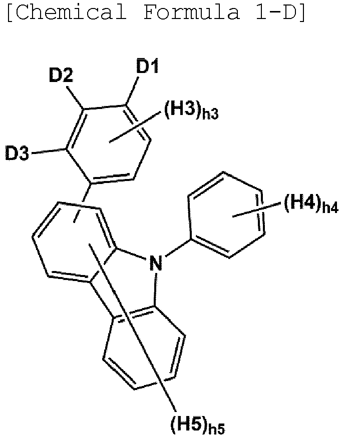
in Chemical Formulae 1-A to 1-D,
Y1 and Y2 are each independently O; S; or NR',
R' is a C6 to C60 aryl group unsubstituted or substituted with deuterium; or a C2 to C60 heteroaryl group,
H1 to H5 are hydrogen; or deuterium,
h1 and h3 are each an integer of 0 to 2, h2 is an integer of 0 to 8, h4 is an integer of 0 to 5, h5 is an integer of 0 to 7, and when h1 and h3 are each 2 or h2, h4 and h5 are each 2 or greater, substituents in the parentheses are the same as or different from each other,
Ar1 and Ar2 are each independently hydrogen; deuterium; or a substituted or unsubstituted C6 to C18 aryl group,
a1 and a2 are each an integer of 0 to 4, and when a1 and a2 are each 2 or greater, substituents in the parentheses are the same as or different from each other,
A1 to A4, B1 to B4, C1 to C3 and D1 to D3 either bond to Chemical Formula 1, or hydrogen; or deuterium, and
Chemical Formula 1 bonds to any one of A1 to A4 of Chemical Formula 1-A, and bonds to any one of B1 to B4 of Chemical Formula 1-B, any one of C1 to C3 of Chemical Formula 1-C or any one of D1 to D3 of Chemical Formula 1-D, which is represented by Am-Bn, Am-Co or Am-Dp, m and n are each 1, 2, 3 or 4, o and p are each 1, 2 or 3, m and n, m and o or m and p are each the same as or different from each other, and when Y1 and Y2 are each O or S, m and n are different.

2. The heterocyclic compound of Claim 1, wherein Ar is represented by any one of the following structural formulae:

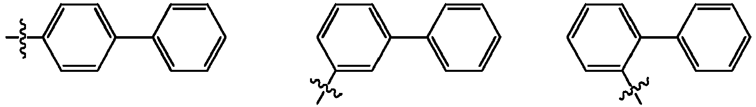

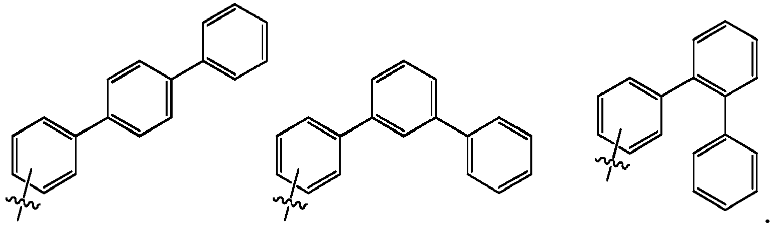

3. The heterocyclic compound of Claim 1, wherein Chemical Formula 1 is represented by any one of the following Chemical Formulae 1-1 to 1-7: 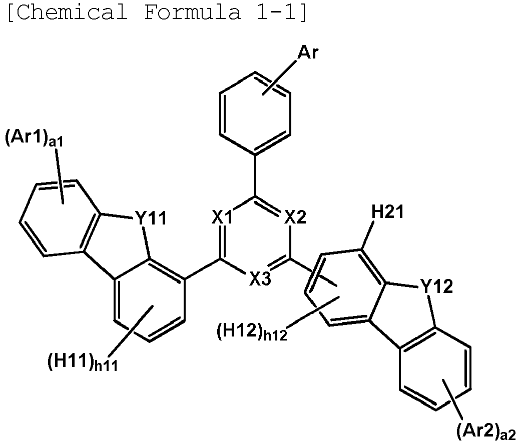 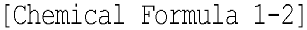 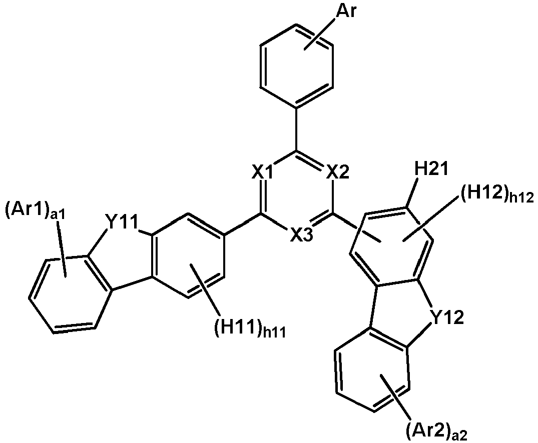 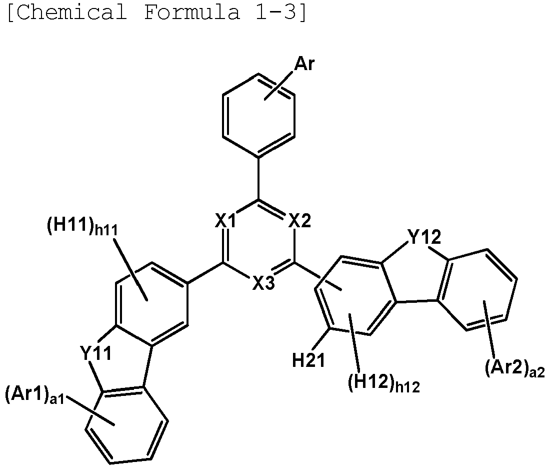 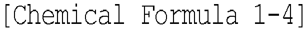 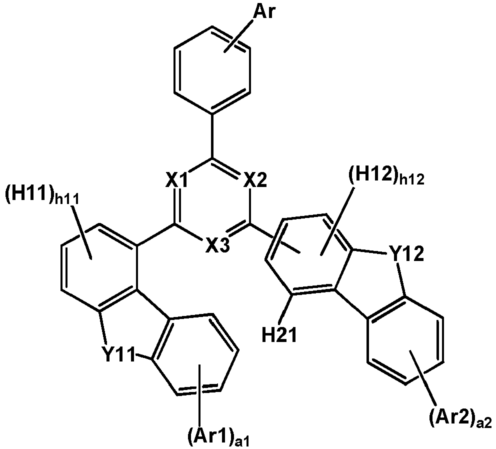 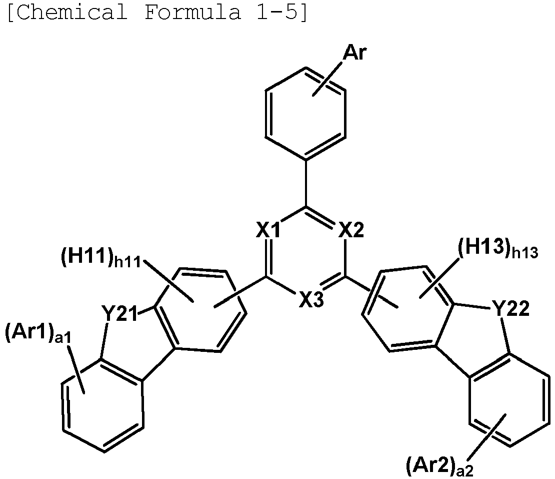 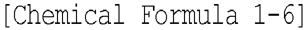 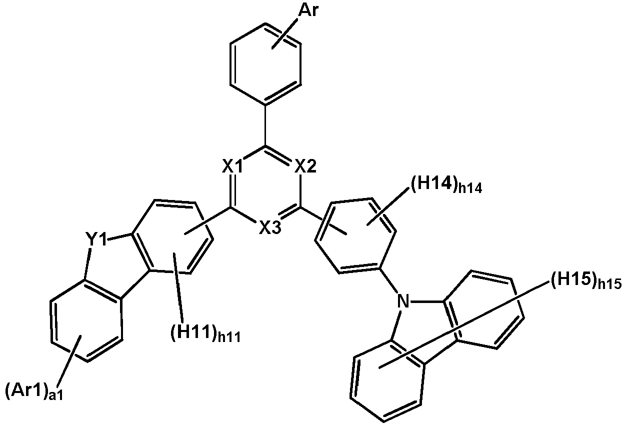 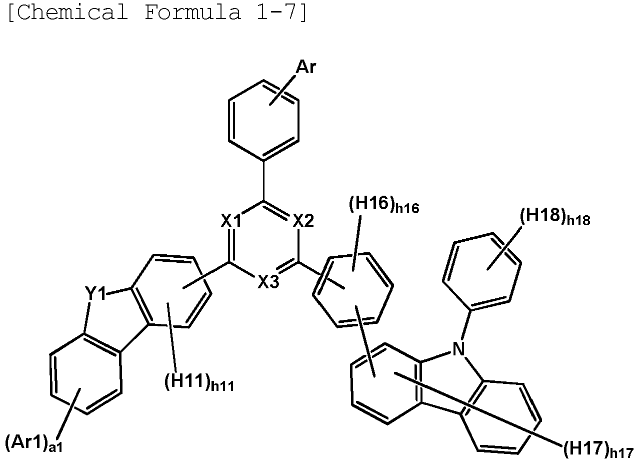
in Chemical Formulae 1-1 to 1-7,
Y11 and Y12 are each independently O or S,
Y21 and Y22 are each independently O; S; or NR', and at least one of Y21 and Y22 is NR',
H11 to H18 and H21 are each independently hydrogen; or deuterium,
h11 and h13 are each an integer of 0 to 3, h12 is an integer of 0 to 2, h14 and h16 are each an integer of 0 to 4, h15 is an integer of 0 to 8, h17 is an integer of 0 to 7 and h18 is an integer of 0 to 5, and when h12 is 2 or h11 and h13 to h18 are each 2 or greater, substituents in the parentheses are the same as or different from each other,
X1 to X3 and Ar each have the same definitions as in Chemical Formula 1, and
Y1, R', Ar1, Ar2, a1 and a2 each have the same definitions as in Chemical Formulae 1-A and 1-B.

4. The heterocyclic compound of Claim 1, wherein Chemical Formula 1 is represented by any one of the following compounds:

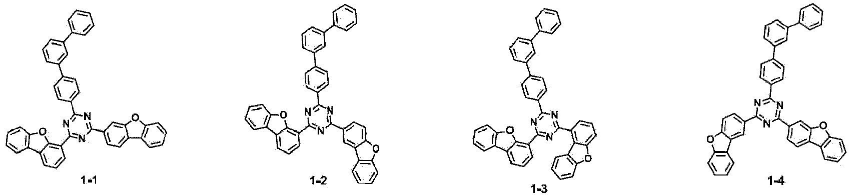

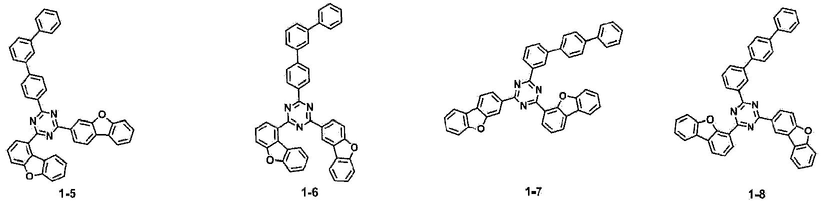

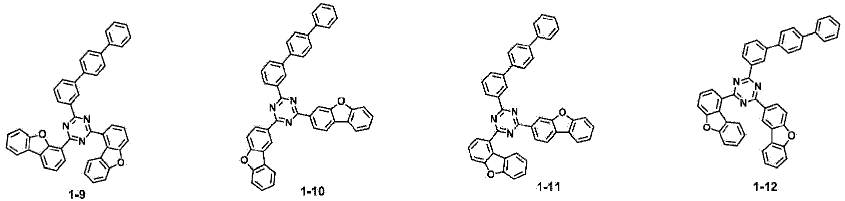

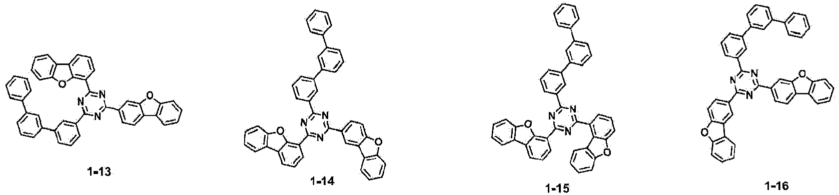

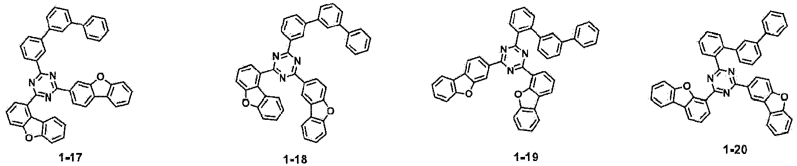

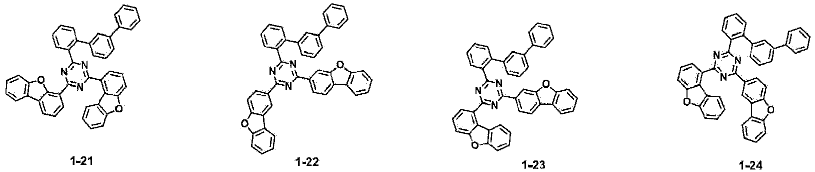

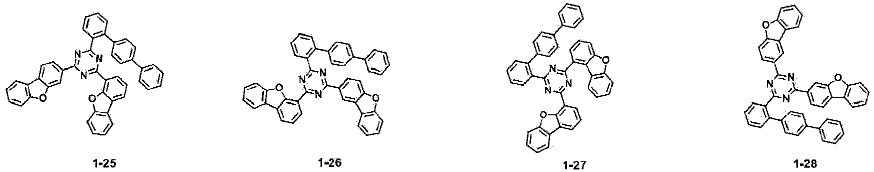

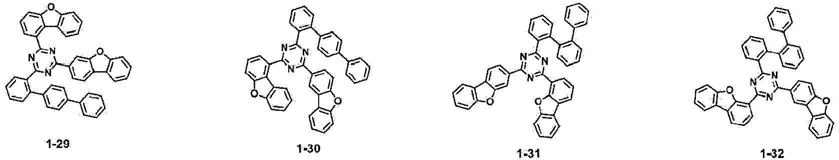

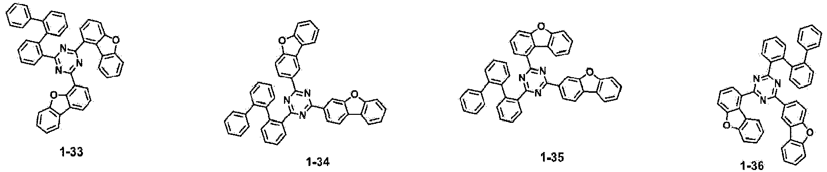

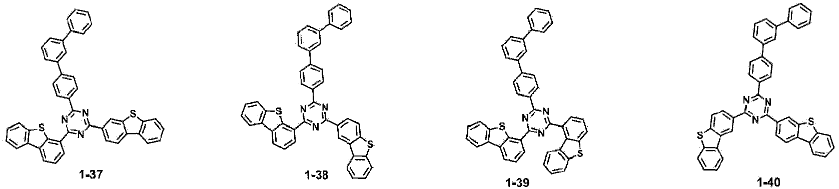

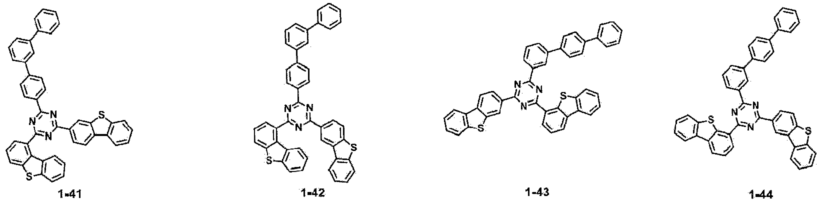

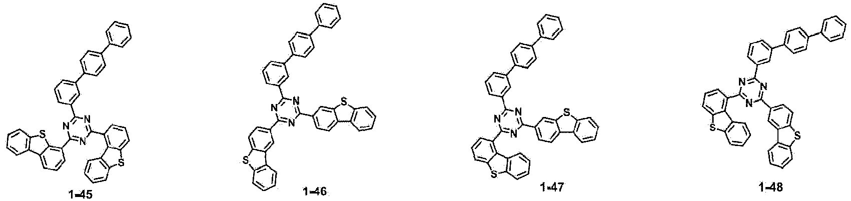

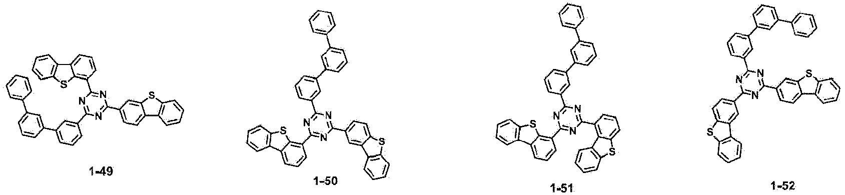

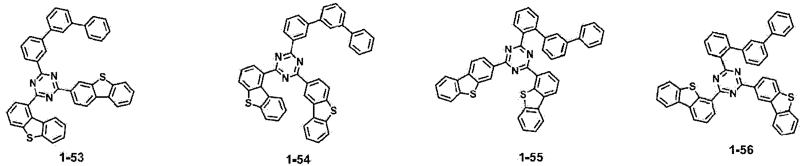

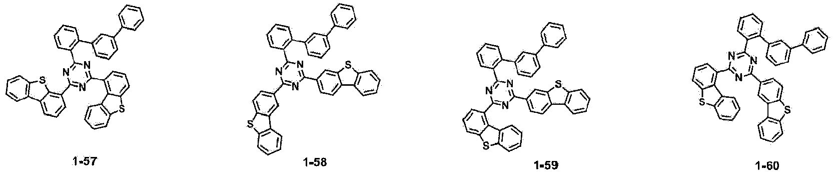

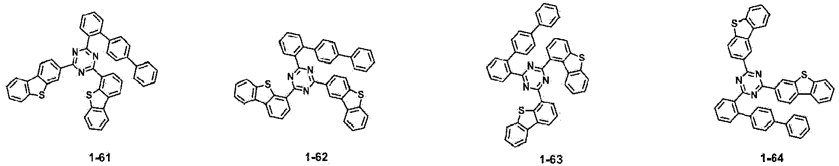

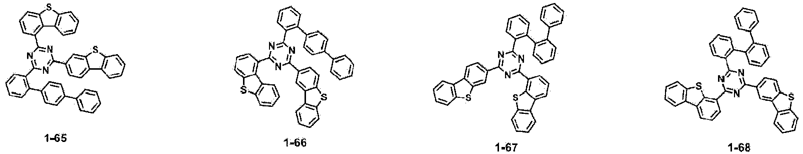

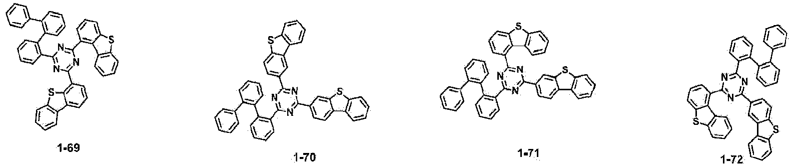

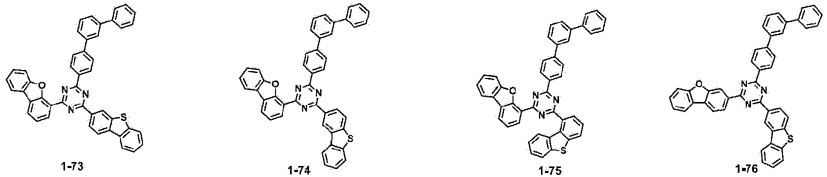

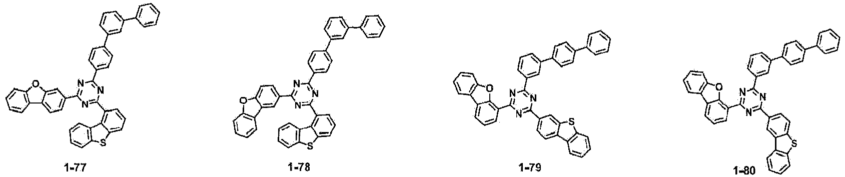

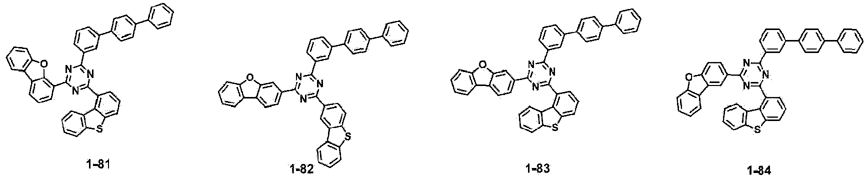

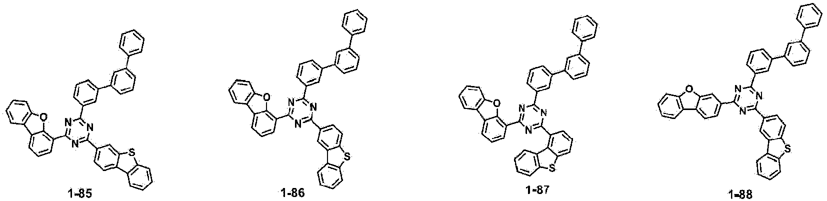

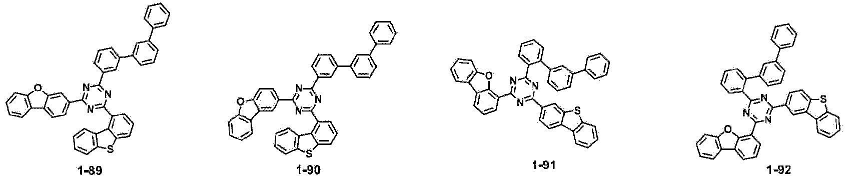

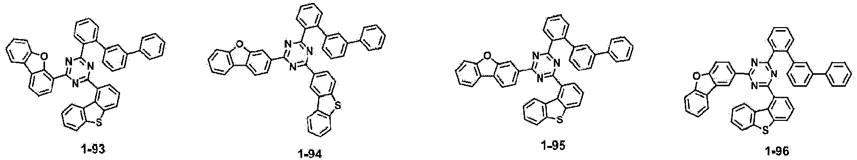

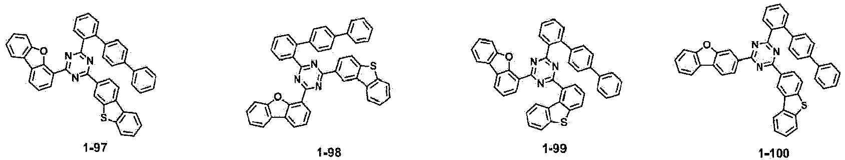

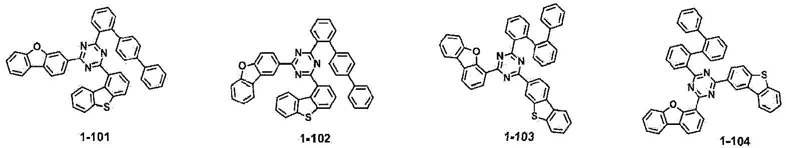

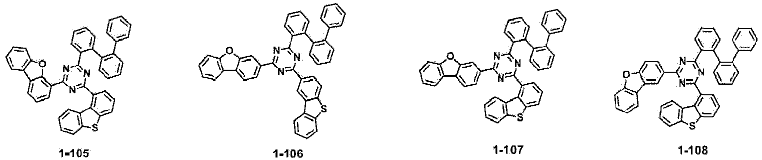

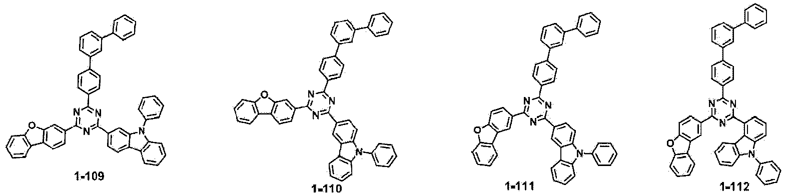

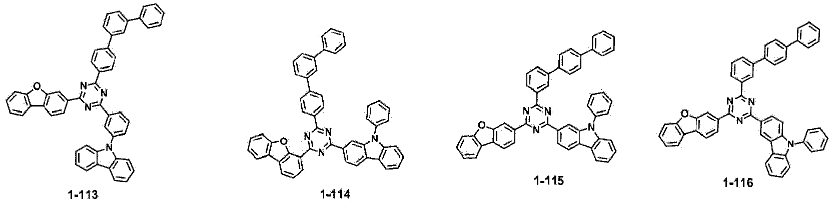

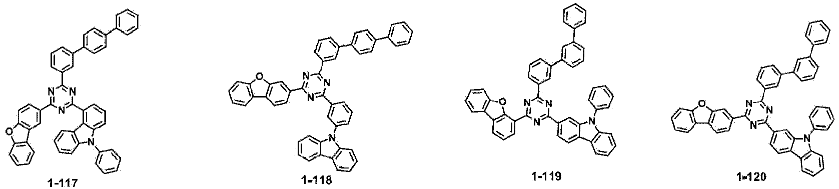

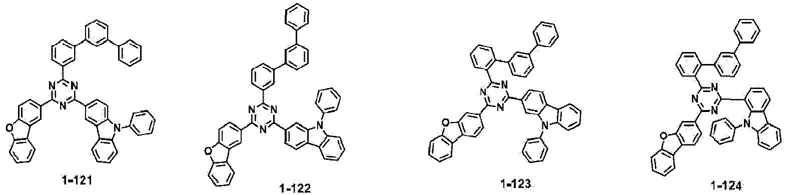

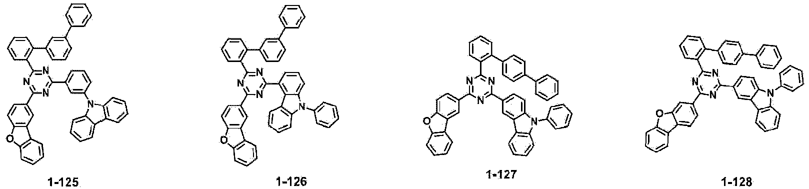

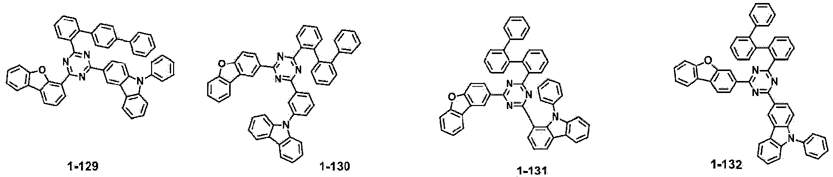

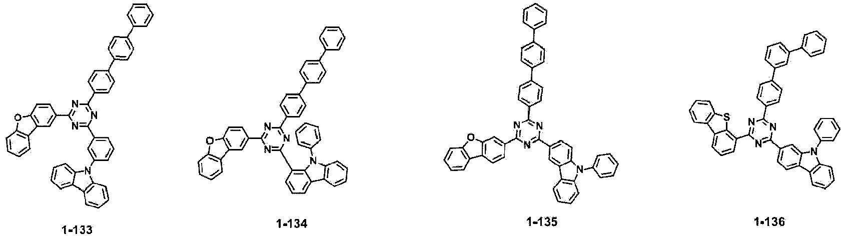

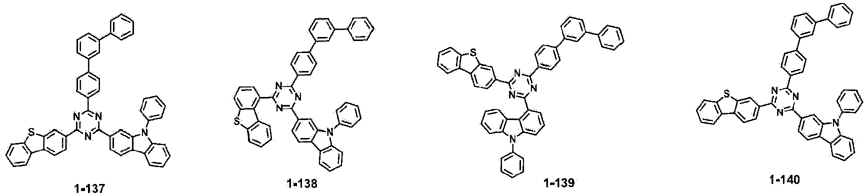

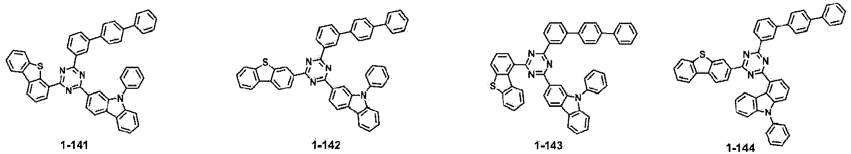

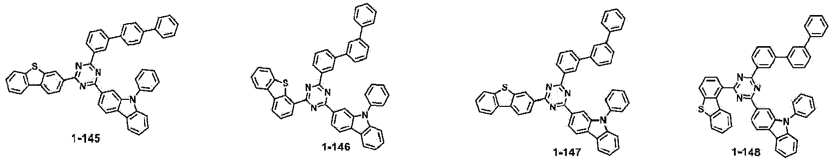

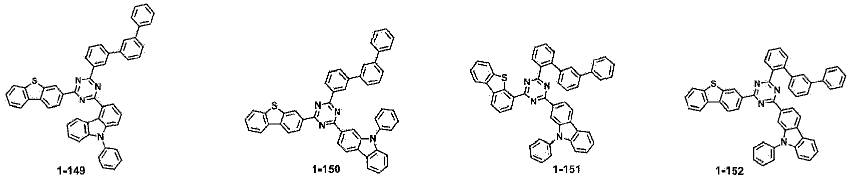

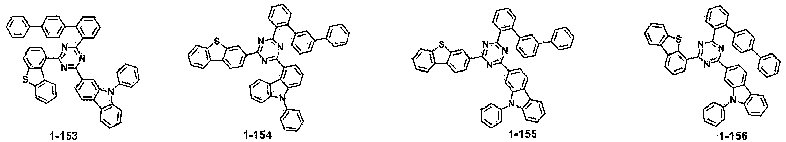

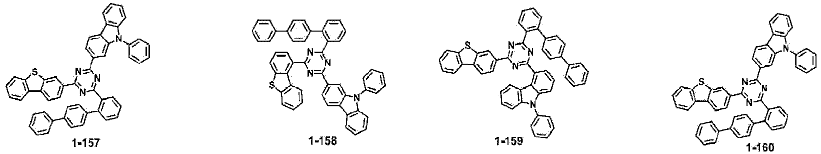

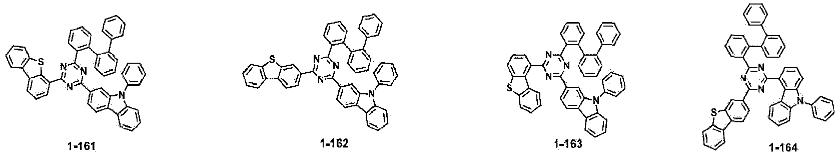

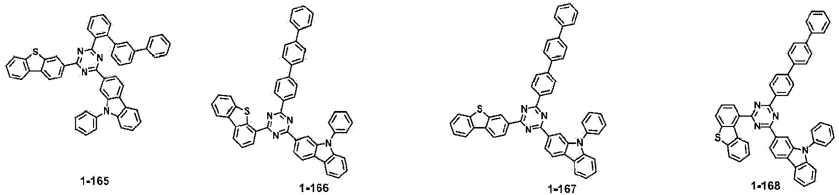

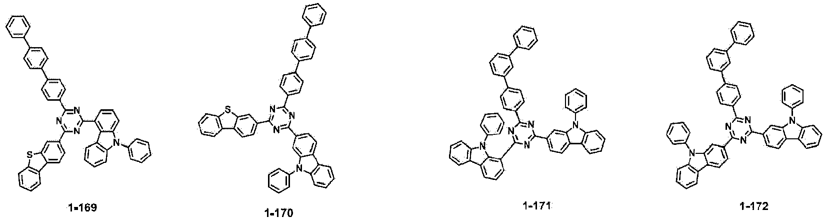

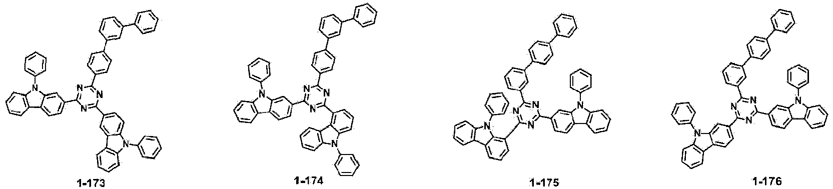

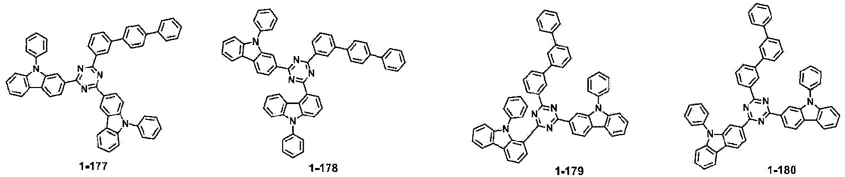

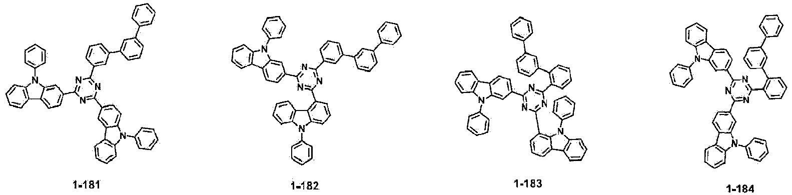

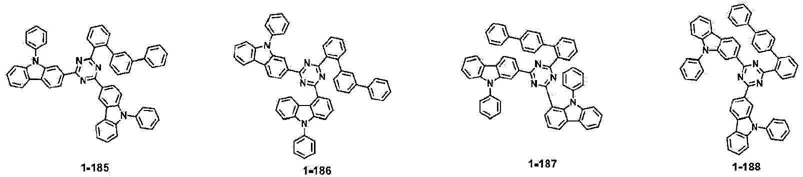

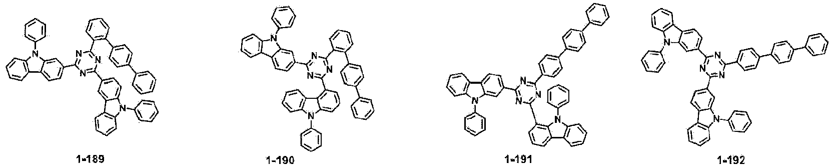

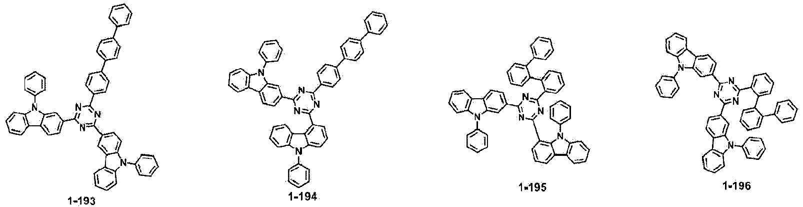

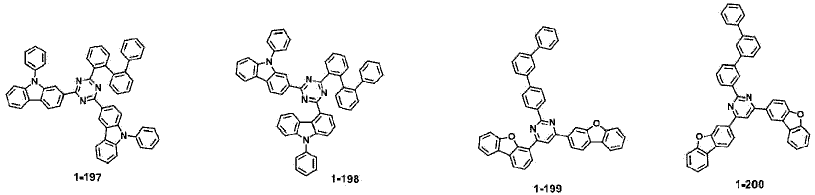

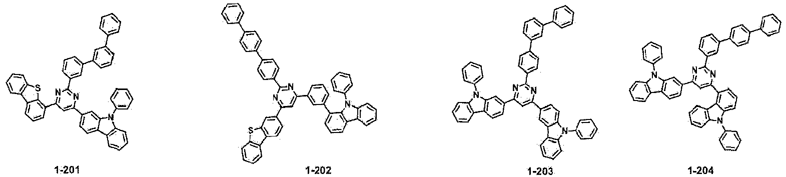

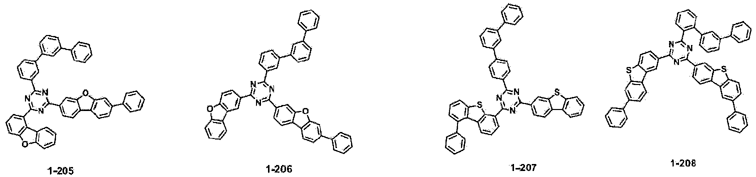

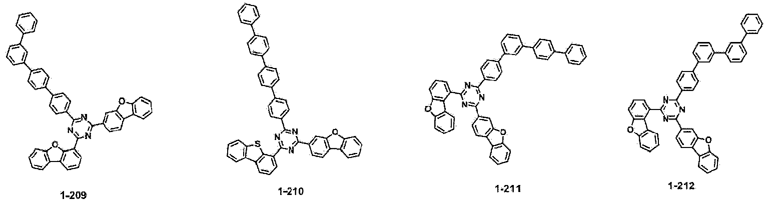

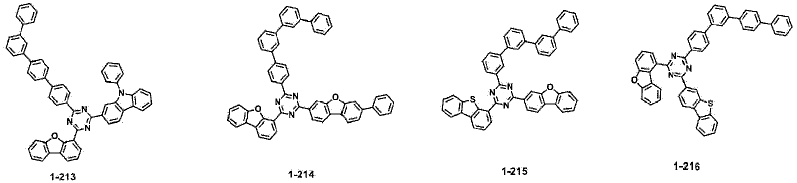

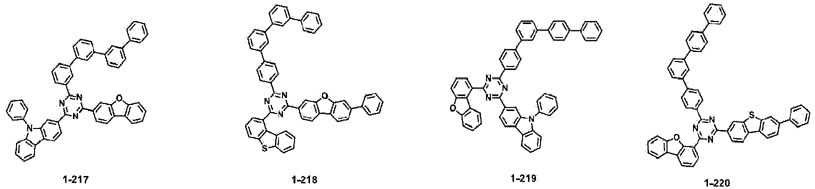

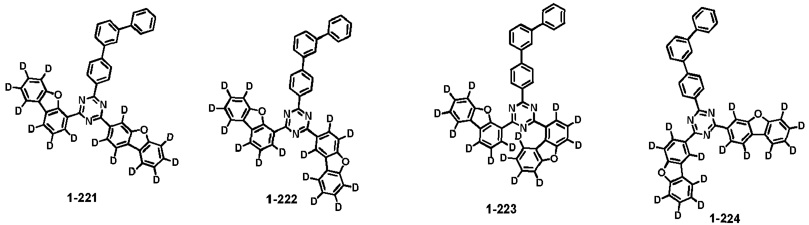

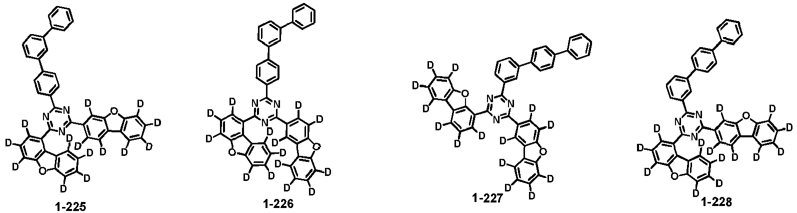

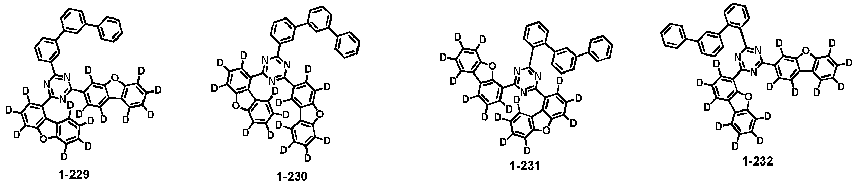

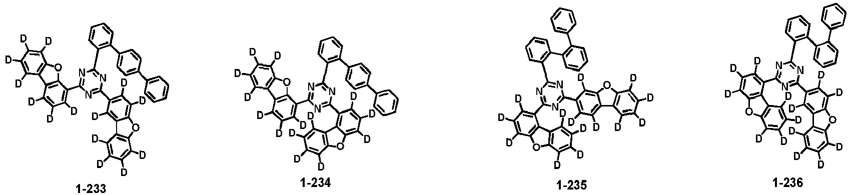

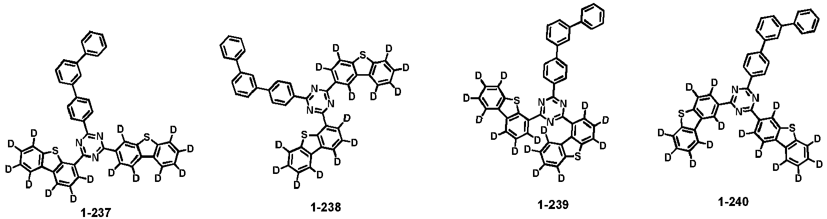

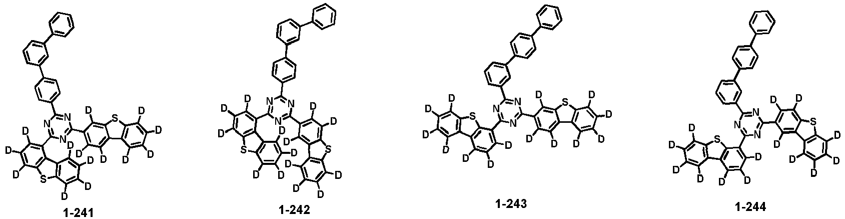

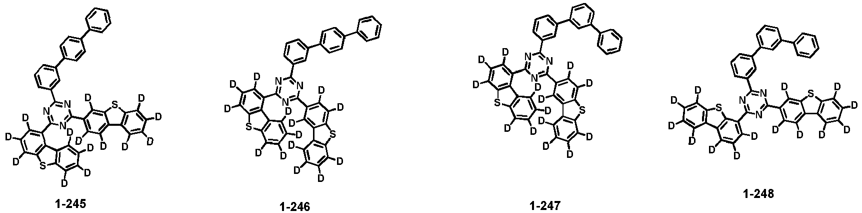

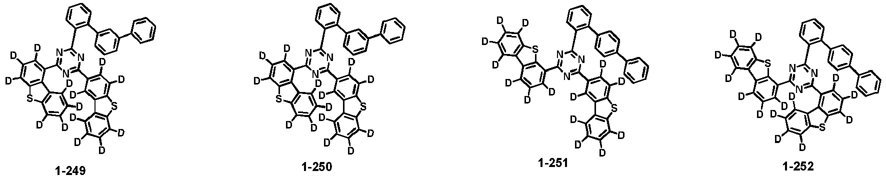

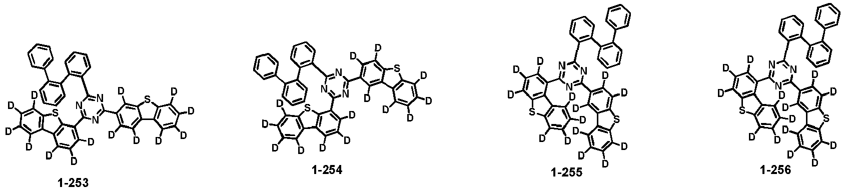

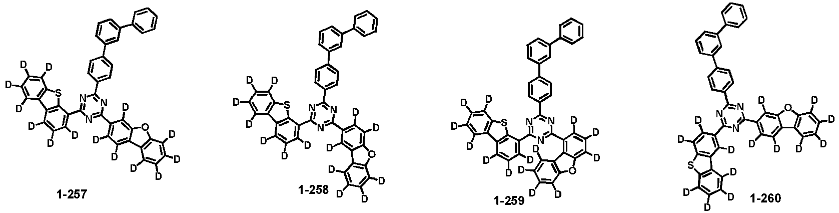

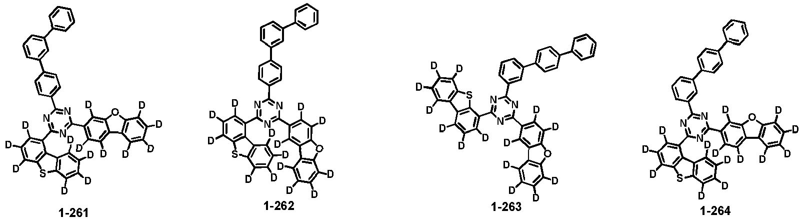

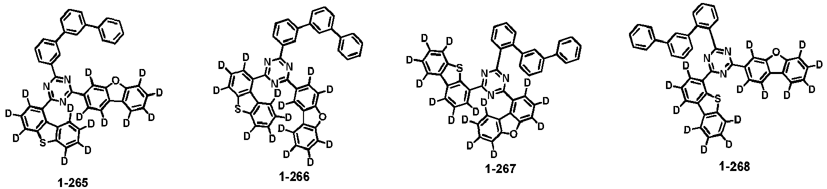

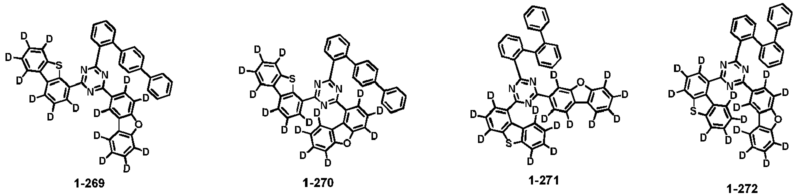

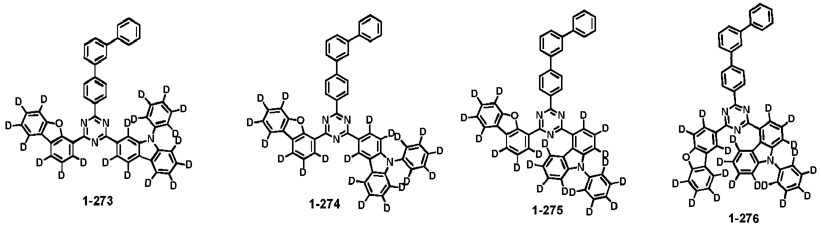

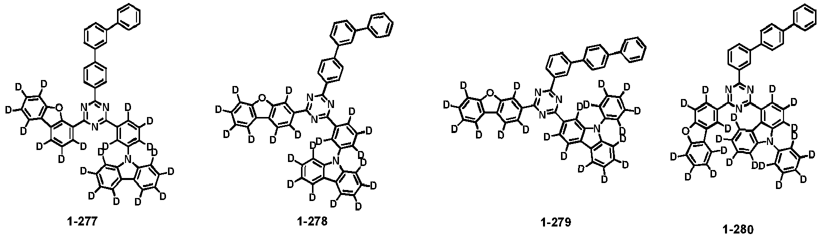

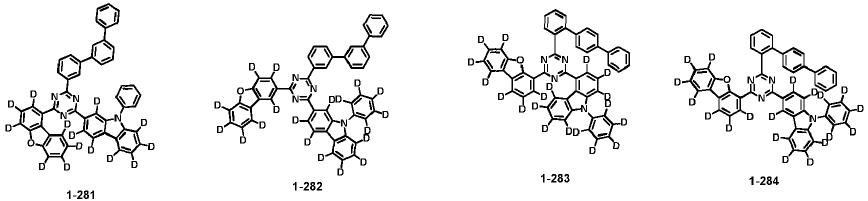

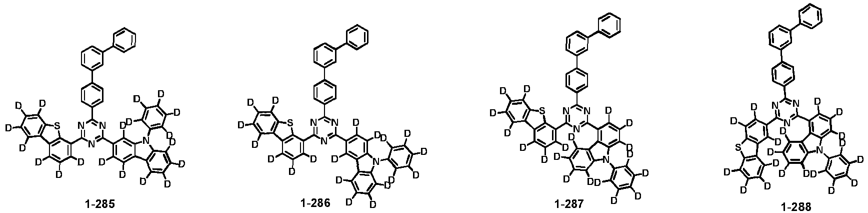

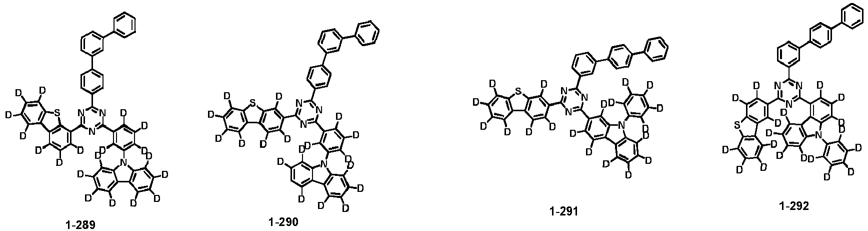

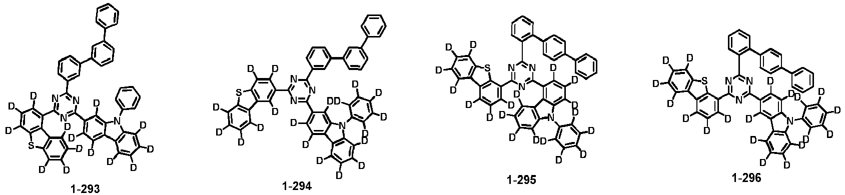

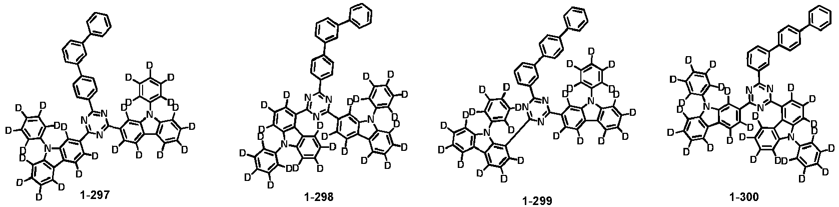

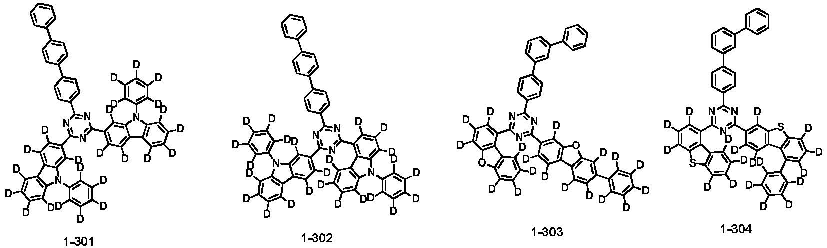

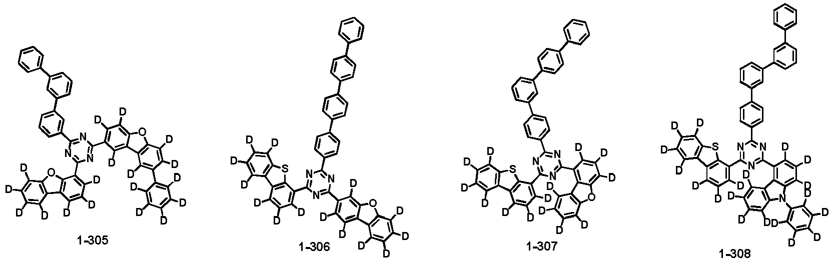

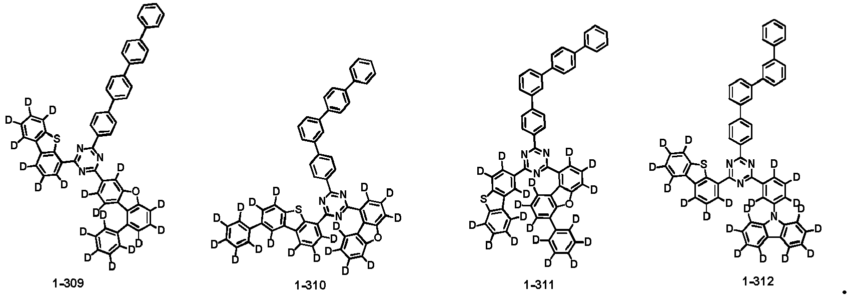

5. An organic light emitting device comprising:
a first electrode;
a second electrode; and
an organic material layer provided between the first electrode and the second electrode,
wherein the organic material layer includes one or more types of the heterocyclic compound of any one of Claims 1 to 4.

6. The organic light emitting device of Claim 5, wherein the organic material layer includes a light emitting layer, and the light emitting layer includes the heterocyclic compound.

7. The organic light emitting device of Claim 5, wherein the organic material layer includes a light emitting layer, the light emitting layer includes a host, and the host includes the heterocyclic compound.

8. The organic light emitting device of Claim 6, wherein the light emitting layer further includes a compound of the following Chemical Formula 2: 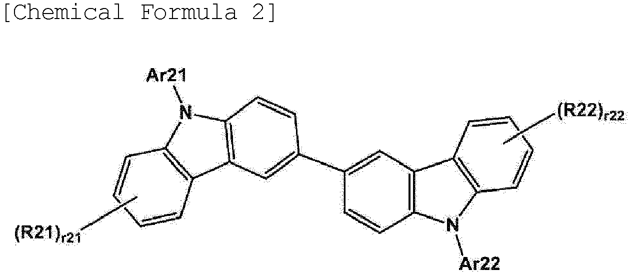
in Chemical Formula 2,
R21 and R22 are each independently hydrogen; deuterium; a halogen group; a cyano group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
Ar21 and Ar22 are each independently a substituted or unsubstituted C6 to C60 aryl group; or a substituted or unsubstituted C2 to C60 heteroaryl group,
r21 is an integer of 0 to 4, and when 2 or greater, R21s are the same as or different from each other, and
r22 is an integer of 0 to 4, and when 2 or greater, R22s are the same as or different from each other.

9. The organic light emitting device of Claim 8, wherein Chemical Formula 2 is represented by any one of the following compounds:

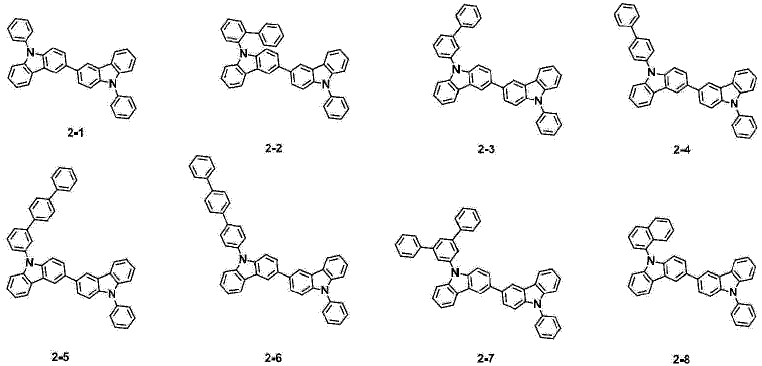

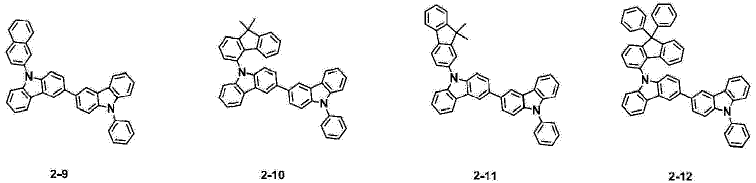

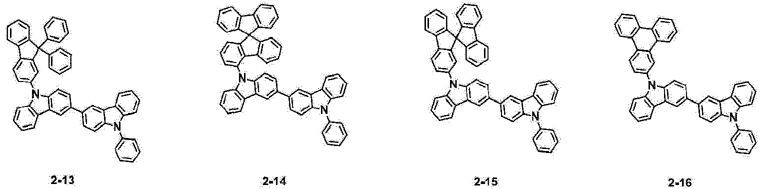

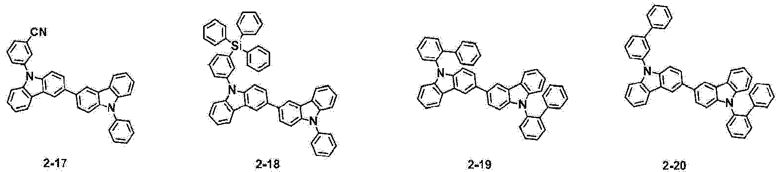

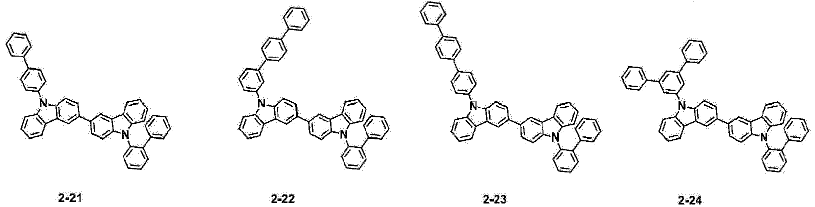

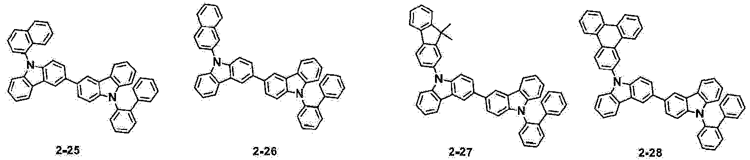

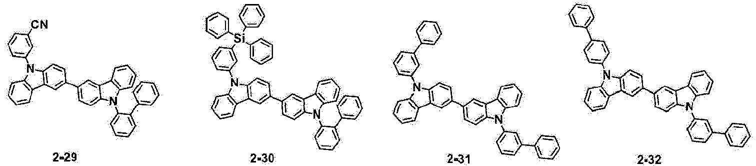

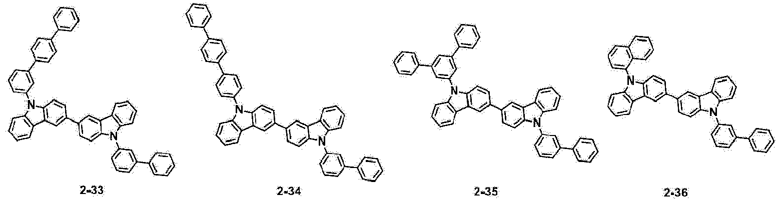

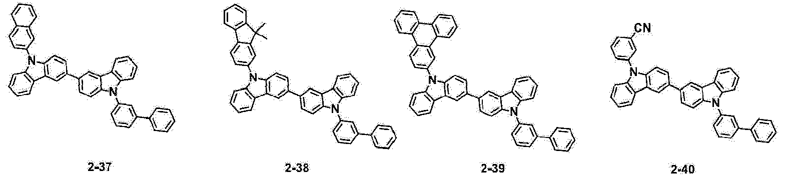

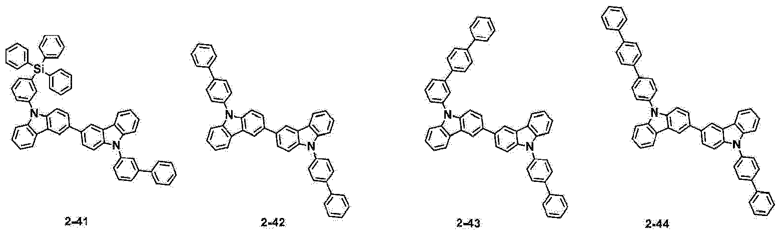

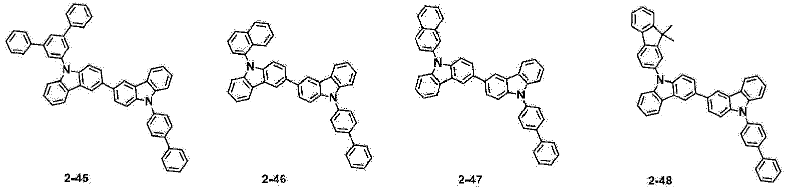

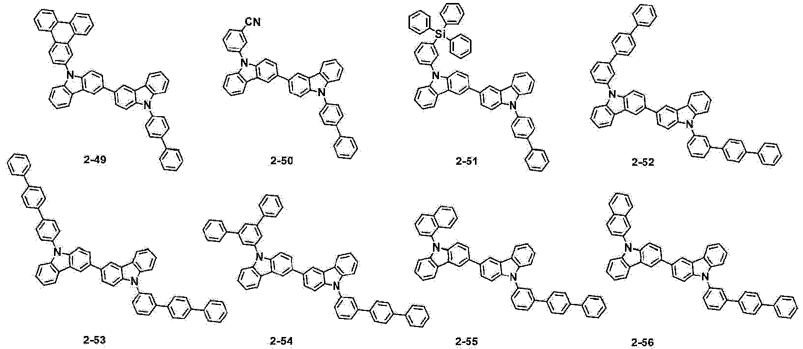

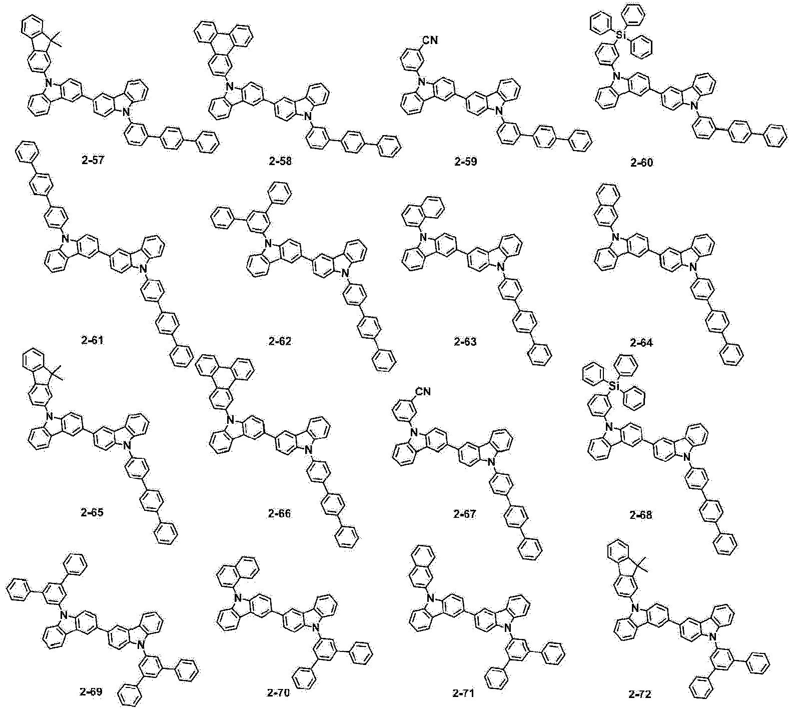

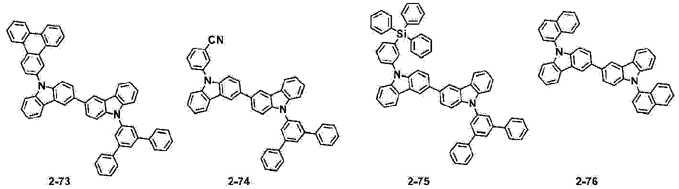

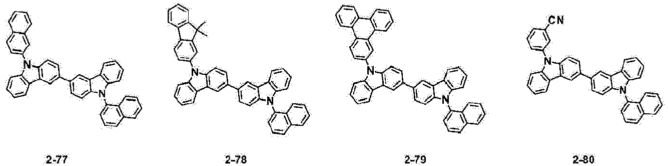

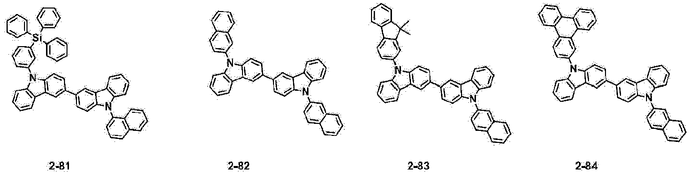

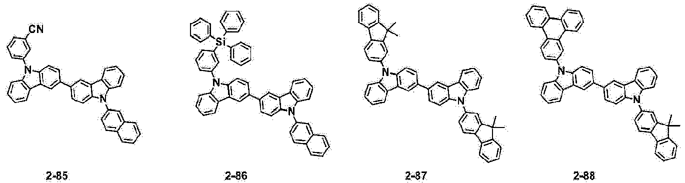

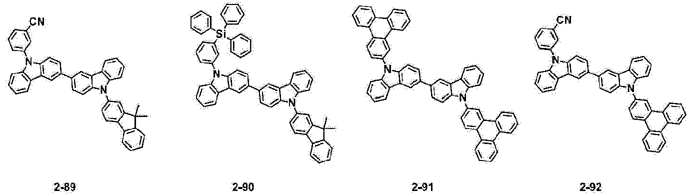

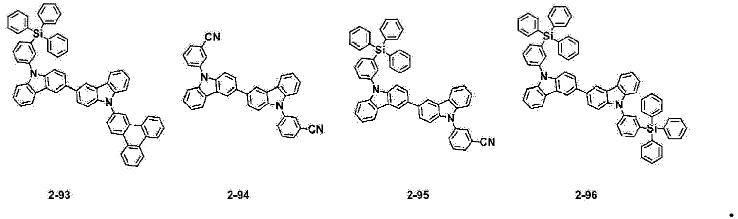

10. A composition for forming an organic material layer, the composition comprising:
the heterocyclic compound of Chemical Formula 1 of Claim 1; and
a compound of the following Chemical Formula 2: 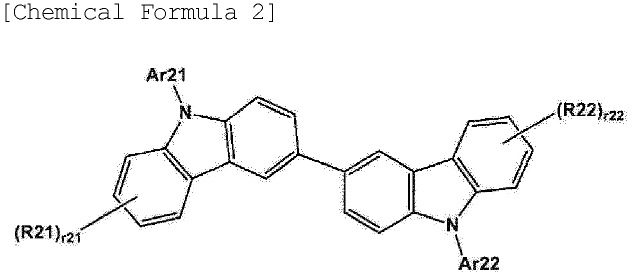
wherein, in Chemical Formula 2,
R21 and R22 are each independently hydrogen; deuterium; a halogen group; a cyano group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group;
Ar21 and Ar22 are each independently a substituted or unsubstituted C6 to C60 aryl group; or a substituted or unsubstituted C2 to C60 heteroaryl group;
r21 is an integer of 0 to 4, and when 2 or greater, R21s are the same as or different from each other; and
r22 is an integer of 0 to 4, and when 2 or greater, R22s are the same as or different from each other.

11. The composition for forming an organic material layer of Claim 10, wherein the heterocyclic compound and the compound of Chemical Formula 2 have a weight ratio of 1:10 to 10:1.
